(19) Europäisches Patentamt
European Patent Office
Office européen des brevets

(11) EP 2 820 047 B1

(12) **EUROPEAN PATENT SPECIFICATION**

(45) Date of publication and mention of the grant of the patent:
**25.04.2018 Bulletin 2018/17**

(21) Application number: **13716734.2**

(22) Date of filing: **01.03.2013**

(51) Int Cl.:
***C07K 16/28*** *(2006.01)*

(86) International application number:
**PCT/EP2013/054223**

(87) International publication number:
**WO 2013/128027 (06.09.2013 Gazette 2013/36)**

(54) **LONG LIFE POLYPEPTIDE BINDING MOLECULES**

LANGLEBIGE POLYPEPTIDBINDUNGSMOLEKÜLE

MOLÉCULES DE LIAISON À UN POLYPEPTIDE À LONGUE DURÉE DE VIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.03.2012 US 201261605681 P**
**02.03.2012 US 201261606268 P**

(43) Date of publication of application:
**07.01.2015 Bulletin 2015/02**

(73) Proprietor: **Amgen Research (Munich) GmbH**
**81477 München (DE)**

(72) Inventors:
- **KUFER, Peter**
  **81477 Munich (DE)**
- **RAUM, Tobias**
  **81477 Munich (DE)**
- **LUTTERBUESE, Ralf**
  **81477 Munich (DE)**
- **HOFFMANN, Patrick**
  **81477 Munich (DE)**
- **MUENZ, Markus**
  **81477 Munich (DE)**
- **BROZY, Johannes**
  **81477 Munich (DE)**
- **KVESIC, Majk**
  **81477 Munich (DE)**

(74) Representative: **Schiweck, Weinzierl & Koch**
**Patentanwälte Partnerschaft mbB**
**Landsberger Straße 98**
**80339 München (DE)**

(56) References cited:
**WO-A2-2008/119567 WO-A2-2010/037836**
**US-A1- 2005 287 153**

- **ZOBEL K ET AL: "Phosphate ester serum albumin affinity tags greatly improve peptide half-life in vivo", BIOORGANIC & MEDICINAL CHEMISTRY LETTERS, PERGAMON, ELSEVIER SCIENCE, GB, vol. 13, no. 9, 5 May 2003 (2003-05-05), pages 1513-1515, XP002377893, ISSN: 0960-894X, DOI: 10.1016/S0960-894X(03)00209-9**
- **BAEUERLE PATRICK A ET AL: "BiTE: Teaching antibodies to engage T-cells for cancer therapy", CURRENT OPINION IN MOLECULAR THERAPEUTICS, THOMSON REUTERS (SCIETIFIC) LTD, vol. 11, no. 1, 1 February 2009 (2009-02-01), pages 22-30, XP009151509, ISSN: 2040-3445**
- **WOLF E ET AL: "BiTEs: bispecific antibody constructs with unique anti-tumor activity", DRUG DISCOVERY TODAY, ELSEVIER, RAHWAY, NJ, US, vol. 10, no. 18, 15 September 2005 (2005-09-15), pages 1237-1244, XP027684780, ISSN: 1359-6446 [retrieved on 2005-09-15]**
- **TRAUNECKER A ET AL: "Janusin: new molecular design for bispecific reagents.", INTERNATIONAL JOURNAL OF CANCER. SUPPLEMENT = JOURNAL INTERNATIONAL DU CANCER. SUPPLEMENT 1992, vol. 7, 1992, pages 51-52, XP009171574, ISSN: 0898-6924**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- TRAUNECKER A ET AL: "BISPECIFIC SINGLE CHAIN MOLECULES (JANUSINS) TARGET CYTOTOXIC LYMPHOCYTES ON HIV INFECTED CELLS", EMBO JOURNAL, OXFORD UNIVERSITY PRESS, SURREY, GB, vol. 10, no. 12, 1 December 1991 (1991-12-01), pages 3655-3659, XP000232579, ISSN: 0261-4189
- BERG J ET AL: "BISPECIFIC ANTIBODIES THAT MEDIATE KILLING OF CELLS INFECTED WITH HUMAN IMMUNODEFICIENCY VIRUS OF ANY STRAIN", PROCEEDINGS OF THE NATIONAL ACADEMY OF SCIENCES, NATIONAL ACADEMY OF SCIENCES, US, vol. 88, no. 11, 1 June 1991 (1991-06-01), pages 4723-4727, XP000208769, ISSN: 0027-8424, DOI: 10.1073/PNAS.88.11.4723
- WONG J T ET AL: "Selective reduction and proliferation of the CD4+ and CD8+ T cell subsets with bispecific monoclonal antibodies: Evidence for inter-T cell-mediated cytolysis", CLINICAL IMMUNOLOGY AND IMMUNOPATHOLOGY, SAN DIEGO, CA, US, vol. 58, no. 2, 1 February 1991 (1991-02-01), pages 236-250, XP026242471, ISSN: 0090-1229, DOI: 10.1016/0090-1229(91)90139-2 [retrieved on 1991-02-01]
- PAUL R HINTON ET AL: "An engineered human IgG1 antibody with longer serum half-life", THE JOURNAL OF IMMUNOLOGY, THE AMERICAN ASSOCIATION OF IMMUNOLOGISTS, US, vol. 176, no. 1, 1 January 2006 (2006-01-01), pages 346-356, XP002484005, ISSN: 0022-1767
- SPORN M B ET AL: "Proliferative Diseases", AMERICAN JOURNAL OF MEDICINE, EXCERPTA MEDICA, INC, UNITED STATES, vol. 70, no. 6, 1 June 1981 (1981-06-01), pages 1231-1236, XP026386403, ISSN: 0002-9343, DOI: 10.1016/0002-9343(81)90832-9 [retrieved on 1981-06-01]

## Description

### FIELD OF THE INVENTION

The present invention relates to binding molecules, which are bispecific antibodies, comprising at least three binding domains comprised in one polypeptide chain, wherein the first binding domain is a binding domain which is binding to a cell surface molecule on a target cell, the second binding domain is a binding domain which is binding to the T cell CD3 receptor complex, and the third domain is a binding domain which is binding to serum albumin, wherein said third domain is positioned at the C-terminus of said second domain, and wherein the three domains are on one polypeptide in the order from the N-terminus to the C-terminus the first binding domain; the second binding domain; and the third binding domain.. Moreover, the invention provides nucleic acid sequences encoding the bispecific antibodies , vectors comprising said nucleic acid sequences and host cells transformed or transfected with said vectors. Furthermore, the invention provides processes for the production of the bispecific antibodies of the invention, medical uses of said bispecific antibodies and kits comprising said bispecific antibodies.

### BACKGROUND OF THE INVENTION

An increased half-life is generally useful in in vivo applications of immunoglobulins, especially antibodies and most especially antibody fragments of small size. Such fragments (Fvs, disulphide bonded Fvs, Fabs, scFvs, dAbs) are likely to suffer from rapid clearance from the body; thus, whilst they are able to reach most parts of the body rapidly, and are quick to produce and easier to handle, their in vivo applications may be limited by their brief persistence in vivo.

Bispecific molecules such as BiTE® (Bispecific T-cell engager) antibodies are recombinant protein constructs made from two flexibly linked, single-chain antibodies (scFv). One scFv of BiTE® antibodies is specific for a selected tumor-associated surface antigen on target cells; the second scFv is specific for CD3, a subunit of the T-cell receptor complex on T-cells. Bispecific molecules directed against CD3 on the one hand and PSMA on the other hand are known e.g. from WO 2010/037836 A2, against CD19, EpCAM, CEA, CD33, EGFR, Her2 and MCSP e.g. from Bauerle et al. (BAEUERLE PATRICK A ET AL: "BiTE: Teaching antibodies to engage T-cells for cancer therapy" CURRENT OPINION IN MOLECULAR THERAPEUTICS vol. 11, 2009, pages 22-30), against EpCAM from Wolf et al. (WOLF E ET AL: "BiTEs: bispecific antibody constructs with unique anti-tumor activity", Drug Discovery Today, Volume 10, Number 18, September 2005) and against CD4 e.g. from Traunecker et al. (TRAUNECKER A ET AL: "Janusin: new molecular design for bispecific reagents." INTERNAT J CANCER. SUPPLEMENT 1992, vol. 7, pages 51-52). By their particular design and bivalent binding, BiTE® antibodies are uniquely suited to transiently bind T-cells to target cells and, at the same time, potently activate the inherent cytolytic potential of T-cells against target cells. BiTE® molecules are small proteins with a molecular weight below the renal cut off that could likely result in a shorter half-life, a feature that is shared by BiTE®s with many other antibody formats. In fact, while it is one the one hand desirable to have a small binding molecule, since, for example, it can quickly reach its designated location in the body and can also reach most parts of the body, the "size" of such a binding molecule is not favorable as regards, in particular, renal clearance. It may also happen that such a binding molecule is faster degraded, since it has, so to say, no good natural protection, unless it was stabilized before, for example, by amino acid changes. Thus, it is a balancing act between small size and stability/renal clearance.

It is therefore desirable to have available a binding molecule, in particular, a bispecific binding molecule that is improved in its stability and/or retarded in its renal clearance, thereby having an overall increased serum half-life, but advantageously still so small that it can be manufactured in good yield and/or conveniently handled.

### SUMMARY OF THE INVENTION

The invention solves this problem by providing means and methods in the form of bispecific antibodies comprising a half-life extending domain which shows an extended serum half-life compared to bispecific antibodies not comprising a half-life extending domain.

Thus, in a first aspect the present invention provides binding molecules, which are bispecific antibodies, comprising at least three consecutive binding domains comprised in one polypeptide chain, wherein

(a) the first binding domain is a domain which is binding to a cell surface molecule on a target cell;
(b) the second binding domain is a domain which is binding to the T cell CD3 receptor complex; and
(c) the third binding domain is a domain which is binding to serum albumin,

wherein said third domain is positioned at the C-terminus of said second domain.

Therein, the three domains are on one polypeptide in the order from the N-terminus to the C-terminus

- the first binding domain;
- the second binding domain; and
- the third binding domain.

In one embodiment of the invention the third domain of the bispecific antibody is an scFv or single domain antibody.

In one embodiment the binding molecule of the invention is a bispecific antibody, wherein

(a) the first binding domain is binding to the cell surface molecule on a human and a non-human primate cell;
(b) the second binding domain is binding to the T cell CD3 receptor complex on a human and a non-human primate cell, and
(c) the third binding domain is binding to human and non-human primate serum albumin.

In one embodiment of the invention the bispecific antibody is characterized in a way that the third binding domain binding to serum albumin is derived from a combinatorial library or an antibody binding domain.

In one embodiment of the invention the bispecific antibody is characterized in a way that the third binding domain comprises between 10 and 25 aa residues.

In one embodiment of the invention the bispecific antibody is characterized in a way that the third binding domain binding to serum albumin comprises the amino acid sequence Asp-Xaa-Cys-Leu-Pro-Xaa-Trp-Gly-Cys-Leu-Trp, wherein Xaa is any amino acid.

In one embodiment of the invention the bispecific antibody is characterized in a way that the third binding domain binding to serum albumin is derived from a CDR of a single domain antibody.

In one embodiment of the invention the bispecific antibody is characterized in a way that the third binding domain is binding to serum albumin with an affinity (KD) of $\leq$ 500 nM.

In one embodiment of the invention the bispecific antibody is characterized in a way that the binding molecule shows cytotoxic activity in an in vitro assay measuring the lysis of target cells by effector cells in the presence of 10% human serum albumin.

In one embodiment of the invention the bispecific antibody is characterized in a way that the molecule consists of a single polypeptide chain.

In one embodiment of the invention the bispecific antibody is characterized in a way that

(a) the first binding domain comprises an antibody derived VL and VH chain; and/or
(b) the second binding domain comprises an antibody derived VL and VH chain.

In one embodiment of the invention the bispecific antibody is characterized in a way that the molecule comprises one or more further heterologous polypeptide.

In one embodiment of the invention the bispecific antibody is characterized in a way that the first binding domain binding to a cell surface molecule is binding to a tumor antigen.

In one embodiment of the invention the bispecific antibody is characterized in a way that the second binding domain binding to the T cell CD3 receptor complex is binding to an epitope of human and Callithrix jacchus, Saguinus oedipus or Saimiri sciureus CD3$\varepsilon$ chain, wherein the epitope is part of an amino acid sequence comprised in the group consisting of SEQ ID NOs: 2, 4, 6, or 8 and comprises at least the amino acid sequence Gln-Asp-Gly-Asn-Glu.

In one embodiment of the invention the bispecific antibody is characterized by an amino acid sequence as depicted in SEQ ID NOs: 51, 52, 54, 55, 57, 58, 60, 61, 75, 76, 81, 82, 85, 86, 90, 91, 85, 96, 100 or 101.

In another aspect the present invention provides a nucleic acid sequence encoding a bispecific antibody of the invention.

In another aspect the present invention provides a vector comprising a nucleic acid sequence of the invention.

In another aspect the present invention provides a host cell transformed or transfected with the nucleic acid sequence of the invention or with the vector of the invention.

In another aspect the present invention provides a process for the production of a bispecific antibody of the invention, said process comprising culturing a host cell of the invention under conditions allowing the expression of the bispecific antibody of the invention and recovering the produced bispecific antibody from the culture.

In another aspect the present invention provides a pharmaceutical composition comprising a bispecific antibody of the invention, or produced according to the process of the invention.

In another aspect the present invention provides the bispecific antibody of the invention, or produced according to the process of the invention for use in the prevention, treatment or amelioration of a disease selected from the group consisting of a proliferative disease, an inflammatory disease, an infectious disease and an autoimmune disease.

In another aspect the present invention provides a kit comprising a bispecific antibody of the invention, a nucleic

acid molecule of the invention, a vector of the invention, or a host cell of the invention.

## DETAILED DESCRIPTION OF THE INVENTION

The mode of action of the bispecific antibodies that binds both to a cell surface molecule on a target cell such as a tumor antigen and to the T cell CD3 receptor complex is commonly known. Bringing a T cell in close vicinity to a target cell, i.e., engaging said T cell results under the circumstances in killing of the target cell by the T cell. This process can be exploited in fighting against proliferative disease, inflammatory disease, infectious disease and autoimmune disease. Thus, fusing anything such as additional amino acid sequences to the CD3 binding domain, i.e., the "effector domain" of a binding molecule or to the target binding domain influences the properties of the binding molecule such that it would no longer exert its function in properly engaging a T cell and/or binding to its target. Indeed, T-cells are equipped with granules containing a deadly combination of pore-forming proteins, called perforins, and cell death-inducing proteases, called granzymes. These proteins are delivered into target cells via a cytolytic synapse that can only form if T-cells are in closest vicinity with a target cell that is aimed to be killed. Normally, closest vicinity between a T cell and a target cell is achieved by the T cell binding to an MHC class I/peptide complex using its matching T-cell receptor. Yet, it is the function of the bispecific antibodies of the present invention to bring a T cell into such close vicinity to a target cell in the absence of T cell receptor/MHC interaction. Hence, one can imagine that fusing anything such as additional amino acid sequences to either or both of the first and/or second binding domain of the bispecific antibodies of the present invention could negatively influence the function thereof, i.e., bringing together a target cell and a T cell in order to kill the target cell.

That being so and bearing in mind that it is highly desirable to increase the serum half-life of the bispecific antibodies in order to stabilize it or prevent it from fast renal clearance and the like, the skilled person seems to be in a dilemma. Indeed, while an increase of the half-life could be achieved by having a bispecific antibody binding to, e.g., serum albumin which requires equipping said bispecific antibody with a domain which is binding to serum albumin, the addition of such a domain could probably adversely affect the properties of said bispecific antibody, e.g., it might lose its function or become at least less effective.

Notwithstanding this potential dilemma and bearing in mind that a bispecific antibody of the present invention could at least be weakened or even inactivated by the addition of a further binding which is binding to serum albumin, the present inventors generated bispecific antibody s that have, in addition a first binding domain which is binding to a cell surface molecule on a target cell and a second binding domain which is binding to the T cell CD3 receptor complex, a third binding domain which is binding to serum albumin.

WO 01/45746 which provides serum albumin binding domains leaves it completely up to the skilled person to which terminus of a protein, in particular an antibody, a serum albumin binding domain should be fused - it can either be the N-or C-terminus and may depend on the circumstances. Thus, the prior art does not provide guidance.

To their surprise, they have found that the third binding domain is to be positioned at the C-terminus of the second domain. This is indeed a surprise, since one could more likely have expected that the first binding domain which is binding to a cell surface molecule on a target cell might not be that sensitive as the second domain which engages the "effector function", i.e., a T cell via binding to the T cell receptor complex because of the reasons explained above (formation of a synapse and exerting the killing of a target cell). However, despite this expectation, the present inventors observed that the addition of a serum albumin binding domain to the first binding domain (binding to the target cell) abolished binding of the bispecific antibody.

All the more, prior art bispecific antibody s, such as diabodies with two binding domains, one for CD3 and a second for a target molecule such as CEA, and equipped with a serum albumin binding domain are constructed in a way that the serum albumin binding domain is fused to the domain binding to the target cell; see Stork et al., Prot Eng Des Sel 20(11), 569-576 (2007) and Mueller et al., J Biol Chem 282(17), 12650-12660 (2007). Thus, also from the prior one would have concluded that a serum albumin binding domain should be added to the domain binding to a cell surface molecule of target cell. A similar approach was done in the construction of DART antibodies such as an ABD-DART (see WO 2010/080538, e.g. Figure 45).

That being so, the present inventors, despite the teaching of the prior art and the expectations grasped from the teaching of the prior art, added a serum albumin binding domain to the C-terminus of the second binding domain that engages a T cell via binding to the T cell receptor complex and were successful in the generation of a bispecific antibody that has an increased serum half-life, while it is still binding to a cell surface molecule on a target cell and binding to the T cell CD3 receptor complex, thereby engaging the T cell in a way that it exerts its killing functions on the target cell. Thus, a bispecific antibody of the present invention in which the binding domains are in the order as described herein (see, e.g. claim 1) is mediating cytotoxicity on a target cell that is effected by T cell engaged by said bispecific antibody.

Due to the third binding domain of the bispecific antibody of the present invention, a bispecific antibody of the present invention has preferably an increased half-life and/or longer persistence times in the body, thereby also providing

a longer functional activity of the bispecific antibody.

In addition, the present inventors have observed that a bispecific antibody of the present invention is also mediating cytotoxicity in vitro in the presence of 10% (v/v) serum albumin, in particular human serum albumin. This is an important feature, since in human blood serum albumin is present at about 10-20% (v/v). In fact, a bispecific antibody of the present invention is not-naturally occurring in a mammal, in particular in a human and, thus, it could well have been that the presence of serum albumin could somehow disturb or interfere with the action of a bispecific antibody of the present invention.

By way of example, the in vitro cytotoxicity assay in the presence of serum albumin, in particular human serum albumin can be used to test a bispecific antibody of the present invention for its capability of mediating cytotoxicity.

Another surprising property of a bispecific antibody of the present invention having the order (set-up/arrangement) of the domains as described herein, e.g. in claim 1, can mainly be produced as monomer. In particular, the present inventors found that more than 80, 85 or even 90% of the bispecific antibody obtainable from host cells expressing said bispecific antibody are in the form of a monomer. This is an important feature, since dimers or even multimers are not desirable since they are assumed to have lost most of their binding capabilities to a target cell (via a cell surface molecule) and/or a T cell (via the T cell receptor complex).

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 15%, more preferably within 10%, and most preferably within 5% of a given value or range.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

The term "bispecific antibody" in the sense of the present disclosure indicates any molecule capable of (specifically) binding to, interacting with or recognizing the surface molecule on a target cell and CD3 receptor complex on a T cell. According to the present invention, bispecific antibodies are preferably polypeptides. Such polypeptides may include proteinaceous parts and non-proteinaceous parts (e.g. chemical linkers or chemical crosslinking agents such as glutaraldehyde).

A bispecific antibody, so to say, provides the scaffold for said one or more binding domains so that said binding domains can bind/interact with the surface molecule on a target cell and CD3 receptor complex on a T cell. For example, such a scaffold could be provided by protein A, in particular, the Z-domain thereof (affibodies), ImmE7 (immunity proteins), BPTI/APPI (Kunitz domains), Ras-binding protein AF-6 (PDZ-domains), charybdotoxin (Scorpion toxin), CTLA-4, Min-23 (knottins), lipocalins (anticalins), neokarzinostatin, a fibronectin domain, an ankyrin consensus repeat domain (Stumpp et al., Curr Opin Drug Discov Devel. 10(2), 153-159 (2007)) or thioredoxin (Skerra, Curr. Opin. Biotechnol. 18, 295-304 (2005); Hosse et al., Protein Sci. 15, 14-27 (2006); Nicaise et al., Protein Sci. 13, 1882-1891 (2004); Nygren and Uhlen, Curr. Opin. Struc. Biol. 7, 463-469 (1997)). A preferred bispecific antibody is an antibody, more preferably a bispecific antibody.

The term "cell surface antigen" as used herein denotes a molecule, which is displayed on the surface of a cell. In most cases, this molecule will be located in or on the plasma membrane of the cell such that at least part of this molecule remains accessible from outside the cell in tertiary form. A non-limiting example of a cell surface molecule, which is located in the plasma membrane is a transmembrane protein comprising, in its tertiary conformation, regions of hydrophilicity and hydrophobicity. Here, at least one hydrophobic region allows the cell surface molecule to be embedded, or inserted in the hydrophobic plasma membrane of the cell while the hydrophilic regions extend on either side of the plasma membrane into the cytoplasm and extracellular space, respectively. Non-limiting examples of cell surface

molecules which are located on the plasma membrane are proteins which have been modified at a cysteine residue to bear a palmitoyl group, proteins modified at a C-terminal cysteine residue to bear a farnesyl group or proteins which have been modified at the C-terminus to bear a glycosyl phosphatidyl inositol ("GPI") anchor. These groups allow covalent attachment of proteins to the outer surface of the plasma membrane, where they remain accessible for recognition by extracellular molecules such as antibodies.

The T cell CD3 receptor complex is a protein complex and is composed of four distinct chains. In mammals, the complex contains a CD3γ chain, a CD3δ chain, and two CD3ε (epsilon) chains. These chains associate with a molecule known as the T cell receptor (TCR) and the ζ chain to generate an activation signal in T lymphocytes. A bispecific antibody of the present invention preferably binds via its second domain to the CD3ε (epsilon) chain of the T cell receptor.

The redirected lysis of target cells via the recruitment of T cells by bispecific molecules involves cytolytic synapse formation and delivery of perforin and granzymes. The engaged T cells are capable of serial target cell lysis, and are not affected by immune escape mechanisms interfering with peptide antigen processing and presentation, or clonal T cell differentiation; see, for example, WO 2007/042261.

The term "bispecific" as used herein refers to a bispecific antibody which comprises at least a first and a second binding domain, wherein the first binding domain is capable of binding to one antigen or target, and the second binding domain is capable of binding to another antigen or target. The "bispecific antibody" of the invention also comprises multispecific bispecific antibody s such as e.g. trispecific bispecific antibody s, the latter ones including three binding domains. However, the term "bispecific" when used herein in the context of bispecific antibody s of the present invention is not to be construed as to exclude further binding domains with binding specificity to molecules other than a cell surface molecule on a target cell and CD3 from the bispecific antibody s of the present invention. A further binding domain is the third binding domain of a bispecific antibody of the present invention which is capable of binding to serum albumin.

It is envisaged that the bispecific antibody of the invention has, in addition to its function to bind to the surface molecule on a target cell and the CD3 receptor complex on a T cell and to serum albumin, a further function. In this format, the bispecific antibody is a quad-or multifunctional bispecific antibody by targeting plasma cells through binding to surface molecule on a target cell, mediating cytotoxic T cell activity through CD3 binding and providing a further function such as a label (fluorescent etc.), and/or a therapeutic agent such as, e.g. a toxin or radionuclide, etc.

The term "binding domain" characterizes in connection with the present invention a domain which is capable of specifically binding to / interacting with a given target epitope or a given target site on the surface molecule on a target cell and the CD3 receptor complex on a T cell. Moreover, the term may also characterize the domain binding serum albumin or bound by serum albumin.

Binding domains can be derived from an binding domain donor such as for example an antibody, protein A, ImmE7 (immunity proteins), BPTI/APPI (Kunitz domains), Ras-binding protein AF-6 (PDZ-domains), charybdotoxin (Scorpion toxin), CTLA-4, Min-23 (knottins), lipocalins (anticalins), neokarzinostatin, a fibronectin domain, an ankyrin consensus repeat domain (Stumpp et al., Curr Opin Drug Discov Devel. 10(2), 153-159 (2007)) or thioredoxin (Skerra, Curr. Opin. Biotechnol. 18, 295-304 (2005); Hosse et al., Protein Sci. 15, 14-27 (2006); Nicaise et al., Protein Sci. 13, 1882-1891 (2004) ; Nygren and Uhlen, Curr. Opin. Struc. Biol. 7, 463-469 (1997)). In case of the binding domains binding to the surface molecule on a target cell and the CD3 receptor complex on a T cell a preferred binding domain is derived from an antibody. It is envisaged that a binding domain of the present invention comprises at least said part of any of the aforementioned binding domains that are required for binding to/interacting with a given target epitope or a given target site on the surface molecule on a target cell and the CD3 receptor complex on a T cell.

It is envisaged that the binding domain of the aforementioned binding domain donors is characterized by that part of these donors that is responsible for binding the respective target, i.e. when that part is removed from the binding domain donor, said donor loses its binding capability. "Loses" means a reduction of at least 50% of the binding capability when compared with the binding donor. Methods to map these binding sites are well known in the art - it is therefore within the standard knowledge of the skilled person to locate/map the binding site of a binding domain donor and, thereby, to "derive" said binding domain from the respective binding domain donors.

The terms "(capable of) binding to", "specifically recognizing", "directed to" and "reacting with" mean in accordance with this invention that a binding domain is capable of specifically interacting with one or more, preferably at least two, more preferably at least three and most preferably at least four amino acids of an epitope.

As used herein, the terms "specifically interacting", "specifically binding" or "specifically bind(s)" mean that a binding domain exhibits appreciable affinity for a particular protein or antigen and, generally, does not exhibit significant reactivity with proteins or antigens other than the surface molecule on a target cell and the CD3 receptor complex on a T cell. "Appreciable affinity" includes binding with an affinity of about 10-6M (KD) or stronger. Preferably, binding is considered specific when binding affinity is about 10-11 to 10-8 M, preferably of about 10-11 to 10-9 M. Whether a binding domain specifically reacts with or binds to a target can be tested readily by, inter alia, comparing the reaction of said binding domain with a target protein or antigen with the reaction of said binding domain with proteins or antigens other than the surface molecule on a target cell and the CD3 receptor complex on a T cell. Preferably, a binding domain

of the invention does not essentially bind or is not capable of binding with proteins or antigens other than the surface molecule on a target cell and the CD3 receptor complex on a T cell (i.e. the first binding domain is not capable of binding with proteins other than this surface molecule on a target cell and the second binding domain is not capable of binding with protein other than the CD3 receptor complex on a T cell).

The term "does not essentially bind", "is not capable of binding" means that a binding domain of the present invention does not bind another protein or antigen other than the surface molecule on a target cell or the CD3 receptor complex on a T cell, i.e., does not show reactivity of more than 30%, preferably more than 20%, more preferably more than 10%, particularly preferably more than 9%, 8%, 7%, 6% or 5% with proteins or antigens other than the surface molecule on a target cell or the CD3 receptor complex on a T cell.

Specific binding is believed to be effected by specific motifs in the amino acid sequence of the binding domain and the antigen. Thus, binding is achieved as a result of their primary, secondary and/or tertiary structure as well as the result of secondary modifications of said structures. The specific interaction of the antigen-interaction-site with its specific antigen may result in a simple binding of said site to the antigen. Moreover, the specific interaction of the antigen-interaction-site with its specific antigen may alternatively or additionally result in the initiation of a signal, e.g. due to the induction of a change of the conformation of the antigen, an oligomerization of the antigen, etc.

Proteins (including fragments thereof, preferably biologically active fragments, and peptides, usually having less than 30 amino acids) comprise one or more amino acids coupled to each other via a covalent peptide bond (resulting in a chain of amino acids). The term "polypeptide" as used herein describes a group of molecules, which consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a hereteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. A "polypeptide" when referred to herein may also be chemically modified such as pegylated. Such modifications are well known in the art.

The term "epitope" refers to a site on an antigen to which a binding domain, such as an antibody or immunoglobulin or derivative or fragment of an antibody or of an immunoglobulin, specifically binds. An "epitope" is antigenic and thus the term epitope is sometimes also referred to herein as "antigenic structure" or "antigenic determinant". Thus, the binding domain is an "antigen-interaction-site". Said binding/interaction is also understood to define a "specific recognition". In one example, said binding domain which (specifically) binds to / interacts with a given target epitope or a given target site on the surface molecule on a target cell and the CD3 receptor complex on a T cell is an antibody or immunoglobulin, and said binding domain is a VH and/or VL region of an antibody or of an immunoglobulin.

"Epitopes" can be formed both by contiguous amino acids or non-contiguous amino acids juxtaposed by tertiary folding of a protein. A "linear epitope" is an epitope where an amino acid primary sequence comprises the recognized epitope. A linear epitope typically includes at least 3 or at least 4, and more usually, at least 5 or at least 6 or at least 7, for example, about 8 to about 10 amino acids in a unique sequence.

A "conformational epitope", in contrast to a linear epitope, is an epitope wherein the primary sequence of the amino acids comprising the epitope is not the sole defining component of the epitope recognized (e.g., an epitope wherein the primary sequence of amino acids is not necessarily recognized by the binding domain). Typically a conformational epitope comprises an increased number of amino acids relative to a linear epitope. With regard to recognition of conformational epitopes, the binding domain recognizes a three-dimensional structure of the antigen, preferably a peptide or protein or fragment thereof (in the context of the present invention, the antigen for one of the binding domains is comprised within the surface molecule on a target cell and the CD3 receptor complex on a T cell). For example, when a protein molecule folds to form a three-dimensional structure, certain amino acids and/or the polypeptide backbone forming the conformational epitope become juxtaposed enabling the antibody to recognize the epitope. Methods of determining the conformation of epitopes include, but are not limited to, x-ray crystallography, two-dimensional nuclear magnetic resonance (2D-NMR) spectroscopy and site-directed spin labelling and electron paramagnetic resonance (EPR) spectroscopy.

The term "albumin binding domain" (ABP) characterizes a sequence motive within the bispecific antibody of the invention that mediates a specific binding to serum albumin from multiple species with high affinity.

Non-limiting examples of ABPs are shown in Table 1.

Table 1: Non-limiting examples of ABP motives

| Peptide | Sequence |
|---|---|
| SA04 | DICLPRWGCLW |
| SA08 | QGLIGDICLPRWGCLWGDSVK |

(continued)

| Peptide | Sequence |
|---|---|
| SA21 | RLIEDICLPRWGCLWEDD |
| SA25 | EDICLPRWGCLWED |
| DX236 | AEGTGDFWFCDRIAWYPQHLCEFLDPE |
| DX321 | AEGTGDRNMCKFSWIRSPAFCARADPE |
| AB01 | AASYSDYDVFGGGTDFGP |
| AB14 | AARYWDYDVFGGGTPVGG |
| AB156 | AARDWDFDVFGGGTPVGG |

The bispecific antibodies of the invention the three domains are on one polypeptide in the order from the N-terminus to the C-terminus

- the first binding domain;
- the second binding domain; and
- the third binding domain.

Also in one embodiment of the invention the third binding domain of the bispecific antibody of the invention is an scFv or single domain antibody.

In one aspect, the bispecific antibody of the present invention comprises three binding domains, wherein

(a) the first binding domain is a domain which is binding to the cell surface molecule on a human and a non-human primate cell;
(b) the second binding domain is a domain which is binding to the T cell CD3 receptor complex on a human and a non-human primate cell, and
(c) the third binding domain is a domain which is binding to human and non-human primate serum albumin.

The term "non-human primate" characterizes the group of all species of the order primate excl uding humans. Thus, the group comprises explicitly the families of Callitrichidae (marmosets and tamarins), Cebidae, Cercopithecidae (Old World Monkeys), Hylobatidae (lesser apes, gibbons) and Hominoidea (great apes). It is also envisaged that the third binding domain is a domain which is binding to human and at least one non-human primate serum albumin, preferably to Cercopithecidae (Old World Monkeys). It is further envisaged that the third binding domain is a domain which is binding to human and at least one non-human primate serum albumin, preferably to Cercopithecidae (Old World Monkeys), and additionally to a rodent serum albumin, such as mouse or rat (see Example 3).

The term "cross-species specificity" or "interspecies specificity" as used herein means binding of a binding domain described herein to the same target molecule in humans and non-human primates. Thus, "cross-species specificity" or "interspecies specificity" is to be understood as an interspecies reactivity to the same molecule X expressed in different species, but not to a molecule other than X. Cross-species specificity of a monoclonal antibody recognizing e.g. human CD3 epsilon, to a non-human primate CD3 epsilon, e.g. macaque CD3 epsilon, can be determined, for instance, by FACS analysis. The FACS analysis is carried out in a way that the respective monoclonal antibody is tested for binding to human and non-human primate cells, e.g. macaque cells, expressing said human and non-human primate CD3 epsilon antigens, respectively. An appropriate assay is shown in the following examples.

For the generation of a binding domain for the bispecific antibody of the invention, e.g. bispecific single chain antibodies as defined herein, monoclonal antibodies binding to both of the respective human and/or non-human primate cell surface antigens can be utilized. Appropriate binding domains for the bispecific polypeptide as defined herein e.g. can be derived from cross-species specific monoclonal antibodies by recombinant methods described in the art. A monoclonal antibody binding to a human cell surface antigen and to the homolog of said cell surface antigen in a non-chimpanzee primate can be tested by FACS assays as set forth above. It is evident to those skilled in the art that cross-species specific antibodies can also be generated by hybridoma techniques described in the literature (Milstein and Köhler, Nature 256 (1975), 495-7). For example, mice may be alternately immunized with human and non-human primate antigen. From these mice, cross-species specific antibody-producing hybridoma cells are isolated via hybridoma technology and analysed by FACS as set forth above. The generation and analysis of bispecific polypeptides such as bispecific single chain antibodies exhibiting cross-species specificity as described herein is shown in the following examples. The advantages of the bispecific single chain antibodies exhibiting cross-species specificity include the points enumerated below.

Moreover, in one aspect of the bispecific antibody of the present invention the third binding domain binding to

serum albumin is derived from a combinatorial library or an antibody binding domain.

As used herein the term "combinatorial library" defines a library of nucleic acid molecules, derived by e.g. gene synthesis using NNS triplets or other methods known to the expert, which code for a deduced set of amino acid sequences varying in one or more amino acid positions with the aim to select or screen for molecules with a specific functionality such as binding to a protein of interest.

The definition of the term "antibody" includes embodiments such as monoclonal, chimeric, single chain, humanized and human antibodies. In addition to full-length antibodies, the definition also includes antibody derivatives and antibody fragments, like, inter alia, Fab fragments. Antibody fragments or derivatives further comprise F(ab')2, Fv, scFv fragments or single domain antibodies such as domain antibodies or nanobodies, single variable domain antibodies or immunoglobulin single variable domain comprising merely one variable domain, which might be VHH, VH or VL, that specifically bind an antigen or epitope independently of other V regions or domains; see, for example, Harlow and Lane (1988) and (1999), loc. cit.; Kontermann and Dübel, Antibody Engineering, Springer, 2nd ed. 2010 and Little, Recombinant Antibodies for Immunotherapy, Cambridge University Press 2009. Said term also includes diabodies or Dual-Affinity Re-Targeting (DART) antibodies. immunoglobulin single variable domains encompass not only an isolated antibody single variable domain polypeptide, but also larger polypeptides that comprise one or more monomers of an antibody single variable domain polypeptide sequence.

Various procedures are known in the art and may be used for the production of such antibodies and/or fragments. Thus, (antibody) derivatives can be produced by peptidomimetics. Further, techniques described for the production of single chain antibodies (see, inter alia, US Patent 4,946,778, Kontermann and Dübel (2010), loc. cit. and Little (2009), loc. cit.) can be adapted to produce single chain antibodies specific for elected polypeptide(s). Also, transgenic animals may be used to express humanized antibodies specific for polypeptides and fusion proteins of this invention. For the preparation of monoclonal antibodies, any technique, providing antibodies produced by continuous cell line cultures can be used. Examples for such techniques include the hybridoma technique (Köhler and Milstein Nature 256 (1975), 495-497), the trioma technique, the human B cell hybridoma technique (Kozbor, Immunology Today 4 (1983), 72) and the EBV hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of a target polypeptide, such as CD3 epsilon (Schier, Human Antibodies Hybridomas 7 (1996), 97-105; Malmborg, J. Immunol. Methods 183 (1995), 7-13). It is also envisaged in the context of this invention that the term "antibody" comprises antibody constructs, which may be expressed in a host as described herein below, e.g. antibody constructs which may be transfected and/or transduced via, inter alia, viruses or plasmid vectors.

Furthermore, the term "antibody" as employed herein also relates to derivatives or variants of the antibodies described herein which display the same specificity as the described antibodies. Examples of "antibody variants" include humanized variants of non-human antibodies, "affinity matured" antibodies (see, e.g. Hawkins et al. J. Mol. Biol. 254, 889-896 (1992) and Lowman et al., Biochemistry 30, 10832- 10837 (1991)) and antibody mutants with altered effector function(s) (see, e.g., US Patent 5, 648, 260, Kontermann and Dübel (2010), loc. cit. and Little(2009), loc. cit.).

The terms "antigen-binding domain", "antigen-binding fragment" and "antibody binding region" when used herein refer to a part of an antibody molecule that comprises amino acids responsible for the specific binding between antibody and antigen. The part of the antigen that is specifically recognized and bound by the antibody is referred to as the "epitope" as described herein above. As mentioned above, an antigen-binding domain may typically comprise an antibody light chain variable region (VL) and an antibody heavy chain variable region (VH); however, it does not have to comprise both. Fd fragments, for example, have two VH regions and often retain some antigen-binding function of the intact antigen-binding domain. Examples of antigen-binding fragments of an antibody include (1) a Fab fragment, a monovalent fragment having the VL, VH, CL and CH1 domains; (2) a F(ab')2 fragment, a bivalent fragment having two Fab fragments linked by a disulfide bridge at the hinge region; (3) an Fd fragment having the two VH and CH1 domains; (4) an Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv). Although the two domains of the Fv fragment, VL and VH are coded for by separate genes, they can be joined, using recombinant methods, by a synthetic linker that enables them to be made as a single protein chain in which the VL and VH regions pair to form monovalent molecules (known as single chain Fv (scFv); see e.g., Huston et al. (1988) Proc. Natl. Acad. Sci USA 85:5879-5883). These antibody fragments are obtained using conventional techniques known to those with skill in the art, and the fragments are evaluated for function in the same manner as are intact antibodies.

In the event that a (synthetic) linker is used, this linker is preferably of a length and sequence sufficient to ensure that each of the first and second domains can, independently from one another, retain their differential binding specificities. Most preferably and as documented in the appended examples, the antibody construct of the invention is a "bispecific single chain antibody construct", more preferably a bispecific single chain Fv (scFv). Bispecific single chain molecules are known in the art and are described in WO 99/54440, Mack, J. Immunol. (1997), 158, 3965-3970, Mack, PNAS,

(1995), 92, 7021-7025, Kufer, Cancer Immunol. Immunother., (1997), 45, 193-197, Löffler, Blood, (2000), 95, 6, 2098-2103, Brühl, Immunol., (2001), 166, 2420-2426, Kipriyanov, J. Mol. Biol., (1999), 293,41-56.

The said variable domains comprised in the herein described antibody constructs may be connected by additional linker sequences. The term "peptide linker" defines in accordance with the present invention an amino acid sequence by which the amino acid sequences of the first domain and the second domain of the antibody construct of the invention are linked with each other. An essential technical feature of such peptide linker is that said peptide linker does not comprise any polymerization activity. Preferred amino acid residues for a peptide linker include Gly, Ser and and Thr are characterized by a length between 5 and 25 amino acid residues. Among the suitable peptide linkers are those described in U.S. Patents 4,751,180 and 4,935,233 or WO 88/09344. A preferred embodiment of a peptide linker is characterized by the amino acid sequence Gly-Gly-Gly-Gly-Ser, i.e. $Gly_4Ser$, or polymers thereof, i.e. $(Gly_4Ser)x$, where x is an integer 1 or greater. The characteristics of said peptide linker, which comprise the absence of the promotion of secondary structures are known in the art and described e.g. in Dall'Acqua et al. (Biochem. (1998) 37, 9266-9273), Cheadle et al. (Mol Immunol (1992) 29, 21-30) and Raag and Whitlow (FASEB (1995) 9(1), 73-80). Peptide linkers which also do not promote any secondary structures are preferred. The linkage of said domains to each other can be provided by, e.g. genetic engineering, as described in the examples. Methods for preparing fused and operatively linked bispecific single chain constructs and expressing them in mammalian cells or bacteria are well-known in the art (e.g. WO 99/54440 or Sambrook et al., Molecular Cloning: A Laboratory Manual, Cold Spring Harbor Laboratory Press, Cold Spring Harbor, New York, 2001).

For peptide linkers, which connect the at least two binding domains in the antibody construct of the invention peptide linkers are preferred which comprise only a few number of amino acid residues, e.g. 12 amino acid residues or less. Thus, peptide linker of 12, 11, 10, 9, 8, 7, 6 or 5 amino acid residues are preferred. An envisaged peptide linker with less than 5 amino acids comprises 4, 3, 2 or one amino acid(s) wherein Gly-rich linkers are preferred. A particularly preferred "single" amino acid in context of said "peptide linker" is Gly. Accordingly, said peptide linker may consist of the single amino acid Gly.

The term "monoclonal antibody" as used herein refers to an antibody obtained from a population of substantially homogeneous antibodies, i.e., the individual antibodies comprising the population are identical except for possible naturally occurring mutations and/or post- translation modifications (e.g., isomerizations, amidations) that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256: 495 (1975), or may be made by recombinant DNA methods (see, e.g., U.S. Patent No. 4,816,567). The "monoclonal antibodies" may also be isolated from phage antibody libraries using the techniques described in Clackson et al., Nature, 352: 624-628 (1991) and Marks et al., J. Mol. Biol., 222: 581-597 (1991), for example.

The monoclonal antibodies of the present invention specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain (s) is (are) identical with or homologous to corresponding sequences in antibodies derived from another species or belonging to another antibody class or subclass, as well as fragments of such antibodies, so long as they exhibit the desired biological activity (U. S. Patent No. 4,816, 567; Morrison et al., Proc. Natl. Acad. Sci. USA, 81: 6851-6855 (1984)). Chimeric antibodies of interest herein include "primatized" antibodies comprising variable domain antigen-binding sequences derived from a non-human primate (e.g., Old World Monkey, Ape etc.) and human constant region sequences.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) of mostly human sequences, which contain minimal sequence derived from non-human immunoglobulin. For the most part, humanized antibodies are human immunoglobulins (recipient antibody) in which residues from a hypervariable region (also CDR) of the recipient are replaced by residues from a hypervariable region of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, "humanized antibodies" as used herein may also comprise residues which are found neither in the recipient antibody nor the donor antibody. These modifications are made to further refine and optimize antibody performance. The humanized antibody optimally also will comprise at least a portion of an immunoglobulin constant region (Fc), typically that of a human immunoglobulin. For further details, see Jones et al., Nature, 321: 522-525 (1986); Reichmann et al., Nature, 332: 323-329 (1988); and Presta, Curr. Op. Struct. Biol., 2: 593-596 (1992).

The term "human antibody" includes antibodies having variable and constant regions corresponding substantially to human germline immunoglobulin sequences known in the art, including, for example, those described by Kabat et al. (See Kabat et al. (1991) loc. cit.). Human antibodies of the invention may include amino acid residues not encoded by human germline immunoglobulin sequences (e.g., mutations introduced by random or site-specific mutagenesis in vitro or by somatic mutation in vivo), for example in the CDRs, and in particular, CDR3. The human antibody can have at least one, two, three, four, five, or more positions replaced with an amino acid residue that is not encoded by the human germline immunoglobulin sequence.

As used herein, "in vitro generated antibody" refers to an antibody where all or part of the variable region (e.g., at least one CDR) is generated in a non-immune cell selection (e.g., an in vitro phage display, protein chip or any other method in which candidate sequences can be tested for their ability to bind to an antigen). This term thus preferably excludes sequences generated by genomic rearrangement in an immune cell.

In one preferred aspect of the invention the third domain binding to serum albumin is derived from a CDR of a single domain antibody. In a preferred aspect of the invention, the third domain binding to serum albumin is not of bacterial origin such as an albumin binding domain from protein G from Streptococcus, such as the ABD3 domain of protein G of Streptococcus strain G418.

In one aspect of the bispecific antibody of the invention the third binding domain comprises between 10 and 25 aa residues.

In a preferred aspect of the invention the third binding domain binding to serum albumin comprises the amino acid sequence Asp-Xaa-Cys-Leu-Pro-Xaa-Trp-Gly-Cys-Leu-Trp, wherein Xaa is any amino acid.

Furthermore, in one aspect of the bispecific antibody of the invention the third binding domain is binding to serum albumin with an affinity (KD) of $\leq$ 500 nM.

The in vivo half-life of the Fab-fragment 4D5, C-terminally tagged with various ABPs mediating different affinities to albumin was investigated in mice, rats and rabbits (Nguyen et al., Prot Eng Des Sel 19(7), 291-297 (2006)). Based on the half-life values determined in these different small species in combination with the Fab-ABP-affinities to murine, rat and rabbit albumin, the beta half-life of an Fab-ABP with 500nM affinity to human albumin was calculated to be 4 days in a 70kg human being. It is anticipated that the same is true for an ABP-tagged bispecific antibodys of the invention with an affinity to human albumin of 500nM, because a bispecific antibodies of the invention such as a BiTE® (bispecific T cell engager) has the same molecular weight as a Fab-fragment and - like a Fab-fragment - consists of a total of 4 immunoglobulin domains.

The same investigations confirmed that higher affinity to albumin (i.e. smaller KD-value) results in longer in vivo half-life. Therefore, an ABP-tagged bispecific antibodys of the invention with an affinity to human albumin of 500nM is expected to have a half-life of 4 days in humans. An ABP-tagged bispecific antibodies of the invention with an affinity to human albumin of <500nM is expected to have a half-life of >4 days in humans. Likewise, an ABP-tagged bispecific antibodies of the invention with an affinity to human albumin of >500nM is expected to have a half-life of <4 days in humans.

As mentioned above, a bispecific antibody of the present invention has preferably an increased serum half-life.

Methods for pharmacokinetic analysis and determination of ligand half-life will be familiar to those skilled in the art. Details may be found in Kennetl, A et al. Chemical Stability of Pharmaceuticals: A Handbook for Pharmacists and in Peters et al, Pharmacokinetc analysis: A Practical Approach (1996). Reference is also made to "Pharmacokinetics", M Gibaldi & D Perron, published by Marcel Dekker, 2nd Rev. ex edition (1982), which describes pharmacokinetic parameters such as t alpha and t beta half-lifes and area under the curve (AUC).

Half-life can be defined as the time taken for the serum concentration of the polypeptide to be reduced by 50%, in vivo, for example due to degradation of the bispecific antibody and/or clearance or sequestration of the bispecific antibody by natural mechanisms. Methods for pharmacokinetic analysis and determination of half-life are familiar to those skilled in the art. Details may be found in Kenneth, A et al. Chemical Stability of Pharmaceuticals: A Handbook for Pharmacists and in Peters et al, Pharmacokinete analysis: A Practical Approach (1996). Reference is also made to "Pharmacokinetics", M Gibaldi & D Perron, published by Marcel Dekker, 2nd revised edition (1982).

It is preferred that a bispecific antibody described herein preferably has a half-life that is at least 1.5 times, preferably at least 2 times, such as at least 5 times, for example at least 10 times or more than 20 times, greater than the half-life of the corresponding therapeutic moiety (i.e., a bispecific antibody not comprising the third domain as described herein) per se.

Also, preferably, any such bispecific antibody has a half-life that is increased with more than 1 hour, preferably more than 2 hours, more preferably of more than 6 hours, such as of more than 12 hours, compared to the half-life of the corresponding therapeutic moiety per se.

Also, preferably, any such bispecific antibody has a half-life that is more than 1 hour, preferably more than 2 hours, more preferably of more than 6 hours, such as of more than 12 hours, and for example of about one day, two days, one week, two weeks or three weeks, and preferably no more than 2 months, although the latter may be less critical, compared to the half-life of the corresponding therapeutic moiety per se.

In addition, or alternatively to the above criteria, the present invention provides a ligand or a composition

comprising a bispecific antibody according to the invention having an AUC value (area under the curve) in the range of 1 mg.min/ml or more. In one embodiment, the lower end of the range is 5, 10, 15, 20, 30,100, 200 or 300 mg. min/ml. In addition, or alternatively, a bispecific antibody according to the invention has an AUC in the range of up to 600 mg. min/ml. In one embodiment, the upper end of the range is 500, 400, 300, 200, 150, 100, 75 or 50 mg.min/ml. Advantageously a bispecific antibody according to the invention will have a AUC in the range selected from the group consisting of the following: 15 to 150 mg.min/ml, 15 to 100 mg. min/ml, 15 to 75 mg.min/ml, and 15 to 50 mg. min/ml.

In one additional aspect of the invention the bispecific antibody shows cytotoxic activity in an in vitro assay measuring the lysis of target cells by effector cells in the presence of 10% human serum albumin.

The cytotoxic activity of a bispecific antibody of the invention is provided by the engagement of a cytotoxic T cell (via the binding domain binding to the T cell CD3 receptor complex on the surface of T cells) and a target cells (via the binding to a cell surface molecule on a target cell). The appended example 2 provides a description of a suitable in vitro assay for testing the cytotoxic activity of the bispecific antibody of the invention in the presence of 10% human serum albumin.

In a preferred aspect of the present invention the bispecific antibody consists of a single polypeptide chain. A non-limiting example for such an single chain bispecific bispecific antibody is the format of the bispecific or bifunctional antibody or immunoglobulin described herein below.

An also preferred aspect of the invention relates to a binding molecule, wherein

(a) the first binding domain comprises an antibody derived VL and VH chain; and/or
(b) the second binding domain comprises an antibody derived VL and VH chain.

Preferably, a "bispecific" or "bifunctional" antibody or immunoglobulin is an artificial hybrid antibody or immunoglobulin having two different heavy/light chain pairs and two different binding sites. Bispecific antibodies can be produced by a variety of methods including fusion of hybridomas or linking of Fab' fragments. See, e.g., Songsivilai & Lachmann, Clin. Exp. Immunol. 79:315-321 (1990). Numerous methods known to those skilled in the art are available for obtaining antibodies or antigen-binding fragments thereof. For example, antibodies can be produced using recombinant DNA methods (U.S. Patent No. 4,816,567). Monoclonal antibodies may also be produced by generation of hybridomas (see e.g., Kohler and Milstein (1975) Nature, 256: 495-499) in accordance with known methods. Hybridomas formed in this manner are then screened using standard methods, such as enzyme-linked immunosorbent assay (ELISA) and surface plasmon resonance (BIACORE™) analysis, to identify one or more hybridomas that produce an antibody that specifically binds with a specified antigen. Any form of the specified antigen may be used as the immunogen, e.g., recombinant antigen, naturally occurring forms, any variants or fragments thereof, as well as antigenic peptide thereof."

One exemplary method of making antibodies includes screening protein expression libraries, e.g., phage or ribosome display libraries. Phage display is described, for example, in Ladner et al., U.S. Patent No. 5,223,409; Smith (1985) Science 228:1315-1317; Clackson et al. (1991) Nature, 352: 624-628.

In addition to the use of display libraries, the specified antigen can be used to immunize a non-human animal, e.g., a rodent, e.g., a mouse, hamster, or rat. In one embodiment, the non-human animal includes at least a part of a human immunoglobulin gene. For example, it is possible to engineer mouse strains deficient in mouse antibody production with large fragments of the human Ig loci. Using the hybridoma technology, antigen-specific monoclonal antibodies derived from the genes with the desired specificity may be produced and selected. See, e.g., XENOMOUSE™, Green et al. (1994) Nature Genetics 7:13-21, US 2003- 0070185, WO 96/34096, and WO96/33735.

A monoclonal antibody can be obtained from a non-human animal, and then modified, e.g., humanized, deimmunized, chimeric, may be produced using recombinant DNA techniques known in the art. A variety of approaches for making chimeric antibodies have been described. See e.g., Morrison et al., Proc. Natl. Acad. Sci U.S.A. 81:6851, 1985; Takeda et al., Nature 314:452, 1985, Cabilly et al., U.S. Patent No. 4,816,567; Boss et al., U.S. Patent No. 4,816,397; Tanaguchi et al., EP 0171496; EP 0173494, GB 2177096. Humanized antibodies may also be produced, for example, using transgenic mice that express human heavy and light chain genes, but are incapable of expressing the endogenous mouse immunoglobulin heavy and light chain genes. Winter describes an exemplary CDR-grafting method that may be used to prepare the humanized antibodies described herein (U.S. Patent No. 5,225,539). All of the CDRs of a particular human antibody may be replaced with at least a portion of a non-human CDR, or only some of the CDRs may be replaced with non-human CDRs. It is only necessary to replace the number of CDRs required for binding of the humanized antibody to a predetermined antigen.

Humanized antibodies or fragments thereof can be generated by replacing sequences of the Fv variable domain that are not directly involved in antigen binding with equivalent sequences from human Fv variable domains. Exemplary methods for generating humanized antibodies or fragments thereof are provided by Morrison (1985) Science 229:1202-1207; by Oi et al. (1986) BioTechniques 4:214; and by US 5,585,089; US 5,693,761; US 5,693,762; US 5,859,205; and US 6,407,213. Those methods include isolating, manipulating, and expressing the nucleic acid sequences that encode all or part of immunoglobulin Fv variable domains from at least one of a heavy or light chain. Such nucleic

acids may be obtained from a hybridoma producing an antibody against a predetermined target, as described above, as well as from other sources. The recombinant DNA encoding the humanized antibody molecule can then be cloned into an appropriate expression vector.

A humanized antibody can be optimized by the introduction of conservative substitutions, consensus sequence substitutions, germline substitutions and/or back mutations. Such altered immunoglobulin molecules can be made by any of several techniques known in the art, (e.g., Teng et al., Proc. Natl. Acad. Sci. U.S.A., 80: 7308-7312, 1983; Kozbor et al., Immunology Today, 4: 7279, 1983; Olsson et al., Meth. Enzymol., 92: 3-16, 1982), and may be made according to the teachings of EP 239 400.

An antibody or fragment thereof may also be modified by specific deletion of human T cell epitopes or "deimmunization" by the methods disclosed in WO 98/52976 and WO 00/34317. Briefly, the heavy and light chain variable domains of an antibody can be analyzed for peptides that bind to MHC class II; these peptides represent potential T cell epitopes (as defined in WO 98/52976 and WO 00/34317). For detection of potential T cell epitopes, a computer modeling approach termed "peptide threading" can be applied, and in addition a database of human MHC class II binding peptides can be searched for motifs present in the VH and VL sequences, as described in WO 98/52976 and WO 00/34317. These motifs bind to any of the 18 major MHC class II DR allotypes, and thus constitute potential T cell epitopes. Potential T-cell epitopes detected can be eliminated by substituting small numbers of amino acid residues in the variable domains, or preferably, by single amino acid substitutions. Typically, conservative substitutions are made. Often, but not exclusively, an amino acid common to a position in human germline antibody sequences may be used. Human germline sequences, e.g., are disclosed in Tomlinson, et al. (1992) J. Mol. Biol. 227:776-798; Cook, G.P. et al. (1995) Immunol. Today Vol. 16 (5): 237-242; and Tomlinson et al. (1995) EMBO J. 14: 14:4628-4638. The V BASE directory provides a comprehensive directory of human immunoglobulin variable region sequences (compiled by Tomlinson, LA. et al. MRC Centre for Protein Engineering, Cambridge, UK). These sequences can be used as a source of human sequence, e.g., for framework regions and CDRs. Consensus human framework regions can also be used, e.g., as described in US Patent No. 6,300,064.

The pairing of a VH and VL together forms a single antigen-binding site. The CH domain most proximal to VH is designated as CH1. Each L chain is linked to an H chain by one covalent disulfide bond, while the two H chains are linked to each other by one or more disulfide bonds depending on the H chain isotype. The VH and VL domains consist of regions of relatively conserved sequences called framework regions (FR1, FR2, FR3, and FR4), which form a scaffold for three regions of hypervariable sequences (complementarity determining regions, CDRs). The CDRs contain most of the residues responsible for specific interactions of the antibody with the antigen. CDRs are referred to as CDR 1, CDR2, and CDR3. Accordingly, CDR constituents on the heavy chain are referred to as H1, H2, and H3, while CDR constituents on the light chain are referred to as L1, L2, and L3.

The term "variable" refers to the portions of the immunoglobulin domains that exhibit variability in their sequence and that are involved in determining the specificity and binding affinity of a particular antibody (i.e., the "variable domain(s)"). Variability is not evenly distributed throughout the variable domains of antibodies; it is concentrated in subdomains of each of the heavy and light chain variable regions. These sub-domains are called "hypervariable" regions or "complementarity determining regions" (CDRs). The more conserved (i.e., non-hypervariable) portions of the variable domains are called the "framework" regions (FRM). The variable domains of naturally occurring heavy and light chains each comprise FRM regions, largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding site (see Kabat et al., loc. cit.). The constant domains are not directly involved in antigen binding, but exhibit various effector functions, such as, for example, antibody-dependent, cell-mediated cytotoxicity and complement activation.

It is also preferred for the bispecific antibody of the invention that first and the second domain form a molecule that is selected from the group of (scFv)2, (single domain mAb)2, scFv-single domain mAb, diabody or oligomeres thereof.

The terms "CDR", and its plural "CDRs", refer to a complementarity determining region (CDR) of which three make up the binding character of a light chain variable region (CDRL1, CDRL2 and CDRL3) and three make up the binding character of a heavy chain variable region (CDRH1, CDRH2 and CDRH3). CDRs contribute to the functional activity of an antibody molecule and are separated by amino acid sequences that comprise scaffolding or framework regions. The exact definitional CDR boundaries and lengths are subject to different classification and numbering systems. CDRs may therefore be referred to by Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example Kabat, Chothia, and/or MacCallum (Kabat et al., loc. cit.; Chothia et al., J. Mol. Biol, 1987, 196: 901; and MacCallum et al., J. Mol. Biol, 1996, 262: 732). However, the numbering in accordance with the so-called Kabat system is preferred.

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gln, Gly, His, Met, Phe, Ser, Thr, Trp, and Tyr).

The term "hypervariable region" (also known as "complementarity determining regions" or CDRs) when used herein refers to the amino acid residues of an antibody which are (usually three or short regions of extreme sequence variability) within the V-region domain of an immunoglobulin which form the antigen-binding site and are the main determinants of antigen specificity. There are at least two methods for identifying the CDR residues: (1) An approach based on cross-species sequence variability (i. e., Kabat et al., loc. cit.); and (2) An approach based on crystallographic studies of antigen-antibody complexes (Chothia, C. et al., J. Mol. Biol. 196: 901-917 (1987)). However, to the extent that two residue identification techniques define regions of overlapping, but not identical regions, they can be combined to define a hybrid CDR. However, in general, the CDR residues are preferably identified in accordance with the so-called Kabat (numbering) system.

The term "framework region" refers to the art-recognized portions of an antibody variable region that exist between the more divergent (i.e., hypervariable) CDRs. Such framework regions are typically referred to as frameworks 1 through 4 (FR1, FR2, FR3, and FR4) and provide a scaffold for the presentation of the six CDRs (three from the heavy chain and three from the light chain) in three dimensional space, to form an antigen-binding surface.

Typically, CDRs form a loop structure that can be classified as a canonical structure. The term "canonical structure" refers to the main chain conformation that is adopted by the antigen binding (CDR) loops. From comparative structural studies, it has been found that five of the six antigen binding loops have only a limited repertoire of available conformations. Each canonical structure can be characterized by the torsion angles of the polypeptide backbone. Correspondent loops between antibodies may, therefore, have very similar three dimensional structures, despite high amino acid sequence variability in most parts of the loops (Chothia and Lesk, J. Mol. Biol., 1987, 196: 901; Chothia et al., Nature, 1989, 342: 877; Martin and Thornton, J. Mol. Biol, 1996, 263: 800). Furthermore, there is a relationship between the adopted loop structure and the amino acid sequences surrounding it. The conformation of a particular canonical class is determined by the length of the loop and the amino acid residues residing at key positions within the loop, as well as within the conserved framework (i.e., outside of the loop). Assignment to a particular canonical class can therefore be made based on the presence of these key amino acid residues. The term "canonical structure" may also include considerations as to the linear sequence of the antibody, for example, as catalogued by Kabat (Kabat et al., loc. cit.). The Kabat numbering scheme (system) is a widely adopted standard for numbering the amino acid residues of an antibody variable domain in a consistent manner and is the preferred scheme applied in the present invention as also mentioned elsewhere herein. Additional structural considerations can also be used to determine the canonical structure of an antibody. For example, those differences not fully reflected by Kabat numbering can be described by the numbering system of Chothia et al and/or revealed by other techniques, for example, crystallography and two or three-dimensional computational modeling. Accordingly, a given antibody sequence may be placed into a canonical class which allows for, among other things, identifying appropriate chassis sequences (e.g., based on a desire to include a variety of canonical structures in a library). Kabat numbering of antibody amino acid sequences and structural considerations as described by Chothia et al., loc. cit. and their implications for construing canonical aspects of antibody structure, are described in the literature.

CDR3 is typically the greatest source of molecular diversity within the antibody-binding site. H3, for example, can be as short as two amino acid residues or greater than 26 amino acids. The subunit structures and three-dimensional configurations of different classes of immunoglobulins are well known in the art. For a review of the antibody structure, see Antibodies: A Laboratory Manual, Cold Spring Harbor Laboratory, eds. Harlow et al., 1988. One of skill in the art will recognize that each subunit structure, e.g., a CH, VH, CL, VL, CDR, FR structure, comprises active fragments, e.g., the portion of the VH, VL, or CDR subunit the binds to the antigen, i.e., the antigen-binding fragment, or, e.g., the portion of the CH subunit that binds to and/or activates, e.g., an Fc receptor and/or complement. The CDRs typically refer to the Kabat CDRs, as described in Sequences of Proteins of immunological Interest, US Department of Health and Human Services (1991), eds. Kabat et al. Another standard for characterizing the antigen binding site is to refer to the hypervariable loops as described by Chothia. See, e.g., Chothia, et al. (1987; J. Mol. Biol. 227:799-817); and Tomlinson et al. (1995) EMBO J. 14: 4628-4638. Still another standard is the AbM definition used by Oxford Molecular's AbM antibody modeling software. See, generally, e.g., Protein Sequence and Structure Analysis of Antibody Variable Domains. In: Antibody Engineering Lab Manual (Ed.: Duebel, S. and Kontermann, R., Springer-Verlag, Heidelberg). Embodiments described with respect to Kabat CDRs can alternatively be implemented using similar described relationships with respect

to Chothia hypervariable loops or to the AbM-defined loops.

The sequence of antibody genes after assembly and somatic mutation is highly varied, and these varied genes are estimated to encode 1010 different antibody molecules (Immunoglobulin Genes, 2nd ed., eds. Jonio et al., Academic Press, San Diego, CA, 1995). Accordingly, the immune system provides a repertoire of immunoglobulins. The term "repertoire" refers to at least one nucleotide sequence derived wholly or partially from at least one sequence encoding at least one immunoglobulin. The sequence(s) may be generated by rearrangement in vivo of the V, D, and J segments of heavy chains, and the V and J segments of light chains. Alternatively, the sequence(s) can be generated from a cell in response to which rearrangement occurs, e.g., in vitro stimulation. Alternatively, part or all of the sequence(s) may be obtained by DNA splicing, nucleotide synthesis, mutagenesis, and other methods, see, e.g., U.S. Patent 5,565,332. A repertoire may include only one sequence or may include a plurality of sequences, including ones in a genetically diverse collection.

In a further preferred aspect of the invention the VL and VH chain of the first and/or the second binding domain are humanized, deimmunized and/or otherwise optimized with regard to their immunogenicity or binding affinity.

In one aspect of the bispecific antibody of the invention the bispecific antibody comprises one or more further heterologous polypeptide. "Heterologous" when used in this context means that the amino acid sequence of the heterologous polypeptide sequence is different from the amino acid sequence of the bispecific antibody of the present invention which comprises said further heterologous polypeptide. The heterologous polypeptide is preferably selected from the group consisting of a HA-tag, myc6-tag, flag-tag, strep-tag, strepII-tag, TAP-tag, HAT-tag, chitin binding domain (CBD), maltose-binding protein, immunoglobulin A (IgA), His-6-tag, glutathione-S-transferase (GST) tag, intein, streptavidine binding protein (SBP) tag, Strep-tag and StrepII tag. Though less preferred, it is also envisaged that said heterologous polypeptide could be an Fc region of an antibody.

In line with the above definition the heterologous peptide can be a domain helpful for the isolation of a bispecific antibody and may be elected from peptide motives or secondarily introduced moieties, which can be captured in an isolation method, e.g. an isolation column. A non-limiting embodiments of such additional domains comprise peptide motives known as Myc-tag, HAT-tag, HA-tag, TAP-tag, GST-tag, chitin binding domain (CBD-tag), maltose binding protein (MBP-tag), Flag-tag, Strep-tag and variants thereof (e.g. StrepII-tag) and His-tag. All herein disclosed bispecific antibodies are preferred to comprise a His-tag domain, which is generally known as a repeat of consecutive His residues in the amino acid sequence of a molecule, preferably of six His residues.

Also in one aspect of the invention the first binding domain of the bispecific antibody binding to a cell surface molecule is binding to a tumor antigen.

The term "tumor antigen" as used herein may be understood as those antigens that are presented on tumor cells. These antigens can be presented on the cell surface with an extracellular part, which is often combined with a transmembrane and cytoplasmic part of the molecule. These antigens can sometimes be presented only by tumor cells and never by the normal ones. Tumor antigens can be exclusively expressed on tumor cells or might represent a tumor specific mutation compared to normal cells. In this case, they are called tumor-specific antigens. More common are antigens that are presented by tumor cells and normal cells, and they are called tumor-associated antigens. These tumor-associated antigens can be overexpressed compared to normal cells or are accessible for antibody binding in tumor cells due to the less compact structure of the tumor tissue compared to normal tissue.

Also in one aspect of the bispecific antibody of the invention the second binding domain binding to the T cell CD3 receptor complex is binding to an epitope of human and Callithrix jacchus, Saguinus oedipus or Saimiri sciureus CD3ε chain, wherein the epitope is part of an amino acid sequence comprised in the group consisting of SEQ ID NOs: 2, 4, 6, or 8 and comprises at least the amino acid sequence Gln-Asp-Gly-Asn-Glu.

In another aspect of the invention, the second binding domain is binding to CD3 epsilon. In still another aspect of the invention, the second binding domain is binding to human CD3 and to macaque CD3, preferably to human CD3 epsilon and to macaque CD3 epsilon. Additionally or alternatively, the second binding domain is binding to Callithrix jacchus, Saguinus oedipus and/or Saimiri sciureus CD3 epsilon. According to these embodiments, one or both binding domains of the bispecific antibody of the invention are preferably cross-species specific for members of the mammalian order of primates. Cross-species specific CD3 binding domains are, for example, described in WO 2008/119567.

It is particularly preferred for the bispecific antibody of the present invention that the second binding domain binding to the T cell CD3 receptor complex comprises a VL region comprising CDR-L1, CDR-L2 and CDR-L3 selected from:

(a) CDR-L1 as depicted in SEQ ID NO: 27 of WO 2008/119567, CDR-L2 as depicted in SEQ ID NO: 28 of WO 2008/119567 and CDR-L3 as depicted in SEQ ID NO: 29 of WO 2008/119567;

(b) CDR-L1 as depicted in SEQ ID NO: 117 of WO 2008/119567, CDR-L2 as depicted in SEQ ID NO: 118 of WO 2008/119567 and CDR-L3 as depicted in SEQ ID NO: 119 of WO 2008/119567; and

(c) CDR-L1 as depicted in SEQ ID NO: 153 of WO 2008/119567, CDR-L2 as depicted in SEQ ID NO: 154 of WO 2008/119567 and CDR-L3 as depicted in SEQ ID NO: 155 of WO 2008/119567.

In an alternatively preferred embodiment of the bispecific antibody of the present invention, the second binding domain binding to the T cell CD3 receptor complex comprises a VH region comprising CDR-H 1, CDR-H2 and CDR-H3 selected from:

(a) CDR-H1 as depicted in SEQ ID NO: 12 of WO 2008/119567, CDR-H2 as depicted in SEQ ID NO: 13 of WO 2008/119567 and CDR-H3 as depicted in SEQ ID NO: 14 of WO 2008/119567;

(b) CDR-H1 as depicted in SEQ ID NO: 30 of WO 2008/119567, CDR-H2 as depicted in SEQ ID NO: 31 of WO 2008/119567 and CDR-H3 as depicted in SEQ ID NO: 32 of WO 2008/119567;

(c) CDR-H1 as depicted in SEQ ID NO: 48 of WO 2008/119567, CDR-H2 as depicted in SEQ ID NO: 49 of WO 2008/119567 and CDR-H3 as depicted in SEQ ID NO: 50 of WO 2008/119567;

(d) CDR-H1 as depicted in SEQ ID NO: 66 of WO 2008/119567, CDR-H2 as depicted in SEQ ID NO: 67 of WO 2008/119567 and CDR-H3 as depicted in SEQ ID NO: 68 of WO 2008/119567;

(e) CDR-H1 as depicted in SEQ ID NO: 84 of WO 2008/119567, CDR-H2 as depicted in SEQ ID NO: 85 of WO 2008/119567 and CDR-H3 as depicted in SEQ ID NO: 86 of WO 2008/119567;

(f) CDR-H1 as depicted in SEQ ID NO: 102 of WO 2008/119567, CDR-H2 as depicted in SEQ ID NO: 103 of WO 2008/119567 and CDR-H3 as depicted in SEQ ID NO: 104 of WO 2008/119567;

(g) CDR-H1 as depicted in SEQ ID NO: 120 of WO 2008/119567, CDR-H2 as depicted in SEQ ID NO: 121 of WO 2008/119567 and CDR-H3 as depicted in SEQ ID NO: 122 of WO 2008/119567;

(h) CDR-H1 as depicted in SEQ ID NO: 138 of WO 2008/119567, CDR-H2 as depicted in SEQ ID NO: 139 of WO 2008/119567 and CDR-H3 as depicted in SEQ ID NO: 140 of WO 2008/119567;

(i) CDR-H1 as depicted in SEQ ID NO: 156 of WO 2008/119567, CDR-H2 as depicted in SEQ ID NO: 157 of WO 2008/119567 and CDR-H3 as depicted in SEQ ID NO: 158 of WO 2008/119567; and

(j) CDR-H1 as depicted in SEQ ID NO: 174 of WO 2008/119567, CDR-H2 as depicted in SEQ ID NO: 175 of WO 2008/119567 and CDR-H3 as depicted in SEQ ID NO: 176 of WO 2008/119567.

It is further preferred for the bispecific antibody of the present invention that the second binding domain binding to the T cell CD3 receptor complex comprises a VL region selected from the group consisting of a VL region as depicted in SEQ ID NO: 35, 39, 125, 129, 161 or 165 of WO 2008/119567.

It is alternatively preferred that the second binding domain binding to the T cell CD3 receptor complex comprises a VH region selected from the group consisting of a VH region as depicted in SEQ ID NO: 15, 19, 33, 37, 51, 55, 69, 73, 87, 91, 105, 109, 123, 127, 141, 145, 159, 163, 177 or 181 of WO 2008/119567.

More preferably, the bispecific antibody of the present invention is characterized by the second binding domain binding to the T cell CD3 receptor complex comprising a VL region and a VH region selected from the group consisting of:

(a) a VL region as depicted in SEQ ID NO: 17 or 21 of WO 2008/119567 and a VH region as depicted in SEQ ID NO: 15 or 19 of WO 2008/119567;

(b) a VL region as depicted in SEQ ID NO: 35 or 39 of WO 2008/119567 and a VH region as depicted in SEQ ID NO: 33 or 37 of WO 2008/119567;

(c) a VL region as depicted in SEQ ID NO: 53 or 57 of WO 2008/119567 and a VH region as depicted in SEQ ID NO: 51 or 55 of WO 2008/119567;

(d) a VL region as depicted in SEQ ID NO: 71 or 75 of WO 2008/119567 and a VH region as depicted in SEQ ID NO: 69 or 73 of WO 2008/119567;

(e) a VL region as depicted in SEQ ID NO: 89 or 93 of WO 2008/119567 and a VH region as depicted in SEQ ID NO: 87 or 91 of WO 2008/119567;

(f) a VL region as depicted in SEQ ID NO: 107 or 111 of WO 2008/119567 and a VH region as depicted in SEQ ID NO: 105 or 109 of WO 2008/119567;

(g) a VL region as depicted in SEQ ID NO: 125 or 129 of WO 2008/119567 and a VH region as depicted in SEQ ID NO: 123 or 127 of WO 2008/119567;

(h) a VL region as depicted in SEQ ID NO: 143 or 147 of WO 2008/119567 and a VH region as depicted in SEQ ID NO: 141 or 145 of WO 2008/119567;

(i) a VL region as depicted in SEQ ID NO: 161 or 165 of WO 2008/119567 and a VH region as depicted in SEQ ID NO: 159 or 163 of WO 2008/119567; and

(j) a VL region as depicted in SEQ ID NO: 179 or 183 of WO 2008/119567 and a VH region as depicted in SEQ ID NO: 177 or 181 of WO 2008/119567.

According to a preferred embodiment of the bispecific antibody of the present invention, in particular the second binding domain binding to the T cell CD3 receptor complex, the pairs of VH-regions and VL-regions are in the format of a single chain antibody (scFv). The VH and VL regions are arranged in the order VH-VL or VL-VH. It is preferred that the VH-region is positioned N-terminally to a linker sequence. The VL-region is positioned C-terminally of the linker sequence.

A preferred embodiment of the above described bispecific antibody of the present invention is characterized by the second binding domain binding to the T cell CD3 receptor complex comprising an amino acid sequence selected from the group consisting of SEQ ID NOs: 23, 25, 41, 43, 59, 61, 77, 79, 95, 97, 113, 115, 131, 133, 149, 151, 167, 169, 185 or 187 of WO 2008/119567.

In one embodiment the bispecific antibody of the invention is characterized by an amino acid sequence as depicted in SEQ ID NOs: 51, 52, 54, 55, 57, 58, 60, 61, 75, 76, 81, 82, 85, 86, 90, 91, 85, 96, 100 or 101.

The bispecific antibody of the present invention is preferably an "isolated" binding molecule. "Isolated" when used to describe the bispecific antibody disclosed herein, means a bispecific antibody that has been identified, separated and/or recovered from a component of its production environment. Preferably, the isolated bispecific antibody is free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. In preferred embodiments, the bispecific antibody will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Ordinarily, however, an isolated antibody will be prepared by at least one purification step.

Amino acid sequence modifications of the bispecific antibodies described herein are contemplated. For example, it may be desirable to improve the binding affinity and/or other biological properties of the antibody. Amino acid sequence variants of the bispecific antibodies are prepared by introducing appropriate nucleotide changes into the bispecific antibodies nucleic acid, or by peptide synthesis.

Such modifications include, for example, deletions from, and/or insertions into, and/or substitutions of, residues within the amino acid sequences of the bispecific antibodies. Any combination of deletion, insertion, and substitution is made to arrive at the final construct, provided that the final construct possesses the desired characteristics. The amino acid changes also may alter post-translational processes of the bispecific antibodies , such as changing the number or position of glycosylation sites. Preferably, 1, 2, 3, 4, 5, 6, 7, 8, 9, or 10 amino acids may be substituted in a CDR, while 1, 2, 3, 4, 5, 6, 7, 8, 9, 10, 11, 12, 13, 14, 15, 16, 17, 18, 19, 20, or 25 amino acids may be substituted in the framework regions (FRs). The substitutions are preferably conservative substitutions as described herein. Additionally or alternatively, 1, 2, 3, 4, 5, or 6 amino acids may be inserted or deleted in each of the CDRs (of course, dependent on their length), while 1, 2, 3, 4, 5, 6, 7, 8, 9, 10, 11, 12, 13, 14, 15, 16, 17, 18, 19, 20, or 25 amino acids may be inserted or deleted in each of the FRs.

A useful method for identification of certain residues or regions of the bispecific antibodies that are preferred locations for mutagenesis is called "alanine scanning mutagenesis" as described by Cunningham and Wells in Science, 244: 1081-1085 (1989). Here, a residue or group of target residues within the bispecific antibody is/are identified (e.g. charged residues such as arg, asp, his, lys, and glu) and replaced by a neutral or negatively charged amino acid (most preferably alanine or polyalanine) to affect the interaction of the amino acids with the epitope.

Those amino acid locations demonstrating functional sensitivity to the substitutions then are refined by introducing further or other variants at, or for, the sites of substitution. Thus, while the site for introducing an amino acid sequence variation is predetermined, the nature of the mutation per se needs not to be predetermined. For example, to analyze the performance of a mutation at a given site, ala scanning or random mutagenesis is conducted at a target codon or region and the expressed bispecific antibody variants are screened for the desired activity.

Preferably, amino acid sequence insertions include amino- and/or carboxyl-terminal fusions ranging in length from 1, 2, 3, 4, 5, 6, 7, 8, 9 or 10 residues to polypeptides containing a hundred or more residues, as well as intrasequence insertions of single or multiple amino acid residues. An insertional variant of the bispecific antibody includes the fusion to the N-or C-terminus of the antibody to an enzyme or a fusion to a polypeptide which increases the serum half-life of the antibody.

Another type of variant is an amino acid substitution variant. These variants have preferably at least 1, 2, 3, 4, 5, 6, 7, 8, 9 or 10 amino acid residues in the bispecific antibody replaced by a different residue. The sites of greatest interest for substitutional mutagenesis include the CDRs of the heavy and/or light chain, in particular the hypervariable regions, but FR alterations in the heavy and/or light chain are also contemplated.

For example, if a CDR sequence encompasses 6 amino acids, it is envisaged that one, two or three of these amino acids are substituted. Similarly, if a CDR sequence encompasses 15 amino acids it is envisaged that one, two, three, four, five or six of these amino acids are substituted.

Generally, if amino acids are substituted in one or more or all of the CDRs of the heavy and/or light chain, it is preferred that the then-obtained "substituted" sequence is at least 60%, more preferably 65%, even more preferably 70%, particularly preferably 75%, more particularly preferably 80% identical to the "original" CDR sequence. This means that it is dependent of the length of the CDR to which degree it is identical to the "substituted" sequence. For example, a CDR having 5 amino acids is preferably 80% identical to its substituted sequence in order to have at least one amino acid substituted. Accordingly, the CDRs of the bispecific antibody may have different degrees of identity to their substituted sequences, e.g., CDRL1 may have 80%, while CDRL3 may have 90%.

Preferred substitutions (or replacements) are conservative substitutions. However, any substitution (including non-conservative substitution or one or more from the "exemplary substitutions" listed in Table 2, below) is envisaged as long as the bispecific antibody retains its capability to bind to the surface molecule on a target cell via the first binding domain and to the CD3 receptor complex on a T cell via the second binding domain and/or its CDRs have an identity to the then substituted sequence (at least 60%, more preferably 65%, even more preferably 70%, particularly preferably 75%, more particularly preferably 80% identical to the "original" CDR sequence).

Conservative substitutions are shown in Table 2 under the heading of "preferred substitutions". If such substitutions result in a change in biological activity, then more substantial changes, denominated "exemplary substitutions" in Table 2, or as further described below in reference to amino acid classes, may be introduced and the products screened for a desired characteristic.

Table 2: Amino Acid Substitutions

| Original | Exemplary Substitutions | Preferred Substitutions |
|---|---|---|
| Ala (A) | val, leu, ile | val |
| Arg (R) | lys, gln, asn | lys |
| Asn (N) | gln, his, asp, lys, arg | gln |
| Asp (D) | glu, asn | glu |
| Cys (C) | ser, ala | ser |
| Gln (Q) | asn, glu | asn |
| Glu (E) | asp, gln | asp |
| Gly (G) | ala | ala |
| His (H) | asn, gln, lys, arg | arg |
| Ile (I) | leu, val, met, ala, phe | leu |
| Leu (L) | norleucine, ile, val, met, ala | ile |
| Lys (K) | arg, gln, asn | arg |
| Met (M) | leu, phe, ile | leu |
| Phe (F) | leu, val, ile, ala, tyr | tyr |

(continued)

| Original | Exemplary Substitutions | Preferred Substitutions |
|---|---|---|
| Pro (P) | ala | ala |
| Ser (S) | thr | thr |
| Thr (T) | ser | ser |
| Trp (W) | tyr, phe | tyr |
| Tyr (Y) | trp, phe, thr, ser | phe |
| Val (V) | ile, leu, met, phe, ala | leu |

Substantial modifications in the biological properties of the bispecific antibody of the present invention are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties: (1) hydrophobic: norleucine, met, ala, val, leu, ile; (2) neutral hydrophilic: cys, ser, thr; (3) acidic: asp, glu; (4) basic: asn, gin, his, lys, arg; (5) residues that influence chain orientation: gly, pro; and (6) aromatic : trp, tyr, phe.

Non-conservative substitutions will entail exchanging a member of one of these classes for another class. Any cysteine residue not involved in maintaining the proper conformation of the bispecific antibody may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

A particularly preferred type of substitutional variant involves substituting one or more hypervariable region residues of a parent antibody (e. g. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (e. g. 6-7 sites) are mutated to generate all possible amino acid substitutions at each site. The antibody variants thus generated are displayed in a monovalent fashion from filamentous phage particles as fusions to the gene III product of M13 packaged within each particle. The phage-displayed variants are then screened for their biological activity (e. g. binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the binding domain and, e.g., the surface molecule on a target cell and CD3 receptor complex on a T cell. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Other modifications of the bispecific antibody are contemplated herein. For example, the bispecific antibody may be linked to one of a variety of non-proteinaceous polymers, e.g., polyethylene glycol, polypropylene glycol, polyoxyalkylenes, or copolymers of polyethylene glycol and polypropylene glycol. The bispecific antibody may also be entrapped in microcapsules prepared, for example, by coacervation techniques or by interfacial polymerization (for example, hydroxymethylcellulose or gelatine-microcapsules and poly (methylmethacylate) microcapsules, respectively), in colloidal drug delivery systems (for example, liposomes, albumin microspheres, microemulsions, nanoparticles and nanocapsules), or in macroemulsions. Such techniques are disclosed in Remington's Pharmaceutical Sciences, 16th edition, Oslo, A., Ed., (1980).

The bispecific antibodies disclosed herein may also be formulated as immuno-liposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA, 82: 3688 (1985); Hwang et al. , Proc. Natl Acad. Sci. USA, 77: 4030 (1980); US Pat. Nos. 4,485,045 and 4,544,545; and WO 97/38731 published October 23, 1997. Liposomes with enhanced circulation time are disclosed in US Patent No. 5,013, 556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the

liposomes as described in Martin et al. J. Biol. Chem. 257: 286-288 (1982) via a disulfide interchange reaction. A chemotherapeutic agent is optionally contained within the liposome. See Gabizon et al. J. National Cancer Inst. 81 (19) 1484 (1989).

When using recombinant techniques, the bispecific antibody can be produced intracellularly, in the periplasmic space, or directly secreted into the medium. If the bispecific antibody is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating antibodies which are secreted to the periplasmic space of E. coli.

The bispecific antibody composition prepared from the cells can be purified using, for example, hydroxylapatite chromatography, gel electrophoresis, dialysis, and affinity chromatography, with affinity chromatography being the preferred purification technique.

In a further aspect, the present invention relates to a nucleic acid sequence encoding a bispecific antibody of the invention. In the case that the bispecific antibody of the invention is a heterodimer or heteromultimer it is also envisaged that the bispecific antibody may be encoded by more than one nucleic acid sequences/molecules, e.g. one nucleic acid sequence/molecule for each single polypeptide chain.

The term "nucleic acid" is well known to the skilled person and encompasses DNA (such as cDNA) and RNA (such as mRNA). The nucleic acid can be double stranded and single stranded. Said nucleic acid molecule is preferably comprised in a vector which is preferably comprised in a host cell. Said host cell is, e.g. after transformation or transfection with the nucleic acid sequence of the invention, expressing the binding molecule. For that purpose the nucleic acid molecule is operatively linked with control sequences.

An alternative aspect of the invention is a vector comprising a nucleic acid sequence as defined herein above. A vector is a nucleic acid molecule used as a vehicle to transfer (foreign) genetic material into a cell. The term "vector" encompasses - but is not restricted to - plasmids, viruses, cosmids and artificial chromosomes. In general, engineered vectors comprise an origin of replication, a multicloning site and a selectable marker. The vector itself is generally a nucleotide sequence, commonly a DNA sequence, that comprises an insert (transgene) and a larger sequence that serves as the "backbone" of the vector. Modern vectors may encompass additional features besides the transgene insert and a backbone: promoter, genetic marker, antibiotic resistance, reporter gene, targeting sequence, protein purification tag. Vectors called expression vectors (expression constructs) specifically are for the expression of the transgene in the target cell, and generally have control sequences such as a promoter sequence that drives expression of the transgene. Insertion of a vector into the target cell is usually called "transformation" for bacterial cells, "transfection" for eukaryotic cells, although insertion of a viral vector is also called "transduction".

Moreover, an alternative aspect of the invention is a host cell transformed or transfected with the nucleic acid sequence as defined herein above. As used herein, the term "host cell" is intended to refer to a cell into which a nucleic acid encoding the bispecific antibody of the invention is introduced by way of transformation, transfection and the like. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

As used herein, the term "expression" includes any step involved in the production of a bispecific antibody of the invention including, but not limited to, transcription, post-transcriptional modification, translation, post-translational modification, and secretion.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

A nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The terms "host cell," "target cell" or "recipient cell" are intended to include any individual cell or cell culture that can be or has/have been recipients for vectors or the incorporation of exogenous nucleic acid molecules, polynucleotides and/or proteins. It also is intended to include progeny of a single cell, and the progeny may not necessarily be completely identical (in morphology or in genomic or total DNA complement) to the original parent cell due to natural, accidental, or deliberate mutation. The cells may be prokaryotic or eukaryotic, and include but are not limited to bacterial

cells, yeast cells, fungal cells, animal cells, and mammalian cells, e.g., murine, rat, macaque or human.

Suitable host cells include prokaryotes and eukaryotic host cells including yeasts, fungi, insect cells and mammalian cells.

The bispecific antibody of the invention can be produced in bacteria. After expression, the bispecific antibody of the invention, preferably the bispecific antibody is isolated from the E. coli cell paste in a soluble fraction and can be purified through, e.g., affinity chromatography and/or size exclusion. Final purification can be carried out similar to the process for purifying antibody expressed e. g, in CHO cells.

In addition to prokaryotes, eukaryotic microbes such as filamentous fungi or yeast are suitable cloning or expression hosts for the bispecific antibody of the invention. Saccharomyces cerevisiae, or common baker's yeast, is the most commonly used among lower eukaryotic host microorganisms. However, a number of other genera, species, and strains are commonly available and useful herein, such as Schizosaccharomyces pombe, Kluyveromyces hosts such as, e.g., K. lactis, K. fragilis (ATCC 12424), K. bulgaricus (ATCC 16045), K. wickeramii (ATCC 24178), K. waltii (ATCC 56500), K. drosophilarum (ATCC 36906), K. thermotolerans, and K. marxianus; yarrowia (EP 402 226); Pichia pastoris (EP 183 070); Candida; Trichoderma reesia (EP 244 234); Neurospora crassa; Schwanniomyces such as Schwanniomyces occidentalis; and filamentous fungi such as, e.g., Neurospora, Penicillium, Tolypocladium, and Aspergillus hosts such as A. nidulans and A. niger.

Suitable host cells for the expression of glycosylated bispecific antibody of the invention, preferably antibody derived bispecific antibodies are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruit fly), and Bombyx mori have been identified. A variety of viral strains for transfection are publicly available, e. g. , the L-1 variant of Autographa californica NPV and the Bm-5 strain of Bombyx mori NPV, and such viruses may be used as the virus herein according to the present invention, particularly for transfection of Spodoptera frugiperda cells.

Plant cell cultures of cotton, corn, potato, soybean, petunia, tomato, Arabidopsis and tobacco can also be utilized as hosts. Cloning and expression vectors useful in the production of proteins in plant cell culture are known to those of skill in the art. See e.g. Hiatt et al., Nature (1989) 342: 76-78, Owen et al. (1992) Bio/Technology 10: 790-794, Artsaenko et al. (1995) The Plant J 8: 745-750, and Fecker et al. (1996) Plant Mol Biol 32: 979-986.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al. , J. Gen Virol. 36 : 59 (1977)); baby hamster kidney cells (BHK, ATCC CCL 10); Chinese hamster ovary cells/-DHFR (CHO, Urlaub et al. , Proc. Natl. Acad. Sci. USA 77: 4216 (1980)); mouse sertoli cells (TM4, Mather, Biol. Reprod. 23: 243-251 (1980)); monkey kidney cells (CVI ATCC CCL 70); African green monkey kidney cells (VERO-76, ATCC CRL1587) ; human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2,1413 8065); mouse mammary tumor (MMT 060562, ATCC CCL5 1); TRI cells (Mather et al., Annals N. Y Acad. Sci. 383 : 44-68 (1982)); MRC 5 cells; FS4 cells; and a human hepatoma line (Hep G2).

When using recombinant techniques, the bispecific antibody of the invention can be produced intracellularly, in the periplasmic space, or directly secreted into the medium. If the bispecific antibody is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating antibodies which are secreted to the periplasmic space of E. coli. Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The bispecific antibody of the invention prepared from the host cells can be purified using, for example, hydroxylapatite chromatography, gel electrophoresis, dialysis, and affinity chromatography, with affinity chromatography being the preferred purification technique.

The matrix to which the affinity ligand is attached is most often agarose, but other matrices are available. Mechanically stable matrices such as controlled pore glass or poly (styrenedivinyl) benzene allow for faster flow rates and shorter processing times than can be achieved with agarose. Where the bispecific antibody of the invention comprises a CH3 domain, the Bakerbond ABXMresin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSETM chromatography on an anion or cation exchange resin (such as a polyaspartic acid column), chromato-focusing, SDS-PAGE, and ammonium sulfate precipitation are also available depending on the antibody to be recovered.

In another aspect, processes are provided for producing bispecific antibodies of the invention, said processes comprising culturing a host cell defined herein under conditions allowing the expression of the bispecific antibody of the invention and recovering the produced bispecific antibody from the culture.

The term "culturing" refers to the in vitro maintenance, differentiation, growth, proliferation and/or propagation of cells under suitable conditions in a medium.

In an alternative embodiment, compositions are provided comprising a bispecific antibody of the invention, or produced according to the process of the invention. Preferably, said composition is a pharmaceutical composition.

As used herein, the term "pharmaceutical composition" relates to a composition for administration to a patient, preferably a human patient. The particular preferred pharmaceutical composition of this invention comprises the bispecific antibody of the invention. Preferably, the pharmaceutical composition comprises suitable formulations of carriers, stabilizers and/or excipients. In a preferred embodiment, the pharmaceutical composition comprises a composition for parenteral, transdermal, intraluminal, intraarterial, intrathecal and/or intranasal administration or by direct injection into tissue. It is in particular envisaged that said composition is administered to a patient via infusion or injection. Administration of the suitable compositions may be effected by different ways, e.g., by intravenous, intraperitoneal, subcutaneous, intramuscular, topical or intradermal administration. In particular, the present invention provides for an uninterrupted administration of the suitable composition. As a non-limiting example, uninterrupted, i.e. continuous administration may be realized by a small pump system worn by the patient for metering the influx of therapeutic agent into the body of the patient. The pharmaceutical composition comprising the bispecific antibody of the invention can be administered by using said pump systems. Such pump systems are generally known in the art, and commonly rely on periodic exchange of cartridges containing the therapeutic agent to be infused. When exchanging the cartridge in such a pump system, a temporary interruption of the otherwise uninterrupted flow of therapeutic agent into the body of the patient may ensue. In such a case, the phase of administration prior to cartridge replacement and the phase of administration following cartridge replacement would still be considered within the meaning of the pharmaceutical means and methods of the invention together make up one "uninterrupted administration" of such therapeutic agent.

The continuous or uninterrupted administration of these bispecific antibodies of the invention may be intravenous or subcutaneous by way of a fluid delivery device or small pump system including a fluid driving mechanism for driving fluid out of a reservoir and an actuating mechanism for actuating the driving mechanism. Pump systems for subcutaneous administration may include a needle or a cannula for penetrating the skin of a patient and delivering the suitable composition into the patient's body. Said pump systems may be directly fixed or attached to the skin of the patient independently of a vein, artery or blood vessel, thereby allowing a direct contact between the pump system and the skin of the patient. The pump system can be attached to the skin of the patient for 24 hours up to several days. The pump system may be of small size with a reservoir for small volumes. As a non-limiting example, the volume of the reservoir for the suitable pharmaceutical composition to be administered can be between 0.1 and 50 ml.

The continuous administration may be transdermal by way of a patch worn on the skin and replaced at intervals. One of skill in the art is aware of patch systems for drug delivery suitable for this purpose. It is of note that transdermal administration is especially amenable to uninterrupted administration, as exchange of a first exhausted patch can advantageously be accomplished simultaneously with the placement of a new, second patch, for example on the surface of the skin immediately adjacent to the first exhausted patch and immediately prior to removal of the first exhausted patch. Issues of flow interruption or power cell failure do not arise.

The inventive compositions may further comprise a pharmaceutically acceptable carrier. Examples of suitable pharmaceutical carriers are well known in the art and include solutions, e.g. phosphate buffered saline solutions, water, emulsions, such as oil/water emulsions, various types of wetting agents, sterile solutions, liposomes, etc. Compositions comprising such carriers can be formulated by well-known conventional methods. Formulations can comprise carbohydrates, buffer solutions, amino acids and/or surfactants. Carbohydrates may be non-reducing sugars, preferably trehalose, sucrose, octasulfate, sorbitol or xylitol. In general, as used herein, "pharmaceutically acceptable carrier" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counter-ions, such as sodium, polyhydric sugar alcohols; amino acids, such as alanine, glycine, asparagine, 2-phenylalanine, and threonine; sugars or sugar alcohols, such as trehalose, sucrose, octasulfate, sorbitol or xylitol stachyose, mannose, sorbose, xylose, ribose, myoinisitose, galactose, lactitol, ribitol, myoinisitol, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone. Such formulations may be used for continuous administrations which may be intravenuous or subcutaneous with and/or without pump systems. Amino acids may be charged amino

acids, preferably lysine, lysine acetate, arginine, glutamate and/or histidine. Surfactants may be detergents, preferably with a molecular weight of >1.2 KD and/or a polyether, preferably with a molecular weight of >3 KD. Non-limiting examples for preferred detergents are Tween 20, Tween 40, Tween 60, Tween 80 or Tween 85. Non-limiting examples for preferred polyethers are PEG 3000, PEG 3350, PEG 4000 or PEG 5000. Buffer systems used in the present invention can have a preferred pH of 5-9 and may comprise citrate, succinate, phosphate, histidine and acetate.

The compositions of the present invention can be administered to the subject at a suitable dose which can be determined e.g. by dose escalating studies by administration of increasing doses of the polypeptide of the invention exhibiting cross-species specificity described herein to non-chimpanzee primates, for instance macaques. As set forth above, the bispecific antibody of the invention exhibiting cross-species specificity described herein can be advantageously used in identical form in preclinical testing in non-chimpanzee primates and as drug in humans. These compositions can also be administered in combination with other proteinaceous and non-proteinaceous drugs. These drugs may be administered simultaneously with the composition comprising the polypeptide of the invention as defined herein or separately before or after administration of said polypeptide in timely defined intervals and doses. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, inert gases and the like. In addition, the composition of the present invention might comprise proteinaceous carriers, like, e.g., serum albumin or immunoglobulin, preferably of human origin. It is envisaged that the composition of the invention might comprise, in addition to the polypeptide of the invention defined herein, further biologically active agents, depending on the intended use of the composition. Such agents might be drugs acting on the gastrointestinal system, drugs acting as cytostatica, drugs preventing hyperurikemia, drugs inhibiting immunoreactions (e.g. corticosteroids), drugs modulating the inflammatory response, drugs acting on the circulatory system and/or agents such as cytokines known in the art. It is also envisaged that the bispecific antibody of the present invention is applied in a co-therapy, i.e., in combination with another anti-cancer medicament.

The biological activity of the pharmaceutical composition defined herein can be determined for instance by cytotoxicity assays, as described in the following examples, in WO 99/54440 or by Schlereth et al. (Cancer Immunol. Immunother. 20 (2005), 1-12). "Efficacy" or "in vivo efficacy" as used herein refers to the response to therapy by the pharmaceutical composition of the invention, using e.g. standardized NCI response criteria. The success or in vivo efficacy of the therapy using a pharmaceutical composition of the invention refers to the effectiveness of the composition for its intended purpose, i.e. the ability of the composition to cause its desired effect, i.e. depletion of pathologic cells, e.g. tumor cells. The in vivo efficacy may be monitored by established standard methods for the respective disease entities including, but not limited to white blood cell counts, differentials, Fluorescence Activated Cell Sorting, bone marrow aspiration. In addition, various disease specific clinical chemistry parameters and other established standard methods may be used. Furthermore, computer-aided tomography, X-ray, nuclear magnetic resonance tomography (e.g. for National Cancer Institute-criteria based response assessment [Cheson BD, Horning SJ, Coiffier B, Shipp MA, Fisher RI, Connors JM, Lister TA, Vose J, Grillo-Lopez A, Hagenbeek A, Cabanillas F, Klippensten D, Hiddemann W, Castellino R, Harris NL, Armitage JO, Carter W, Hoppe R, Canellos GP. Report of an international workshop to standardize response criteria for non-Hodgkin's lymphomas. NCI Sponsored International Working Group. J Clin Oncol. 1999 Apr;17(4):1244]), positron-emission tomography scanning, white blood cell counts, differentials, Fluorescence Activated Cell Sorting, bone marrow aspiration, lymph node biopsies/histologies, and various lymphoma specific clinical chemistry parameters (e.g. lactate dehydrogenase) and other established standard methods may be used.

Another major challenge in the development of drugs such as the pharmaceutical composition of the invention is the predictable modulation of pharmacokinetic properties. To this end, a pharmacokinetic profile of the drug candidate, i.e. a profile of the pharmacokinetic parameters that affect the ability of a particular drug to treat a given condition, can be established. Pharmacokinetic parameters of the drug influencing the ability of a drug for treating a certain disease entity include, but are not limited to: half-life, volume of distribution, hepatic first-pass metabolism and the degree of blood serum binding. The efficacy of a given drug agent can be influenced by each of the parameters mentioned above.

"Half-life" when used herein in the context of a drug means the time where 50% of an administered drug are eliminated through biological processes, e.g. metabolism, excretion, etc.

By "hepatic first-pass metabolism" is meant the propensity of a drug to be metabolized upon first contact with the liver, i.e. during its first pass through the liver.

"Volume of distribution" means the degree of retention of a drug throughout the various compartments of the body, like e.g. intracellular and extracellular spaces, tissues and organs, etc. and the distribution of the drug within these compartments.

"Degree of blood serum binding" means the propensity of a drug to interact with and bind to blood serum proteins, such as albumin, leading to a reduction or loss of biological activity of the drug.

Pharmacokinetic parameters also include bioavailability, lag time (Tlag), Tmax, absorption rates, more onset and/or Cmax for a given amount of drug administered. "Bioavailability" means the amount of a drug in the blood compartment. "Lag time" means the time delay between the administration of the drug and its detection and measurability in blood or plasma.

"Tmax" is the time after which maximal blood concentration of the drug is reached, and "Cmax" is the blood concentration maximally obtained with a given drug. The time to reach a blood or tissue concentration of the drug which is required for its biological effect is influenced by all parameters. Pharmacokinetic parameters of bispecific single chain antibodies exhibiting cross-species specificity, which may be determined in preclinical animal testing in non-chimpanzee primates as outlined above, are also set forth e.g. in the publication by Schlereth et al. (Cancer Immunol. Immunother. 20 (2005), 1-12).

The term "toxicity" as used herein refers to the toxic effects of a drug manifested in adverse events or severe adverse events. These side events might refer to a lack of tolerability of the drug in general and/or a lack of local tolerance after administration. Toxicity could also include teratogenic or carcinogenic effects caused by the drug.

The term "safety", "in vivo safety" or "tolerability" as used herein defines the administration of a drug without inducing severe adverse events directly after administration (local tolerance) and during a longer period of application of the drug. "Safety", "in vivo safety" or "tolerability" can be evaluated e.g. at regular intervals during the treatment and follow-up period. Measurements include clinical evaluation, e.g. organ manifestations, and screening of laboratory abnormalities. Clinical evaluation may be carried out and deviations to normal findings recorded/coded according to NCI-CTC and/or MedDRA standards. Organ manifestations may include criteria such as allergy/immunology, blood/bone marrow, cardiac arrhythmia, coagulation and the like, as set forth e.g. in the Common Terminology Criteria for adverse events v3.0 (CTCAE). Laboratory parameters which may be tested include for instance hematology, clinical chemistry, coagulation profile and urine analysis and examination of other body fluids such as serum, plasma, lymphoid or spinal fluid, liquor and the like. Safety can thus be assessed e.g. by physical examination, imaging techniques (i.e. ultrasound, x-ray, CT scans, Magnetic Resonance Imaging (MRI), other measures with technical devices (i.e. electrocardiogram), vital signs, by measuring laboratory parameters and recording adverse events. For example, adverse events in non-chimpanzee primates in the uses and methods according to the invention may be examined by histopathological and/or histochemical methods.

The term "effective dose" or "effective dosage" is defined as an amount sufficient to achieve or at least partially achieve the desired effect. The term "therapeutically effective dose" is defined as an amount sufficient to cure or at least partially arrest the disease and its complications in a patient already suffering from the disease. Amounts effective for this use will depend upon the severity of the infection and the general state of the subject's own immune system. The term "patient" includes human and other mammalian subjects that receive either prophylactic or therapeutic treatment.

The term "effective and non-toxic dose" as used herein refers to a tolerable dose of an inventive bispecific antibody which is high enough to cause depletion of pathologic cells, tumor elimination, tumor shrinkage or stabilization of disease without or essentially without major toxic effects. Such effective and non-toxic doses may be determined e.g. by dose escalation studies described in the art and should be below the dose inducing severe adverse side events (dose limiting toxicity, DLT).

The above terms are also referred to e.g. in the Preclinical safety evaluation of biotechnology-derived pharmaceuticals S6; ICH Harmonised Tripartite Guideline; ICH Steering Committee meeting on July 16, 1997.

The appropriate dosage, or therapeutically effective amount, of the bispecific antibody of the invention will depend on the condition to be treated, the severity of the condition, prior therapy, and the patient's clinical history and response to the therapeutic agent. The proper dose can be adjusted according to the judgment of the attending physician such that it can be administered to the patient one time or over a series of administrations. The pharmaceutical composition can be administered as a sole therapeutic or in combination with additional therapies such as anti-cancer therapies as needed.

The pharmaceutical compositions of this invention are particularly useful for parenteral administration, i.e., subcutaneously, intramuscularly, intravenously, intra-articular and/or intra-synovial. Parenteral administration can be by bolus injection or continuous infusion.

If the pharmaceutical composition has been lyophilized, the lyophilized material is first reconstituted in an appropriate liquid prior to administration. The lyophilized material may be reconstituted in, e.g., bacteriostatic water for injection (BWFI), physiological saline, phosphate buffered saline (PBS), or the same formulation the protein had been in prior to lyophilization.

Preferably, the bispecific antibody of the invention or produced by a process of the invention is used in the

prevention, treatment or amelioration of a disease selected from a proliferative disease, a tumorous disease, an inflammatory disease, an infectious disease and an autoimmune disease/immunological disorder.

An alternative aspect of the invention provides a method for the treatment or amelioration of a disease selected from a proliferative disease, a tumorous disease, an inflammatory disease, an infectious disease and an autoimmune disease/immunological disorder comprising the step of administering to a patient in the need thereof the bispecific antibody of the invention or produced by a process of the invention.

The formulations described herein are useful as pharmaceutical compositions in the treatment and/or prevention of the pathological medical condition as described herein in a patient in need thereof. The term "treatment" refers to both therapeutic treatment and prophylactic or preventative measures. Treatment includes the application or administration of the formulation to the body, an isolated tissue, or cell from a patient who has a disease/disorder, a symptom of a disease/disorder, or a predisposition toward a disease/disorder, with the purpose to cure, heal, alleviate, relieve, alter, remedy, ameliorate, improve, or affect the disease, the symptom of the disease, or the predisposition toward the disease.

Those "in need of treatment" include those already with the disorder, as well as those in which the disorder is to be prevented. The term "disease" is any condition that would benefit from treatment with the protein formulation described herein. This includes chronic and acute disorders or diseases including those pathological conditions that predispose the mammal to the disease in question. Non-limiting examples of diseases/disorders to be treated herein include proliferative diseases, tumorous diseases, inflammatory diseases, infectious diseases and autoimmune diseases/immunological disorders.

In another aspect, kits are provided comprising a bispecific antibody of the invention, a nucleic acid molecule of the invention, a vector of the invention, or a host cell of the invention. The kit may comprise one or more vials containing the bispecific antibody and instructions for use. The kit may also contain means for administering the bispecific antibody of the present invention such as a syringe, pump, infuser or the like.

## BRIEF DESCRIPTION OF THE DRAWINGS

Figures 1(1)-1(3): FACS binding analysis of designated bispecific antibody constructs to CHO cells expressing human PSMA as described in Example 1, and the human CD3+ T cell line HPB-ALL, respectively. The FACS staining was performed as described in Example 1. The bold (dark) lines represent cells incubated with cell culture supernatant of transfected cells expressing the bispecific antibody constructs. The light (grey) histograms show the negative controls. Supernatant of untransfected CHO cells was used as negative control.

Figures 2(1)-2(2): The figure shows results of chromium release assays measuring cytotoxic activity induced by designated bispecific antibody constructs redirected to CHO cell line transfected with human PSMA. As effector cells stimulated human CD 8+ T cells were used. The assays was performed in RPMI 1640 supplemented with 10% HSA and is described in Example 2.

Figure 3: Cytotoxicity analysis of EpCAM BiTE® molecules in the unmodified form or as fusion with SA21 or with HSA. Dose response curves were generated with addition of FCS, without addition of FCS, with addition 10% human serum and with addition of 20% human serum.

Figure 4: Cytotoxicity analysis of EpCAM BiTE® molecules in the unmodified form or as fusion with SA21 or with HSA. Dose response curves were generated with addition of 8 g/l HSA, with addition of 20 g/l HSA, with addition 10% human serum and with addition of 20% human serum.

Figure 5: The diagrams of figures 5(1) to 5(6) show results of FACS based cytotoxicity assays measuring cytotoxic activity induced by designated bispecific antibody constructs redirected to CHO cell line transfected with HIV gp140. As effector cells stimulated human CD $8^+$ T cells were used. The assays was performed in RPMI 1640 supplemented with 10% FCS (5(1), 5(3) and 5(5)) or 50% Human Serum (5(2), 5(4) and 5(6) respectively and is described in Example 9.

Figure 6: HSA / MSA Biacore experiment (surface plasmon resonance) Diagrams show affinity analyses of designated bispecific antibody constructs to immobilized HSA or MSA respectively. The Biacore experiments were performed as described in Example 10. The diagrams show the association and dissociation characteristics of designated bispecific constructs at different concentrations.

Figure 7: human CD3 Biacore experiment (surface plasmon resonance) Affinity analyses of designated bispecific antibody constructs to immobilized human CD3. The Biacore experiments were performed as described in Example 11. The diagrams show the association and dissociation characteristics of designated bispecific constructs at different concentrations.

Figure 8: Affinity analyses of designated bispecific antibody constructs to immobilized human CD3. The Biacore experiments were performed as described in Example 12. The diagrams show the association and dissociation characteristics of designated bispecific constructs at different concentrations.

Figure 9: SEC profile of CD4(1+2)xaCD3LxSA21 The diagram shows the size exclusion profile of CD4(1+2)xaCD3LxSA21. Peaks of aggregate, multimeric and monomer forms are indicated. The blue line indicates optical absorption at 280 nm. The purification method is described in Example 13.

## EXAMPLES

The following examples illustrate the invention. These examples should not be construed as to limit the scope of this invention. The examples are included for purposes of illustration, and the present invention is limited only by the claims.

### Example 1: Bispecific binding and interspecies cross-reactivity

For flow cytometry human PSMA-transfected CHO target cells (the general procedure for the transfection of CHO cells with cell surface molecules to create appropriate target cells is derivable from WO 2008/119567) were used. 200,000 cells of the respective cell lines were incubated for 30 min on ice with 50 μl of purified bispecific molecules at a concentration of 5 μg/ml. The cells were washed twice in PBS with 2% FCS and binding of the constructs was detected with a murine PentaHis antibody (Qiagen; diluted 1:20 in 50 μl PBS with 2% FCS). After washing, bound PentaHis antibodies were detected with an Fc gamma-specific antibody (Dianova) conjugated to phycoerythrin, diluted 1:100 in PBS with 2% FCS.

Figures 1 shows the histograms of those FACS analyses. The results with respect to the detection of binding to the target PSMA and to CD3 are summarized in the appended tables 3 and 4. The parental variants of the identified BiTE® molecules without an ABP are described in detail, including specific examples for the amino acid sequence, in WO 2010/037836.

Table 3: Binding characteristics of PSMA BiTE® antibody molecules with ABP at the N-terminus

| Construct | PSMA binding | CD3 binding |
|---|---|---|
| AB01 x PSMA x CD3 | - | + |
| AB14 x PSMA x CD3 | - | + |
| AB156 x PSMA x CD3 | - | + |
| DX236 x PSMA x CD3 | - | + |
| DX321 x PSMA x CD3 | - | + |
| SA04 x PSMA x CD3 | - | + |
| SA08 x PSMA x CD3 | - | + |
| SA21 x PSMA x CD3 | - | + |
| SA25 x PSMA x CD3 | - | + |

Table 4: Binding characteristics of PSMA BiTE® antibody molecules with ABP at the C-terminus

| Construct | PSMA binding | CD3 binding |
|---|---|---|
| PSMA x CD3 x AB01 | + | + |
| PSMA x CD3 x AB14 | + | + |
| PSMA x CD3 x AB156 | + | + |

(continued)

| Construct | PSMA binding | CD3 binding |
|---|---|---|
| PSMA x CD3 x DX236 | + | + |
| PSMA x CD3 x DX321 | + | + |
| PSMA x CD3 x SA04 | + | + |
| PSMA x CD3 x SA08 | + | + |
| PSMA x CD3 x SA21 | + | + |
| PSMA x CD3 x SA25 | + | + |

**Example 2: Cytotoxic activity**

***Chromium release assay with stimulated human T cells***

Stimulated T cells enriched for $CD8^+$ T cells were obtained as described below.

A petri dish (145 mm diameter, Greiner bio-one GmbH, Kremsmünster) was coated with a commercially available anti-CD3 specific antibody (OKT3, Orthoclone) in a final concentration of 1 μg/ml for 1 hour at 37°C. Unbound protein was removed by one washing step with PBS. 3 - 5 x $10^7$ human PBMC were added to the precoated petri dish in 120 ml of RPMI 1640 with stabilized glutamine / 10% FCS / IL-2 20 U/ml (Proleukin®, Chiron) and stimulated for 2 days. On the third day, the cells were collected and washed once with RPMI 1640. IL-2 was added to a final concentration of 20 U/ml and the cells were cultured again for one day in the same cell culture medium as above.

$CD8^+$ cytotoxic T lymphocytes (CTLs) were enriched by depletion of CD4+ T cells and CD56+ NK cells using Dynal-Beads according to the manufacturer's protocol.

Macaque or human PSMA-transfected CHO target cells (the general procedure for the transfection of CHO cells with cell surface molecules to create appropriate target cells is derivable from WO 2008/119567) were washed twice with PBS and labeled with 11.1 MBq 51Cr in a final volume of 100 μl RPMI with 50% FCS for 60 minutes at 37°C. Subsequently, the labeled target cells were washed 3 times with 5 ml RPMI and then used in the cytotoxicity assay. The assay was performed in a 96-well plate in a total volume of 200 μl supplemented RPMI (as above) with an E:T ratio of 10:1. A starting concentration of 0.01 - 1 μg/ml of purified bispecific antibody and threefold dilutions thereof were used. Incubation time for the assay was 18 hours. Cytotoxicity was determined as relative values of released chromium in the supernatant relative to the difference of maximum lysis (addition of Triton-X) and spontaneous lysis (without effector cells). All measurements were carried out in quadruplicates. Measurement of chromium activity in the supernatants was performed in a Wizard 3" gammacounter (Perkin Elmer Life Sciences GmbH, Köln, Germany). Analysis of the results was carried out with Prism 5 for Windows (version 5.0, GraphPad Software Inc., San Diego, California, USA). $EC_{50}$ values calculated by the analysis program from the sigmoidal dose response curves were used for comparison of cytotoxic activity.

The results for the kill of PSMA expressing target cells are shown in figure 2.

**Example 3: Biacore-based determination of BiTE®-antibody affinity to serum albumin of different species**

To confirm binding of the tagged PSMA BiTE® molecules to human serum albumin and serum albumins of other species, the following serum albumin preparations were provided: Human serum albumin (HSA), Sigma A9511; Rhesus monkey serum albumin (RSA), bio World 22070099; Mouse serum albumin (MSA), Sigma A3139; Bovine serum albumin (BSA), Sigma A7906.

Binding experiments were performed using plasmon resonance measurements in a Biacore machine. In detail, CM5 Sensor Chips (GE Healthcare) were immobilized with the respective serum albumin protein using acetate buffer pH 4.5 according to the manufacturer's manual. The BiTE® antibody samples were loaded in five relevant concentrations e.g.: 50 nM, 25 nM, 12.5 nM, 6.25 nM and 3.13 nM diluted in HBS-EP running buffer (GE Healthcare). For affinity determinations the flow rate was 35 μl/min for 3 min, then HBS-EP running buffer was applied for 10 min again at a flow rate of 35 μl/ml. Regeneration of the chip was performed using a buffer consisting of 100 mM glycine 0.5 M NaCl pH 3.0 solution. Data sets were analyzed using BiaEval Software. In some cases binding curves could perfectly or not at all be matched with the theoretical curves underlying the evaluation software. In the latter case the binding was determined as "positive"; in the case of closer match numbers are presented but have to be seen as approximation.

Binding affinities of the respective BiTE® antibodies to serum albumin of different species as determined by Biacore are summarized in table 5.

Table 5: Affinities (KD) of tagged PSMA BiTE® antibody against serum albumins of different species measured in Biacore experiments (surface plasmon resonance).

| *BiTE® antibody* | *KD [nM] HSA* | *KD [nM] RSA* | *KD [nM] MSA* | *KD [nM] BSA* |
|---|---|---|---|---|
| PSMA x SA04 | negative | positive | positive | negative |
| PSMA x SA08 | 116 | 42 | 77 | 434 |
| PSMA x SA21 | 137 | 49 | 70 | positive |
| PSMA x SA25 | 1650 | 95 | 147 | positive |
| PSMA x DX236 | 3060 | Negative | negative | negative |
| PSMA x DX321 | positive | Positive | negative | negative |
| PSMA x AB01 | 17400 | 810 | negative | negative |
| PSMA x AB14 | 282 | 94 | negative | negative |
| PSMA x AB156 | 426 | 96 | negative | negative |

HSA = human serum albumin, RSA = rhesus serum albumin, MSA = mouse serum albumin, BSA = bovine serum albumin. "Positive" means detectable binding which could not be determined accurately by the BiaEval software; "Negative" means no significant binding under the chosen experimental conditions.

As depicted in table 5, all of the tested PSMA BiTE antibodies containing different serum binding peptide tags showed significant binding to human serum albumin with the exception of PSMA x SA04 that were negative on human serum albumin but showed distinct binding to rhesus serum albumin. Moreover, all of the tagged BiTE antibodies tested showed distinct binding to rhesus serum albumin, except for PSMA-DX236. Furthermore, of the tested BiTE antibodies, PSMA x SA04, PSMA x SA08, PSMA x SA21 and PSMA x SA25 showed distinct binding to mouse serum albumin, whereas PSMA x DX236, PSMA x DX321, PSMA x AB1, PSMA x AB14 and PSMA x AB156 not show significant binding to murine serum albumin.

Among the molecules tested, only PSMA x SA08, PSMA x SA21 and PSMA x SA25 showed distinct binding to bovine serum albumin.

**Example 4: Purification scheme of BiTE® antibodies with/without a C-terminal ABP**

Chinese hamster ovary CHO cells stable transfected with a pEF DHFR vector coding the BiTE® antibody sequence were cultivated in roller bottles till 60% remaining vitality was reached. Cell culture supernatant was cleared by 0.2 μm filtration and applied to a column filled with a zinc loaded chelating gel Fractogel EMD Chelate (Merck, Darmstadt) for immobilized metal affinity chromatography (IMAC) capture.

After sample loading remaining cell culture supernatant was washed out and after a pre-elution step the HIS6 tagged BiTE® antibodies were eluted by applying a buffer containing 500 mM imidazol.

Eluted volume containing the BiTE® antibodies was given on a Superdex S200 (GE Healthcare, Munich) size exclusion chromatography (SEC) column to separate the BiTE® monomeric protein from dimeric BiTE® protein and other proteins.

Fractions containing the BiTE® monomeric protein were pooled and protein concentration was determined by measurement of optical absorption at 280 nm wavelength and 1 cm lightpath length and multiplication with the protein sequence specific absorption factor.

BiTE® Monomer percentage was calculated by the area under the curve in the SEC chromatograms of the BiTE® dimer and BiTE® monomer fraction. Monomer percentage = (Area of Monomer / (Area Monomer + Area Dimer))* 100.

As apparent from the tables below (tables 6 to 8) for all tested BiTE® antibody variants of anti PSMA BiTE® and anti human EpCAM BiTE® favorable yield of BiTE® monomeric protein and monomer percentage were achieved.

Table 6: BiTE® antibody monomer yield and monomer percentage of anti PSMA BiTE® variants equipped with a C-terminal added albumin binding peptide ABP showing favorable yield and monomer percentage above 80%

| **BiTE® Antibody** | **Monomer Yield [μg/L supernatant]** | **BiTE® Antibody Monomer %** |
|---|---|---|
| PSMA x CD3 x SA04 | 3070 | 90 |
| PSMA x CD3 x SA08 | 2243 | 85 |
| PSMA x CD3 x SA21 | 1537 | 84 |

(continued)

| BiTE® Antibody | Monomer Yield [μg/L supernatant] | BiTE® Antibody Monomer % |
|---|---|---|
| PSMA x CD3 x SA25 | 3384 | 85 |
| PSMA x CD3 x AB01 | 2572 | 92 |
| PSMA x CD3 x AB14 | 4337 | 88 |
| PSMA x CD3 x AB156 | 2605 | 93 |
| PSMA x CD3 x DX236 | 716 | 87 |
| PSMA x CD3 x DX321 | 13392 | 85 |

Table 7: Results of the Flow cytometry screening of samples of cell culture supernatants of anti PSMA BiTE® variants, equipped with a n-terminal added albumin binding peptide ABP for binding to cells expressing the PSMA antigen prior to purification. All cell culture supernatants not showing any binding in this assay were excluded from the purification procedure.

| BiTE® Antibody | FACS Binding | Monomer Percentage |
|---|---|---|
| SA04 x PSMA x CD3 | No functional protein (FACS) | - |
| SA08 x PSMA x CD3 | No functional protein (FACS) | - |
| SA21 x PSMA x CD3 | No functional protein (FACS) | - |
| SA25 x PSMA x CD3 | No functional protein (FACS) | - |
| AB01 x PSMA x CD3 | No functional protein (FACS) | - |
| AB14 x PSMA x CD3 | No functional protein (FACS) | - |
| AB156 x PSMA x CD3 | No functional protein (FACS) | - |
| DX232 x PSMA x CD3 | No functional protein (FACS) | - |
| DX236 x PSMA x CD3 | No functional protein (FACS) | - |
| DX321 x PSMA x CD3 | No functional protein (FACS) | - |

Table 8: BiTE antibody monomer yield and monomer percentage of anti human EpCAM BiTE® variant equipped with a C-terminal added albumin binding peptide ABP showing favorable yield and monomer percentage above 80%

| BiTE® molecule | Monomer Yield [μg/L supernatant] | BiTE® Antibody Monomer % |
|---|---|---|
| EpCAM x CD3 x SA21 | 4390 | 91 |

**Example 5: Human EpCAM BiTE® molecules**

Examples for parental human EpCAM BiTE® molecules are described in WO 2005/040220.

Bioactivity of EpCAM BiTE® was assessed in a cell-based assay making use of the Toxilight BioAssay Kit (Cambrex/Lonza). This Kit allows measuring the cytotoxic effect of human PBMCs (effector cells) on the EpCAM-expressing breast cancer cell line MDA-MB-453 in presence of EpCAM BITE®, by quantification of dead cells according to the following principle:

The assay was based on the bioluminescent measurement of the enzyme adenylate kinase (AK). AK was present in all cells. Loss of cell membrane integrity by dead cells results in the leakage of AK in the surrounding medium. Addition of the ToxiLight reaction reagent (containing ADP and the enzyme luciferase) triggers a two-step reaction resulting in light emission, which can be measured using a luminometer. The emitted light is linearly related to the AK concentration and correlates with the number of dead cells.

$$Mg^{++}ADP+ADP \xleftrightarrow{\text{Adenylate Kinase}} Mg^{++}ATP+AMP$$

$$ATP + Luciferin + O_2 \xleftrightarrow{Luciferase;\ Mg^{++}} Oxyluciferin + AMP + PP_i + CO_2 + Light$$

***Performance and data analysis:***

Effector (human PBMC, $2x10^6$/well) and target cells (MDA-MB-453, $1x10^5$/well) are co-cultivated in an effector to target ratio of 20:1 in presence of different EpCAM BITE® concentrations (250 ng/mL - 4.2 pg/mL). After 16 to 20 hours the ToxiLight Reagent is added and luminescence is measured (for detailed description see SOP-BIA-110-012).

The % specific lysis is calculated for each EpCAM BiTE® concentration and the data are fitted for determination of half-maximal cytotoxicity ($EC_{50}$). Although the $EC_{50}$ value describes the biological potency of EpCAM BiTE®, the absolute values can vary which is normal for a cell-based assay with different effector cell donors. Thus a relative potency is calculated for each dose response analysis in comparison to a EpCAM BiTE® working standard based on the following formula:

$$\text{Relative Potency} = EC_{50}\ \text{sample}/EC_{50}\ \text{standard}$$

Validity of the relative potency determination is controlled by various parameters e.g. curve fit, ratio of curve amplitudes, ratio of slopes, etc.

The initial cytotoxicity assay conditions do not contain HSA as the assay is developed to test clinical trial material for release and stability.

Addition of human serum was tested and relative bioactivity of the EpCAM BiTE®-SA21 construct was 51-66% for the 10% serum condition and 59-66% for the 20% serum condition (table 13). So the reduction in bioactivity by covalently adding the HSA-binding peptide and using the conditions described was in the range of 50-60%.

A more pronounced reduction in relative bioactivity was observed for the EpCAM BiTE construct that contained the covalently linked HSA. But also here the bioactivity was not completely blocked; remaining bioactivities were 19-31% and 24-29% for 10 and 20% human serum conditions (table 14).

Different assay conditions, like addition of FCS or HSA did not alter the observed effect on the relative bioactivity (table 9)

Dose response curves of the experiments described are shown in figure 3 and 4.

Table 9: $EC_{50}$ values for EpCAM BiTE® variants

| ***$EC_{50}$ [pM]*** | | | |
|---|---|---|---|
| | EpCAM BiTE® | -SA21 | -HSA |
| with FCS | 25 | 22 | 176 |
| w/o FCS | (864) | 602 | 2147 |
| 10% hu Serum | 69 | 105 | 358 |
| 20% hu Serum | 99 | 169 | 406 |
| ***$EC_{50}$ [pM]*** | | | |
| | EpCAM BiTE® | -SA21 | -HSA |
| 8 g/l HSA | 428 | 409 | 566 |
| 20 g/l HSA | 128 | 367 | 567 |
| 10% hu Serum | 217 | 425 | 700 |
| 20% hu Serum | 263 | 400 | 918 |

Table 10: Relative bioactivity compared to unmodified EpCAM BiTE®

| ***EpCAM BiTE®-SA21*** | |
|---|---|
| 10% hu Serum | 51% |
| 20% hu Serum | 66% |
| ***EpCAM BiTE®-SA21*** | |
| 10% hu Serum | 66% |

(continued)

| | |
|---|---|
| ***EpCAM BiTE®-SA21*** | |
| 20% hu Serum | 59% |

Table 11: Relative bioactivity compared to unmodified EpCAM BiTE®

| | |
|---|---|
| ***EpCAM BiTE®-HSA*** | |
| 10% hu Serum | 19% |
| 20% hu Serum | 24% |
| ***EpCAM BiTE®-HSA-*** | |
| 10% hu Serum | 31% |
| 20% hu Serum | 29% |

**Example 7: Generation of stably transfected HIV gp140 expressing CHO cells**

The extracellular domain of HIV gp160 (gp140) of the HIV-1 HXB2 strain (Uniprot KB, Acc. No. P04578) was fused to the transmembrane and intracellular domain of EpCAM. The open reading frame of this fusion protein was cloned into a pEF DHFR vector according to standard procedures (the general procedure for the transfection of CHO cells with cell surface molecules to create appropriate target cells is derivable from WO 2008/119567) and then used for the analyses of the designated bispecific constructs.

**Example 8: Generation of HIV bispecific constructs**

The HIV target binding domain of the bispecific constructs described below are of human CD4 origin. Constructs named CD4(1+2) contain the first two domains of human CD4. Constructs CD4 D1.1 or CD4 D1.2 are of human CD4 domain 1 origin containing single point mutations and are described by Chen, W., et al. (2011, J Virol 85(18): 9395-9405). The two constructs described here represent the proof of their applicability in the BiTE context, but is not limited to these two CD4 domain 1 constructs i.e. the described bispecific format can be applied to all described variants thereof, especially concerning the published variants CD4 D1.3 to CD4 D1.19. Generally, molecules with intrinsic binding moieties to HIV proteins accessible at the plasma membrane are applicable in the bispecific context, e.g. constructs containing only parts of human CD4 like the core peptide sequence, aptamers of the human CD4 protein or chimeras of e.g. rat CD4 with human CD4 insertions involved in gp120 binding as illustrated by James H. M. Simon (1993, J. Exp. Med, Volume 177, April 1993, 949-954) can be used in the BiTE context. Next to binding domains of human CD4 origin, antibody specificities of anti HIV human monoclonal antibodies such as B12, VRC01 or 4E10 in scFv format in VH-VL or VL-VH orientation are also applicable in the BiTE context and have been characterized concerning their bispecific binding and cytotoxic activity. The described HIV gp120 binding domains were fused to a cross-specific human CD3 binder. Long lived version of these parental HIV specific constructs have been designed and cloned according to standard procedures and are characterized below.

**Example 9: Cytotoxic activity bispecific constructs for HIV positive target cells**

FACS based cytotoxicity assay with stimulated human T-cells: Stimulated T-cells enriched for $CD8^+$ T-cells were obtained as described below.

A petri dish (145 mm diameter, Greiner bio-one GmbH, Kremsmünster) was coated with a commercially available anti-CD3 specific antibody (OKT3, Orthoclone) and anti-CD28 (Art.No. 340975, BD) in a final concentration of 1 $\mu$g/ml for 1 hour at 37°C. Unbound protein was removed by one washing step with PBS. 3 - 5 x $10^7$ human PBMC were added to the precoated petri dish in 100 ml of RPMI 1640 with stabilized glutamine / 10% FCS / IL-2 20 U/ml (Proleukin®, Chiron) and stimulated for 3 days. On the third day, the cells were collected and washed once with RPMI 1640. IL-2 was added to a final concentration of 20 U/ml and the cells were cultured again for one day in the cell culture medium mentioned above.

$CD8^+$ cytotoxic T lymphocytes (CTLs) were enriched by depletion of non $CD8^+$ cells using a human $CD8^+$ T Cell Isolation Kit according to the manufacturer's (Miltenyi Biotec) protocol.

HIV gp140-transfected CHO target cells (the general procedure for the transfection of CHO cells with cell surface molecules to create appropriate target cells is derivable from WO 2008/119567) were washed with PBS plus

2% FCS and labeled with Vybrant® DiD cell-labeling solution (Invitrogen, 125,000 cells/ml, 5 μl dye per $10^6$ cells) in PBS plus 2% FCS for 3 minutes at 37°C. Subsequently, the labeled target cells were washed 3 times with 50 ml PBS plus 2% FCS and then used in the cytotoxicity assay. The assay was performed in a 96-well plate in a total volume of 200 μl supplemented RPMI medium (as above) with an Effector:Target ratio of 10:1. A starting concentration of 2-10 μg/ml of purified bispecific antibody and three to four-fold dilutions thereof were used. Incubation time for the assay was 18-24 hours.

After the incubation, the cells were resuspended and transferred into a 96 well v-bottom plate. Adhering target cells were trypsinized for 5 minutes at 37°C, resuspended in PBS / 2% FCS and combined with the other cells from the respective wells. The plates were centrifuged for 4 minutes at 1200 rpm, the supernatant was discarded and the cells were resuspended in PBS plus 2% FCS plus 1 μg/ml propidium iodide (PI) (supplied by Sigma Aldrich). Cytotoxicity was determined as percentage of DiD / PI double positive cells relative to the overall number of DiD positive cells as determined by flow cytometry using a FACSCanto II (BD Biosciences, Heidelberg, Germany). All measurements were carried out in duplicates. Analyses of the results were carried out with Prism 5 for Windows (version 5.0, GraphPad Software Inc., San Diego, California, USA). $EC_{50}$ values were calculated by the analysis program from the sigmoidal dose or variable slope response curves and then used for comparison of cytotoxic activity.

The results for the cytotoxic activity using RPMI 1640 supplemented with 10% FCS or 50% Human Serum (PAA Laboratories GmbH, Art.No. C11-021) respectively on HIV gp140 expressing target cells are shown in figure 5(1)-5(4).

$EC_{50}$ values (i.e. BiTE® concentration at half maximal lysis of target cells) are shown for the designated bispecific molecules in tables 12 to 17 to illustrate their cytotoxic activity.

Table 12: $EC_{50}$ values of N-terminal ABP tagged BiTE® antibodies under the influence of 10% FCS

| **BiTE®** | **CD4(1+2)xaCD3** | **SA21xCD4(1+2)xaCD3** |
|---:|---:|---:|
| **$EC_{50}$ [ng/ml]** | 0.12 | 0.93 |
| **Factor*** | 1 | 7.75 |
| **BiTE®** | **SA21LxCD4(1+2)xaCD3** | **SA21LxCD4(1+2)xaCD3** |
| **$EC_{50}$ [ng/ml]** | 2.0 | 0.78 |
| **Factor*** | 16.6 | 6.5 |
| **BiTE®** | **SA25xCD4(1+2)xaCD3** | |
| **$EC_{50}$ [ng/ml]** | 0.29 | |
| **Factor*** | 2.41 | |
| Factor*: Factor describes the $EC_{50}$ value difference of the designated bispecific molecule compared to the parental bispecific molecule without ABP tag. | | |

Table 13: $EC_{50}$ values of N-terminal ABP tagged BiTE® antibodies under the influence of 50% Human Serum

| **BiTE®** | **CD4(1+2)xaCD3** | **SA21xCD4(1+2)xaCD3** |
|---:|---:|---:|
| **$EC_{50}$ [ng/ml]** | 0.24 | 4.0 |
| **Factor*** | 1 | 16.6 |
| **BiTE®** | **SA21LxCD4(1+2)xaCD3** | **SA21LxCD4(1+2)xaCD3** |
| **$EC_{50}$ [ng/ml]** | 5.5 | 2.7 |
| **Factor*** | 22.9 | 11.25 |
| **BiTE®** | **SAx25CD4(1+2)xaCD3** | |
| **$EC_{50}$ [ng/ml]** | 0.78 | |
| **Factor*** | 3.25 | |
| Factor*: Factor describes the $EC_{50}$ value difference of the designated bispecific molecule compared to the parental bispecific molecule without ABP tag. | | |

Table 14: $EC_{50}$ values of C-terminal ABP tagged BiTE® antibodies under the influence of 10% FCS

| BiTE® | CD4(1+2)xaCD3 | CD4(1+2)xaCD3xSA21 |
|---|---|---|
| $EC_{50}$ [ng/ml] | 0.12 | 0.23 |
| Factor* | 1 | 1.92 |
| BiTE® | CD4(1+2)xaCD3LxSA21 | CD4(1+2)xaCD3LxSA21 |
| $EC_{50}$ [ng/ml] | 0.23 | 0.35 |
| Factor* | 1.92 | 2,92 |
| BiTE® | CD4(1+2)xaCD3xSA25 | |
| $EC_{50}$ [ng/ml] | 0.21 | |
| Factor* | 1.75 | |
| Factor*: Factor describes the $EC_{50}$ value difference of the designated bispecific molecule compared to the parental bispecific molecule without ABP tag. | | |

Table 15: $EC_{50}$ values of C-terminal ABP tagged BiTE® antibodies under the influence of 50% Human Serum

| BiTE® | CD4(1+2)xaCD3 | CD4(1+2)xaCD3xSA21 |
|---|---|---|
| $EC_{50}$ [ng/ml] | 0.24 | 1.2 |
| Factor* | 1 | 5 |
| BiTE® | CD4(1+2)xaCD3LxSA21 | CD4(1+2)xaCD3LxSA21 |
| $EC_{50}$ [ng/ml] | 1.2 | 1.7 |
| Factor* | 5 | 7.08 |
| BiTE® | CD4(1+2)xaCD3xSA25 | |
| $EC_{50}$ [ng/ml] | 0.74 | |
| Factor* | 3.08 | |
| Factor*: Factor describes the $EC_{50}$ value difference of the designated bispecific molecule compared to the parental bispecific molecule without ABP tag. | | |

Comparing the $EC_{50}$ values of C-terminal SA21 tagged bispecific molecules (Table 15, Figure 5(4)) with the N-terminal SA21 tagged bispecific molecules (Table 13, Figure 5(2)) indicates a significant negative influence in the cytotoxic activity for N-terminal SA21 tagged bispecific molecules indicated by increased $EC_{50}$ values in presence of 50% Human Serum. This phenomenon can be observed irrelevant of the position of the SA21 tag at the molecule's terminus, i.e. no restoration of cytotoxic activity by the addition of five AA or 15 AA linker between the ABP tag and the neighboring binding domain. In numbers, N-terminal SA21 tagged BiTE® molecules show a 11 to 23 fold decrease in cytotoxic actvitiy compared to 5 to 7 fold decrease when the SA21 tag is added to the C-terminus of the bispecific molecule. The described observation can also be seen in the presence of 10% FCS, i.e. less influence of serum albumin due to lower number of molecules and different species. A decrease of six to 16 fold for N-terminal SA21 tagged bispecific molecules (Table 12) compared to 2 to 3 fold decrease for C-terminal SA21 tagged BiTE® antibodies (Table 14).

Table 16: $EC_{50}$ values of BiTE® antibodies ABP tagged between the target and CD3 binding domain under the influence of 10% FCS

| BiTE® | CD4(1+2)xaCD3 | CD4(1+2)SA21xaCD3 |
|---|---|---|
| $EC_{50}$ [ng/ml] | 0.055 | 0.75 |
| Factor* | 1 | 13.64 |
| BiTE® | CD4(1+2)LxSA21LxaCD3 | CD4(1+2)LxSA21LxaCD3 |
| $EC_{50}$ [ng/ml] | 0.99 | 3.7 |

(continued)

| | | |
|---|---|---|
| **Factor*** | 18 | 67.28 |
| Factor*: Factor describes the $EC_{50}$ value difference of the designated bispecific molecule compared to the parental bispecific molecule without ABP tag. | | |

Table 17: $EC_{50}$ values of BiTE® antibodies ABP tagged between the target and CD3 binding domain under the influence of 50% Human Serum

| **BiTE®** | **CD4(1+2)xaCD3** | **CD4(1+2)SA21xaCD3** |
|---|---|---|
| **$EC_{50}$ [ng/ml]** | **0.11** | **5.1** |
| **Factor*** | **1** | **43.36** |
| **BiTE®** | **CD4(1+2)LSA21LxaCD3** | **CD4(1+2)LxSA21LxaCD3** |
| **$EC_{50}$ [ng/ml]** | **2.9** | **~8.0** |
| **Factor*** | **26.36** | **72.72** |
| Factor*: Factor describes the $EC_{50}$ value difference of the designated bispecific molecule compared to the parental bispecific molecule without ABP tag. | | |

Locating the SA21 ABP tag between the HIV and CD3 binding specificities leads to a decrease in cytotoxic activity in the presence of 10% FCS dependent of the length of the ABP tag insertion (Table 16 and figure 5(5)). This decrease in cytotoxic activity is partially exaggerated in the presence of 50% Human Serum (Table 17 and figure 5(6)).

**Example 10:** Biacore-based determination of BiTE®-antibody affinity to serum albumin of different species

To confirm binding of the tagged HIV specific BiTE® molecules to human serum albumin and serum albumines of other species, the following serum albumin preparations were provided: Human serum albumin (HSA), Sigma A9511; Mouse serum albumin (MSA), Sigma A3139;

Binding experiments were performed using plasmon resonance measurements in a Biacore machine. In detail, CM5 Sensor Chips (GE Healthcare) were immobilized with the respective serum albumin protein using acetate buffer pH 3 according to the manufacturer's manual. The BiTE® antibody samples were loaded five relevant concentrations e.g.: 400 nM, 200 nM, 100 nM, 50 nM, 25 nM (low concentrated BiTE®'s concentrations: 200 nM, 100 nM, 50 nM, 25 nM, 12.5 nM) diluted in HBS-EP running buffer (GE Healthcare). For affinity determinations the flow rate was 35 μl/min for 3 min, then HBS-EP running buffer was applied for 6 min again at a flow rate of 35 μl/ml. Regeneration of the chip was performed using a buffer consisting of 100 mM glycine 0.5 M NaCl pH 3.0 solution for 60 seconds. Data sets were analyzed using BiaEval Software. In some cases binding curves could perfectly or not at all be matched with the theoretical curves underlying the evaluation software. In the latter case the binding was determined as "positive"; in the case of closer match numbers are presented but have to be seen as approximation.

Binding affinities of the respective BiTE® antibodies to HSA as determined by Biacore are summarized in table 18 and figure 6.

Table 18: Affinities (KD) of tagged HIV BiTE® antibody against human serum albumin measured in Biacore experiments (surface plasmon resonance). HSA = human serum albumine, MSA = mouse serum albumin, "Positive" means detectable binding which could not be determined accurately by the BiaEval software; "Negative" means no significant binding under the chosen experimental conditions.

| ***BiTE® antibody*** | ***KD [nM] HSA*** | ***KD [nM] MSA*** |
|---|---|---|
| **CD4(1+2)xaCD3** | Negative | Negative |
| **SA21CD4(1+2)xaCD3** | 226 | 87 |
| **SA21LCD4(1+2)xaCD3** | 536 | 72 |
| **SA21LxCD4(1+2)xaCD3** | 260 | 107 |
| **SA25CD4(1+2)xaCD3** | Positive | Positive |

(continued)

| *BiTE® antibody* | *KD [nM] HSA* | *KD [nM] MSA* |
|---|---|---|
| **CD4(1+2)xaCD3SA21** | 201 | 113 |
| **CD4(1+2)xaCD3LSA21** | Positive | Positive |
| **CD4(1+2)xaCD3LxSA21** | Positive | Positive |
| **CD4(1+2)xaCD3SA25** | 198 | 63 |
| **CD4(1+2)SA21aCD3** | 465 | 70 |
| **CD4(1+2)LSA21LaCD3** | 773 | 231 |
| **CD4(1+2)LxSA21LxI2C** | 4520 | 8210 |

Concerning the affinity of the ABP tagged bispecific molecules they all bound to the immobilized HSA and MSA proteins. The bispecific molecules harboring the ABP tag between their binding specificities showed worse affinities to HSA and MSA than the bispecific molecules tagged with SA21 or SA25 at either one of their termini.

**Example 11:** Biacore-based determination of BiTE®-antibody affinity to human CD3

To confirm binding of the C-terminal ABP tagged HIV BiTE® molecules to human CD3, a human CD3 peptide comprising the CD3 epitope was fused to human Fc according to standard procedures and used for the affinity measurement.

Binding experiments were performed using plasmon resonance measurements in a Biacore machine. In detail, CM5 Sensor Chips (GE Healthcare) were immobilized with the human CD3 x huFc protein using acetate buffer pH 4.5 according to the manufacturer's manual. The BiTE antibody samples were loaded five relevant concentrations e.g.: 50 nM, 25 nM, 12.5nM, 6.25 nM, 3.125 nM diluted in HBS-EP running buffer (GE Healthcare). For affinity determinations the flow rate was 35 μl/min for 3 min, then HBS-EP running buffer was applied for 8 min again at a flow rate of 35 μl/ml. Regeneration of the chip was performed using a buffer consisting of 100 mM glycine 0.5 M NaCl pH 1.5 solution for 60 seconds. Data sets were analyzed using BiaEval Software.

Table 19: Affinities (KD) of tagged HIV BiTE® antibody against human CD3 FC fusion protein measured in Biacore experiments (surface plasmon resonance).

| *BiTE® antibody* | *KD [nM] huCD3* | **Factor*** |
|---|---|---|
| **CD4(1+2)xaCD3** | 2.31 | 1 |
| **CD4(1+2)xaCD3xSA21** | 4.33 | 1.9 |
| **CD4(1+2)xaCD3xSA25** | 3.77 | 1.6 |
| Factor*: Decreased CD3 affinity in comparison to parental BiTE® molecule without ABP tag | | |

The C-terminal addition of an ABP tag leads to a maximal 2 fold decrease of the neighboring binding specificity (i.e. CD3), see table 19 and figure 7.

**Example 12:** Biacore-based determination of BiTE®-antibody affinity to HIV gp120

To confirm binding of the N-terminal ABP tagged HIV BiTE® molecules to HIV gp120, the following HIV gp120 was provided: Recombinant HIV-1 IIIB Envelope Glycoprotein gp120 (Baculovirus), Product# 1001, Immunodiagnostics, Incorporation.

Binding experiments were performed using plasmon resonance measurements in a Biacore machine. In detail, CM5 Sensor Chips (GE Healthcare) were immobilized with the respective HIV gp120 protein using acetate buffer pH 4.5 according to the manufacturer's manual. The BiTE antibody samples were loaded five relevant concentrations e.g.: 100nM, 50 nM, 25 nM, 12.5nM, 6.25 nM, 3.125 nM diluted in HBS-EP running buffer (GE Healthcare). For affinity determinations the flow rate was 35 μl/min for 3 min, then HBS-EP running buffer was applied for 7 min again at a flow rate of 35 μl/ml. Regeneration of the chip was performed using a buffer consisting of 4 M MgCl2 solution for 60 seconds. Data sets were analyzed using BiaEval Software.

Table 20: Affinities (KD) of tagged HIV BiTE® antibody against HIV gp120 protein measured in Biacore experiments (surface plasmon resonance).

| ***BiTE® antibody*** | ***KD [nM] gp120*** | ***Factor**** |
|---|---|---|
| **CD4**(1+2)**xaCD3** | 0.71 | 1 |
| **SA21CD4(1+2)xaCD3** | 4.61 | 6.5 |
| **SA25CD4**(1+2)**xaCD3** | 10.3 | 14.5 |
| Factor*: Decreased HIV gp120 affinity in comparison to parental BiTE® molecule without ABP tag | | |

The N-terminal addition of an ABP tag leads to 6 to 14 fold decrease of the neighboring binding specificity (i.e. anti HIV gp120), see table 20 and figure 8.

**Example 13:** Purification scheme of BiTE® antibodies with a c-terminal ABP

Human embryonal kidney (HEK 293-F, Invitrogen) cells were transiently transfected with 200 μg of a pEF DHFR vector coding the BiTE® antibody sequence using 293 fectin (according to the manufacturer's manual, Invitrogen). Transfected cells were cultivated in Erlenmeyer cell culture flasks for 72 hours at 37 °C, 135 rpm, 5% $CO_2$. Alternatively, Chinese hamster ovary CHO cells stably transfected with a pEF DHFR vector coding the BiTE® antibody sequence were cultivated in roller bottles till 60% remaining vitality was reached. Cell culture supernatant was cleared by centrifugation at 4'000 rpm for 10 minutes and subsequent filtration (0.2 μm) and applied to a column filled with a zinc loaded chelating gel Fractogel EMD Chelate (Merck, Darmstadt) for immobilized metal affinity chromatography (IMAC) capture.

After sample loading remaining cell culture supernatant was washed out and after a pre-elution step the HIS6 tagged BiTE® antibodies were eluted by applying a buffer containing 500 mM imidazol.

Eluted volume containing the BiTE® antibodies was given on a Superdex S200 (GE Healthcare, Munich) size exclusion chromatography (SEC) column to separate the BiTE monomeric protein from dimeric BiTE® protein and other proteins.

Fractions containing the BiTE® monomeric protein were pooled and protein concentration was determined by measurement of optical absorption at 280 nm wavelength and 1 cm lightpath length and multiplication with the protein sequence specific absorption factor.

Exemplary purification showing the SEC chromatogram of the unmodified CD4(1+2)xaCD3LxSA21 BiTE® is shown in figure 9.

The inventions illustratively described herein may suitably be practiced in the absence of any element or elements, limitation or limitations, not specifically disclosed herein. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

Sequences

| SEQ ID NO. | DESIGNATION | SOURCE | TYPE | SEQUENCE |
|---|---|---|---|---|
| 1 | Human CD3ε extracellul ar domain | human | Aa | QDGNEEMGGITQTPYKVSISGTTVILTCPQYPGSEILWQHNDKNIGGDEDDKNIGSDEDHLSLKEFSELEQS GYYVCYPRGSKPEDANFYLYLRARVCENCMEMD |
| 2 | Human CD3ε 1-27 | human | Aa | QDGNEEMGGITQTPYKVSISGTTVILT |
| 3 | *Callithrix jacchus* CD3ε extracellular domain | *Callithrix jacchus* | Aa | QDGNEEMGDTTQNPYKVSISGTTVTLTCPRYDGHEIKWLVNSQNKEGHEDHLLLEDFSEMEQSGYYACLSKE TPAEEASHYLYLKARVCENCVEVD |
| 4 | *Callithrix jacchus* CD3ε 1-27 | *Callithrix jacchus* | aa | QDGNEEMGDTTQNPYKVSISGTTVTLT |
| 5 | *Saguinus oedipus* CD3ε extracellular domain | *Saguinus oedipus* | aa | QDGNEEMGDTTQNPYKVSISGTTVTLTCPRYDGHEIKWLVNSQNKEGHEDHLLLEDFSEMEQSGYYACLSKE TPAEEASHYLYLKARVCENCVEVD |
| 6 | *Saguinus oedipus* CD3ε 1-27 | *Saguinus oedipus* | aa | QDGNEEMGDTTQNPYKVSISGTTVTLT |
| 7 | *Saimiri sciureus* CD3ε extracellular domain | *Saimiri sciureus* | aa | QDGNEEIGDTTQNPYKVSISGTTVTLTCPRYDGQEIKWLVNDQNKEGHEDHLLLEDFSEMEQSGYYACLSKE TPTEEASHYLYLKARVCENCVEVD |
| 8 | *Saimiri sciureus* CD3ε 1-27 | *Saimiri sciureus* | aa | QDGNEEIGDTTQNPYKVSISGTTVTLT |
| 9 | SA04 | artificial | aa | DICLPRWGCLW |
| 10 | SA04 | artificial | nt | GACATCTGCCTGCCTAGATGGGGCTGCCTGTGG |
| 11 | SA08 | artificial | aa | QGLIGDICLPRWGCLWGDSVK |
| 12 | SA08 | artificial | nt | CGGCTGATCGAGGACATCTGCCTGCCCAGATGGGGCTGCCTGTGGGAGGACGAC |
| 13 | SA21 | artificial | aa | RLIEDICLPRWGCLWEDD |
| 14 | SA21 | artificial | nt | CGGCTGATCGAGGACATCTGCCTGCCCAGATGGGGCTGCCTGTGGGAGGACGAC |
| 15 | SA25 | artificial | aa | EDICLPRWGCLWED |
| 16 | SA25 | artificial | nt | GAGGACATCTGCCTGCCCAGATGGGGCTGCCTGTGGGAGGAC |
| 17 | DX236 | artificial | aa | AEGTGDFWFCDRIAWYPQHLCEFLDPE |

(continued)

| SEQ ID NO. | DESIGNATION | SOURCE | TYPE | SEQUENCE |
|---|---|---|---|---|
| 18 | DX236 | artificial | nt | GCTGAGGGCACCGGCGATTTCTGGTTCTGCGACCGGATCGCCTGGTATCCCCAGCACCTGTGCGAGTTTCTG GACCCCGAA |
| 19 | DX321 | artificial | aa | AEGTGDRNMCKFSWIRSPAFCARADPE |
| 20 | DX321 | artificial | nt | GCTGAGGGCACCGGCGACCGGAACATGTGCAAGTTCAGCTGGATCCGGTCCCCCGCCTTCTGCGCCAGAGCC GACCCTGAA |
| 21 | AB01 | artificial | aa | AASYSDYDVFGGGTDFGP |
| 22 | AB01 | artificial | nt | GCCGCTAGCTACTCCGACTACGACGTGTTCGGCGGAGGCACCGACTTCGGGCCA |
| 23 | AB14 | artificial | aa | AARYWDYDVFGGGTPVGG |
| 24 | AB14 | artificial | nt | GCCGCTCGGTACTGGGACTACGACGTGTTCGGCGGAGGCACACCCGTGGGGGGC |
| 25 | AB156 | artificial | aa | AARDWDFDVFGGGTPVGG |
| 26 | AB156 | artificial | nt | GCCGCTCGGGACTGGGACTTCGACGTGTTCGGCGGAGGCACACCCGTGGGGGGC |
| 27 | EpCAM x CD3 x SA21 x His-tag | artificial | nt | GAGCTCGTGATGACACAGTCTCCATCCTCCCTGACTGTGACAGCAGGAGAGAAGGTCACTATGAGCTGCAAG TCCAGTCAGAGTCTGTTAAACAGTGGAAATCAAAAGAACTACTTGACCTGGTACCAGCAGAAACCAGGGCAG CCTCCTAAACTGTTGATCTACTGGGCATCCACTAGGGAATCTGGGGTCCCTGATCGCTTCACAGGCAGTGGA TCTGGAACAGATTTCACTCTCACCATCAGCAGTGTGCAGGCTGAAGACCTGGCAGTTTATTACTGTCAGAAT GATTATAGTTATCCGCTCACGTTCGGTGCTGGGACCAAGCTTGAGATCAAAGGTGGTGGTGGTTCTGGCGGC GGCGGCTCCGGTGGTGGTGGTTCTGAGGTGCAGCTGCTCGAGCAGTCTGGAGCTGAGCTGGTAAGGCCTGGG |

(continued)

| SEQ ID NO. | DESIGNATION | SOURCE | TYPE | SEQUENCE |
|---|---|---|---|---|
| | | | | ACTTCAGTGAAGATATCCTGCAAGGCTTCTGGATACGCCTTCACTAACTACTGGCTAGGTTGGGTAAAGCAG<br>AGGCCTGGACATGGACTTGAGTGGATTGGAGATATTTTCCCTGGAAGTGGTAATATCCACTACAATGAGAAG<br>TTCAAGGGCAAAGCCACACTGACTGCAGACAAATCTTCGAGCACAGCCTATATGCAGCTCAGTAGCCTGACA<br>TTTGAGGACTCTGCTGTCTATTTCTGTGCAAGACTGAGGAACTGGGACGAGCCTATGGACTACTGGGGCCAA<br>GGGACCACGGTCACCGTCTCCTCCGGAGGTGGTGGCTCCGACGTCCAACTGGTGCAGTCAGGGGCTGAAGTG<br>AAAAAACCTGGGGCCTCAGTGAAGGTGTCCTGCAAGGCTTCTGGCTACACCTTTACTAGGTACACGATGCAC<br>TGGGTAAGGCAGGCACCTGGACAGGGTCTGGAATGGATTGGATACATTAATCCTAGCCGTGGTTATACTAAT<br>TACGCAGACAGCGTCAAGGGCCGCTTCACAATCACTACAGACAAATCCACCAGCACAGCCTACATGGAACTG<br>AGCAGCCTGCGTTCTGAGGACACTGCAACCTATTACTGTGCAAGATATTATGATGATCATTACTGCCTTGAC<br>TACTGGGGCCAAGGCACCACGGTCACCGTCTCCTCAGGCGAAGGTACTAGTACTGGTTCTGGTGGAAGTGGA<br>GGTTCAGGTGGAGCAGACGACATTGTACTGACCCAGTCTCCAGCAACTCTGTCTCTGTCTCCAGGGGAGCGT<br>GCCACCCTGAGCTGCAGAGCCAGTCAAAGTGTAAGTTACATGAACTGGTACCAGCAGAAGCCGGGCAAGGCA<br>CCCAAAAGATGGATTTATGACACATCCAAAGTGGCTTCTGGAGTCCCTGCTCGCTTCAGTGGCAGTGGGTCT<br>GGGACCGACTACTCTCTCACAATCAACAGCTTGGAGGCTGAAGATGCTGCCACTTATTACTGCCAACAGTGG<br>AGTAGTAACCCGCTCACGTTCGGTGGCGGGACCAAGGTGGAGATCAAACGGCTGATCGAGGACATCTGCCTG<br>CCCAGATGGGGCTGCCTGTGGGAGGACGACCATCATCACCATCATCAT |
| 28 | EpCAM x CD3 x SA21 x His-tag | artificial | aa | ELVMTQSPSSLTVTAGEKVTMSCKSSQSLLNSGNQKNYLTWYQQKPGQPPKLLIYWASTRESGVPDRFTGSG<br>SGTDFTLTISSVQAEDLAVYYCQNDYSYPLTFGAGTKLEIKGGGGSGGGGSGGGGSEVQLLEQSGAELVRPG<br>TSVKISCKASGYAFTNYWLGWVKQRPGHGLEWIGDIFPGSGNIHYNEKFKGKATLTADKSSSTAYMQLSSLT<br>FEDSAVYFCARLRNWDEPMDYWGQGTTVTVSSGGGGSDVQLVQSGAEVKKPGASVKVSCKASGYTFTRYTMH<br>WVRQAPGQGLEWIGYINPSRGYTNYADSVKGRFTITTDKSTSTAYMELSSLRSEDTATYYCARYYDDHYCLD<br>YWGQGTTVTVSSGEGTSTGSGGSGGSGGADDIVLTQSPATLSLSPGERATLSCRASQSVSYMNWYQQKPGKA<br>PKRWIYDTSKVASGVPARFSGSGSGTDYSLTINSLEAEDAATYYCQQWSSNPLTFGGGTKVEIKRLIEDICL<br>PRWGCLWEDDHHHHHH |
| 29 | EpCAM x CD3 x SA21 | artificial | aa | ELVMTQSPSSLTVTAGEKVTMSCKSSQSLLNSGNQKNYLTWYQQKPGQPPKLLIYWASTRESGVPDRFTGSG<br>SGTDFTLTISSVQAEDLAVYYCQNDYSYPLTFGAGTKLEIKGGGGSGGGGSGGGGSEVQLLEQSGAELVRPG<br>TSVKISCKASGYAFTNYWLGWVKQRPGHGLEWIGDIFPGSGNIHYNEKFKGKATLTADKSSSTAYMQLSSLT<br>FEDSAVYFCARLRNWDEPMDYWGQGTTVTVSSGGGGSDVQLVQSGAEVKKPGASVKVSCKASGYTFTRYTMH<br>WVRQAPGQGLEWIGYINPSRGYTNYADSVKGRFTITTDKSTSTAYMELSSLRSEDTATYYCARYYDDHYCLD<br>YWGQGTTVTVSSGEGTSTGSGGSGGSGGADDIVLTQSPATLSLSPGERATLSCRASQSVSYMNWYQQKPGKA<br>PKRWIYDTSKVASGVPARFSGSGSGTDYSLTINSLEAEDAATYYCQQWSSNPLTFGGGTKVEIKRLIEDICL<br>PRWGCLWEDD |

(continued)

| SEQ ID NO. | DESIGNATION | SOURCE | TYPE | SEQUENCE |
|---|---|---|---|---|
| 30 | CD4(1+2) | artificial | NT | AAGAAAGTGGTGCTGGGCAAGAAAGGCGACACCGTGGAACTGACCTGCACCGCCTCCCAGAAGAAGTCCATC CAGTTCCACTGGAAGAACTCCAACCAGATCAAGATCCTGGGCAACCAGGGCAGCTTCCTGACCAAGGGCCCC TCCAAGCTGAACGACCGGGCCGACTCCAGACGGTCCCTGTGGGATCAGGGCAACTTCCCACTGATCATCAAG AACCTGAAGATCGAGGACTCCGACACCTACATCTGCGAGGTGGAAGATCAGAAAGAAGAGGTGCAGCTGCTG GTGTTCGGCCTGACCGCCAACAGCGACACCCATCTGCTGCAGGGCCAGAGCCTGACCCTGACCCTGGAAAGC CCCCCTGGCTCCAGCCCTTCCGTGCAGTGCCGGTCCCCTCGGGGCAAGAACATCCAGGGCGGCAAGACCCTG TCCGTGTCCCAGCTGGAACTGCAGGACAGCGGCACCTGGACCTGTACCGTGCTGCAGAACCAGAAAAAGGTG |
| | | | | GAATTCAAGATCGACATCGTGGTGCTGGCCTTCCAGAAGGCT |
| 31 | CD4(1+2) | artificial | AA | KKVVLGKKGDTVELTCTASQKKSIQFHWKNSNQIKILGNQGSFLTKGPSKLNDRADSRRSLWDQGNFPLIIK NLKIEDSDTYICEVEDQKEEVQLLVFGLTANSDTHLLQGQSLTLTLESPPGSSPSVQCRSPRGKNIQGGKTL SVSQLELQDSGTWTCTVLQNQKKVEFKIDIVVLAFQKA |
| 32 | CD4(1+2)xaCD3 x His tag | artificial | NT | AAGAAAGTGGTGCTGGGCAAGAAAGGCGACACCGTGGAACTGACCTGCACCGCCTCCCAGAAGAAGTCCATC CAGTTCCACTGGAAGAACTCCAACCAGATCAAGATCCTGGGCAACCAGGGCAGCTTCCTGACCAAGGGCCCC TCCAAGCTGAACGACCGGGCCGACTCCAGACGGTCCCTGTGGGATCAGGGCAACTTCCCACTGATCATCAAG AACCTGAAGATCGAGGACTCCGACACCTACATCTGCGAGGTGGAAGATCAGAAAGAAGAGGTGCAGCTGCTG GTGTTCGGCCTGACCGCCAACAGCGACACCCATCTGCTGCAGGGCCAGAGCCTGACCCTGACCCTGGAAAGC CCCCCTGGCTCCAGCCCTTCCGTGCAGTGCCGGTCCCCTCGGGGCAAGAACATCCAGGGCGGCAAGACCCTG TCCGTGTCCCAGCTGGAACTGCAGGACAGCGGCACCTGGACCTGTACCGTGCTGCAGAACCAGAAAAAGGTG GAATTCAAGATCGACATCGTGGTGCTGGCCTTCCAGAAGGCTTCCGGAGGTGGTGGATCCGAGGTGCAGCTG GTCGAGTCTGGAGGAGGATTGGTGCAGCCTGGAGGGTCATTGAAACTCTCATGTGCAGCCTCTGGATTCACC TTCAATAAGTACGCCATGAACTGGGTCCGCCAGGCTCCAGGAAAGGGTTTGGAATGGGTTGCTCGCATAAGA AGTAAATATAATAATTATGCAACATATTATGCCGATTCAGTGAAAGACAGGTTCACCATCTCCAGAGATGAT TCAAAAAACACTGCCTATCTACAAATGAACAACTTGAAGACTGAGGACACTGCCGTGTACTACTGTGTGAGA CATGGGAACTTCGGTAATAGCTACATATCCTACTGGGCTTACTGGGGCCAAGGGACTCTGGTCACCGTCTCC TCAGGTGGTGGTGGTTCTGGCGGCGGCGGCTCCGGTGGTGGTGGTTCTCAGACTGTTGTGACTCAGGAACCT TCACTCACCGTATCACCTGGTGGAACAGTCACACTCACTTGTGGCTCCTCGACTGGGGCTGTTACATCTGGC AACTACCCAAACTGGGTCCAACAAAAACCAGGTCAGGCACCCCGTGGTCTAATAGGTGGGACTAAGTTCCTC GCCCCCGGTACTCCTGCCAGATTCTCAGGCTCCCTGCTTGGAGGCAAGGCTGCCCTCACCCTCTCAGGGGTA CAGCCAGAGGATGAGGCAGAATATTACTGTGTTCTATGGTACAGCAACCGCTGGGTGTTCGGTGGAGGAACC AAACTGACTGTCCTACATCATCACCATCATCAT |

(continued)

| SEQ ID NO. | DESIGNATION | SOURCE | TYPE | SEQUENCE |
|---|---|---|---|---|
| 33 | CD4(1+2)xaCD3 x His tag | artificial | AA | KKVVLGKKGDTVELTCTASQKKSIQFHWKNSNQIKILGNQGSFLTKGPSKLNDRADSRRSLWDQGNFPLIIK NLKIEDSDTYICEVEDQKEEVQLLVFGLTANSDTHLLQGQSLTLTLESPPGSSPSVQCRSPRGKNIQGGKTL SVSQLELQDSGTWTCTVLQNQKKVEFKIDIVVLAFQKASGGGGSEVQLVESGGGLVQPGGSLKLSCAASGFT FNKYAMNWVRQAPGKGLEWVARIRSKYNNYATYYADSVKDRFTISRDDSKNTAYLQMNNLKTEDTAVYYCVR HGNFGNSYISYWAYWGQGTLVTVSSGGGGSGGGGSGGGGSQTVVTQEPSLTVSPGGTVTLTCGSSTGAVTSG NYPNWVQQKPGQAPRGLIGGTKFLAPGTPARFSGSLLGGKAALTLSGVQPEDEAEYYCVLWYSNRWVFGGGT KLTVLHHHHHH |
| 34 | CD4 D1.1 | artificial | NT | AAGAAAGTCGTGATCGGCAAGAAAGGCGACACCGTGGAACTGACCTGCACCGCCTCCCAGAAGAAGTCCATC CAGTTCCACTGGAAGAACTCCAACCAGATCAAGATCCTGGGCAACCAGGGCAGCTTCCTGACCAAGGGCCCC TCCAAGCTGAACGACCGGGTGGACTCTCGGAGATCCCTGTGGGATCAGGGCAACTTCCCACTGATCATCAAG AACCTGAAGCCCGAGGACTCCGACACCTACATCTGCGAGGTGGAAGATCAGAAAGAAGAGGTGCAGCTGATC GTGCTGGGC |
| 35 | CD4 D1.1 | artificial | AA | KKVVIGKKGDTVELTCTASQKKSIQFHWKNSNQIKILGNQGSFLTKGPSKLNDRVDSRRSLWDQGNFPLIIK NLKPEDSDTYICEVEDQKEEVQLIVLG |
| 36 | CD4 D1.1xaCD3 x | artificial | NT | AAGAAAGTCGTGATCGGCAAGAAAGGCGACACCGTGGAACTGACCTGCACCGCCTCCCAGAAGAAGTCCATC CAGTTCCACTGGAAGAACTCCAACCAGATCAAGATCCTGGGCAACCAGGGCAGCTTCCTGACCAAGGGCCCC |
| | His tag | | | TCCAAGCTGAACGACCGGGTGGACTCTCGGAGATCCCTGTGGGATCAGGGCAACTTCCCACTGATCATCAAG AACCTGAAGCCCGAGGACTCCGACACCTACATCTGCGAGGTGGAAGATCAGAAAGAAGAGGTGCAGCTGATC GTGCTGGGCTCCGGAGGTGGTGGATCCGAGGTGCAGCTGGTCGAGTCTGGAGGAGGATTGGTGCAGCCTGGA GGGTCATTGAAACTCTCATGTGCAGCCTCTGGATTCACCTTCAATAAGTACGCCATGAACTGGGTCCGCCAG GCTCCAGGAAAGGGTTTGGAATGGGTTGCTCGCATAAGAAGTAAATATAATAATTATGCAACATATTATGCC GATTCAGTGAAAGACAGGTTCACCATCTCCAGAGATGATTCAAAAAACACTGCCTATCTACAAATGAACAAC TTGAAGACTGAGGACACTGCCGTGTACTACTGTGTGAGACATGGGAACTTCGGTAATAGCTACATATCCTAC TGGGCTTACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAACAGTCACA CTCACTTGTGGCTCCTCGACTGGGGCTGTTACATCTGGCAACTACCCAAACTGGGTCCAACAAAAACCAGGT CAGGCACCCCGTGGTCTAATAGGTGGGACTAAGTTCCTCGCCCCCGGTACTCCTGCCAGATTCTCAGGCTCC CTGCTTGGAGGCAAGGCTGCCCTCACCCTCTCAGGGGTACAGCCAGAGGATGAGGCAGAATATTACTGTGTT CTATGGTACAGCAACCGCTGGGTGTTCGGTGGAGGAACCAAACTGACTGTCCTACATCATCACCATCATCAT |

(continued)

| SEQ ID NO. | DESIGNATION | SOURCE | TYPE | SEQUENCE |
|---|---|---|---|---|
| 37 | CD4 D1.1xaCD3 x His tag | artificial | AA | KKVVIGKKGDTVELTCTASQKKSIQFHWKNSNQIKILGNQGSFLTKGPSKLNDRVDSRRSLWDQGNFPLIIK<br>NLKPEDSDTYICEVEDQKEEVQLIVLGSGGGGSEVQLVESGGGLVQPGGSLKLSCAASGFTFNKYAMNWVRQ<br>APGKGLEWVARIRSKYNNYATYYADSVKDRFTISRDDSKNTAYLQMNNLKTEDTAVYYCVRHGNFGNSYISY<br>WAYWGQGTLVTVSSGGGGSGGGGSGGGGSQTVVTQEPSLTVSPGGTVTLTCGSSTGAVTSGNYPNWVQQKPG<br>QAPRGLIGGTKFLAPGTPARFSGSLLGGKAALTLSGVQPEDEAEYYCVLWYSNRWVFGGGTKLTVLHHHHHH |
| 38 | CD4 D1.2 | artificial | NT | AAGAAAGTGGTGTACGGCAAGAAAGGCGACACCGTGGAACTGACCTGCACCGCCTCCCAGAAGAAGAACATC<br>CAGTTCCACTGGAAGAACTCCAACCAGATCAAGATCCTGGGCAACCAGGGCAGCTTCCTGACCAAGGGCCCC<br>TCCAAGCTGAACGACCGGGCCGACTCTCGGAGATCCCTGTGGGATCAGGGCAACTTCCCACTGATCATCAAG<br>AACCTGAAGCCCGAGGACTCCGACACCTACATCTGCGAGGTGGAAGATCAGAAAGAAGAGGTGCAGCTGGTG<br>GTCGTGGGC |
| 39 | CD4 D1.2 | artificial | AA | KKVVYGKKGDTVELTCTASQKKNIQFHWKNSNQIKILGNQGSFLTKGPSKLNDRADSRRSLWDQGNFPLIIK<br>NLKPEDSDTYICEVEDQKEEVQLVVVG |
| 40 | CD4 D1.2xaCD3 x His tag | artificial | NT | AAGAAAGTGGTGTACGGCAAGAAAGGCGACACCGTGGAACTGACCTGCACCGCCTCCCAGAAGAAGAACATC<br>CAGTTCCACTGGAAGAACTCCAACCAGATCAAGATCCTGGGCAACCAGGGCAGCTTCCTGACCAAGGGCCCC<br>TCCAAGCTGAACGACCGGGCCGACTCTCGGAGATCCCTGTGGGATCAGGGCAACTTCCCACTGATCATCAAG<br>AACCTGAAGCCCGAGGACTCCGACACCTACATCTGCGAGGTGGAAGATCAGAAAGAAGAGGTGCAGCTGGTG<br>GTCGTGGGCTCCGGAGGTGGTGGATCCGAGGTGCAGCTGGTCGAGTCTGGAGGAGGATTGGTGCAGCCTGGA<br>GGGTCATTGAAACTCTCATGTGCAGCCTCTGGATTCACCTTCAATAAGTACGCCATGAACTGGGTCCGCCAG<br>GCTCCAGGAAAGGGTTTGGAATGGGTTGCTCGCATAAGAAGTAAATATAATAATTATGCAACATATTATGCC<br>GATTCAGTGAAAGACAGGTTCACCATCTCCAGAGATGATTCAAAAAACACTGCCTATCTACAAATGAACAAC<br>TTGAAGACTGAGGACACTGCCGTGTACTACTGTGTGAGACATGGGAACTTCGGTAATAGCTACATATCCTAC<br>TGGGCTTACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAACAGTCACA<br>CTCACTTGTGGCTCCTCGACTGGGGCTGTTACATCTGGCAACTACCCAAACTGGGTCCAACAAAAACCAGGT<br>CAGGCACCCCGTGGTCTAATAGGTGGGACTAAGTTCCTCGCCCCCGGTACTCCTGCCAGATTCTCAGGCTCC<br>CTGCTTGGAGGCAAGGCTGCCCTCACCCTCTCAGGGGTACAGCCAGAGGATGAGGCAGAATATTACTGTGTT |
| | | | | CTATGGTACAGCAACCGCTGGGTGTTCGGTGGAGGAACCAAACTGACTGTCCTACATCATCACCATCATCAT |
| 41 | CD4 D1.2xaCD3 x His tag | artificial | AA | KKVVYGKKGDTVELTCTASQKKNIQFHWKNSNQIKILGNQGSFLTKGPSKLNDRADSRRSLWDQGNFPLIIK<br>NLKPEDSDTYICEVEDQKEEVQLVVVGSGGGGSEVQLVESGGGLVQPGGSLKLSCAASGFTFNKYAMNWVRQ<br>APGKGLEWVARIRSKYNNYATYYADSVKDRFTISRDDSKNTAYLQMNNLKTEDTAVYYCVRHGNFGNSYISY<br>WAYWGQGTLVTVSSGGGGSGGGGSGGGGSQTVVTQEPSLTVSPGGTVTLTCGSSTGAVTSGNYPNWVQQKPG<br>QAPRGLIGGTKFLAPGTPARFSGSLLGGKAALTLSGVQPEDEAEYYCVLWYSNRWVFGGGTKLTVLHHHHHH |

(continued)

| SEQ ID NO. | DESIGNATION | SOURCE | TYPE | SEQUENCE |
|---|---|---|---|---|
| 42 | SA21CD4(1+2) | artificial | NT | CGGCTGATCGAGGACATCTGCCTGCCTAGATGGGGCTGCCTGTGGGAGGACGACAAGAAAGTGGTGCTGGGC AAGAAAGGCGACACCGTGGAACTGACCTGCACCGCCTCCCAGAAGAAGTCCATCCAGTTCCACTGGAAGAAC TCCAACCAGATCAAGATCCTGGGCAACCAGGGCAGCTTCCTGACCAAGGGCCCCTCCAAGCTGAACGACCGG GCCGACTCTCGGAGATCCCTGTGGGATCAGGGCAACTTCCCACTGATCATCAAGAACCTGAAGATCGAGGAT TCCGACACCTACATCTGCGAGGTGGAAGATCAGAAAGAAGAGGTGCAGCTGCTGGTGTTCGGCCTGACCGCC AACTCCGATACCCATCTGCTGCAGGGCCAGTCCCTGACCCTGACACTGGAATCTCCACCCGGCTCCAGCCCT TCCGTGCAGTGCAGATCTCCCAGAGGCAAGAACATCCAGGGCGGCAAGACCCTGTCCGTGTCCCAGCTGGAA CTGCAGGACTCTGGCACCTGGACCTGTACCGTGCTGCAGAACCAGAAAAAGGTGGAATTCAAGATCGACATC GTGGTGCTGGCCTTCCAGAAGGCC |
| 43 | SA21CD4(1+2) | artificial | AA | RLIEDICLPRWGCLWEDDKKVVLGKKGDTVELTCTASQKKSIQFHWKNSNQIKILGNQGSFLTKGPSKLNDR ADSRRSLWDQGNFPLIIKNLKIEDSDTYICEVEDQKEEVQLLVFGLTANSDTHLLQGQSLTLTLESPPGSSP SVQCRSPRGKNIQGGKTLSVSQLELQDSGTWTCTVLQNQKKVEFKIDIVVLAFQKA |
| 44 | SA21CD4(1+2)x aCD3 x His tag | artificial | NT | CGGCTGATCGAGGACATCTGCCTGCCTAGATGGGGCTGCCTGTGGGAGGACGACAAGAAAGTGGTGCTGGGC AAGAAAGGCGACACCGTGGAACTGACCTGCACCGCCTCCCAGAAGAAGTCCATCCAGTTCCACTGGAAGAAC TCCAACCAGATCAAGATCCTGGGCAACCAGGGCAGCTTCCTGACCAAGGGCCCCTCCAAGCTGAACGACCGG GCCGACTCTCGGAGATCCCTGTGGGATCAGGGCAACTTCCCACTGATCATCAAGAACCTGAAGATCGAGGAT TCCGACACCTACATCTGCGAGGTGGAAGATCAGAAAGAAGAGGTGCAGCTGCTGGTGTTCGGCCTGACCGCC AACTCCGATACCCATCTGCTGCAGGGCCAGTCCCTGACCCTGACACTGGAATCTCCACCCGGCTCCAGCCCT TCCGTGCAGTGCAGATCTCCCAGAGGCAAGAACATCCAGGGCGGCAAGACCCTGTCCGTGTCCCAGCTGGAA CTGCAGGACTCTGGCACCTGGACCTGTACCGTGCTGCAGAACCAGAAAAAGGTGGAATTCAAGATCGACATC GTGGTGCTGGCCTTCCAGAAGGCCTCCGGAGGTGGTGGATCCGAGGTGCAGCTGGTCGAGTCTGGAGGAGGA TTGGTGCAGCCTGGAGGGTCATTGAAACTCTCATGTGCAGCCTCTGGATTCACCTTCAATAAGTACGCCATG AACTGGGTCCGCCAGGCTCCAGGAAAGGGTTTGGAATGGGTTGCTCGCATAAGAAGTAAATATAATAATTAT GCAACATATTATGCCGATTCAGTGAAAGACAGGTTCACCATCTCCAGAGATGATTCAAAAAACACTGCCTAT CTACAAATGAACAACTTGAAGACTGAGGACACTGCCGTGTACTACTGTGTGAGACATGGGAACTTCGGTAAT AGCTACATATCCTACTGGGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGTATCACCT GGTGGAACAGTCACACTCACTTGTGGCTCCTCGACTGGGGCTGTTACATCTGGCAACTACCCAAACTGGGTC CAACAAAAACCAGGTCAGGCACCCCGTGGTCTAATAGGTGGGACTAAGTTCCTCGCCCCCGGTACTCCTGCC AGATTCTCAGGCTCCCTGCTTGGAGGCAAGGCTGCCCTCACCCTCTCAGGGGTACAGCCAGAGGATGAGGCA GAATATTACTGTGTTCTATGGTACAGCAACCGCTGGGTGTTCGGTGGAGGAACCAAACTGACTGTCCTACAT CATCACCATCATCAT |

(continued)

| SEQ ID NO. | DESIGNATION | SOURCE | TYPE | SEQUENCE |
|---|---|---|---|---|
| 45 | SA21CD4(1+2)x aCD3 x His tag | artificial | AA | RLIEDICLPRWGCLWEDDKKVVLGKKGDTVELTCTASQKKSIQFHWKNSNQIKILGNQGSFLTKGPSKLNDRADSRRSLWDQGNFPLIIKNLKIEDSDTYICEVEDQKEEVQLLVFGLTANSDTHLLQGQSLTLTLESPPGSSPSVQCRSPRGKNIQGGKTLSVSQLELQDSGTWTCTVLQNQKKVEFKIDIVVLAFQKASGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLHHHHHH |
| 46 | SA25CD4(1+2) | artificial | NT | GAGGACATCTGCCTGCCCAGATGGGGCTGCCTGTGGGAGGACAAGAAAGTGGTGCTGGGCAAGAAAGGCGACACCGTGGAACTGACCTGCACCGCCTCCCAGAAGAAGTCCATCCAGTTCCACTGGAAGAACTCCAACCAGATCAAGATCCTGGGCAACCAGGGCAGCTTCCTGACCAAGGGCCCCTCCAAGCTGAACGACCGGGCCGACTCTCGGAGATCCCTGTGGGATCAGGGCAACTTCCCACTGATCATCAAGAACCTGAAGATCGAGGACTCCGACACCTACATCTGCGAGGTGGAAGATCAGAAAGAAGAGGTGCAGCTGCTGGTGTTCGGCCTGACCGCCAACAGCGACACCCATCTGCTGCAGGGCCAGTCCCTGACCCTGACACTGGAATCTCCACCCGGCTCCAGCCCTTCCGTGCAGTGCAGATCTCCCAGAGGCAAGAACATCCAGGGCGGCAAGACCCTGTCCGTGTCCCAGCTGGAACTGCAGGACTCTGGCACCTGGACCTGTACCGTGCTGCAGAACCAGAAAAAGGTGGAATTCAAGATCGACATCGTGGTGCTGGCCTTCCAGAAGGCC |
| 47 | SA25CD4(1+2) | artificial | AA | EDICLPRWGCLWEDKKVVLGKKGDTVELTCTASQKKSIQFHWKNSNQIKILGNQGSFLTKGPSKLNDRADSRRSLWDQGNFPLIIKNLKIEDSDTYICEVEDQKEEVQLLVFGLTANSDTHLLQGQSLTLTLESPPGSSPSVQCRSPRGKNIQGGKTLSVSQLELQDSGTWTCTVLQNQKKVEFKIDIVVLAFQKA |

(continued)

| SEQ ID NO. | DESIGNATION | SOURCE | TYPE | SEQUENCE |
|---|---|---|---|---|
| 48 | SA25CD4(1+2)x aCD3 x His tag | artificial | NT | GAGGACATCTGCCTGCCCAGATGGGGCTGCCTGTGGGAGGACAAGAAAGTGGTGCTGGGCAAGAAAGGCGAC<br>ACCGTGGAACTGACCTGCACCGCCTCCCAGAAGAAGTCCATCCAGTTCCACTGGAAGAACTCCAACCAGATC<br>AAGATCCTGGGCAACCAGGGCAGCTTCCTGACCAAGGGCCCCTCCAAGCTGAACGACCGGGCCGACTCTCGG<br>AGATCCCTGTGGGATCAGGGCAACTTCCCACTGATCATCAAGAACCTGAAGATCGAGGACTCCGACACCTAC<br>ATCTGCGAGGTGGAAGATCAGAAAGAAGAGGTGCAGCTGCTGGTGTTCGGCCTGACCGCCAACAGCGACACC<br>CATCTGCTGCAGGGCCAGTCCCTGACCCTGACACTGGAATCTCCACCCGGCTCCAGCCCTTCCGTGCAGTGC<br>AGATCTCCCAGAGGCAAGAACATCCAGGGCGGCAAGACCCTGTCCGTGTCCCAGCTGGAACTGCAGGACTCT<br>GGCACCTGGACCTGTACCGTGCTGCAGAACCAGAAAAAGGTGGAATTCAAGATCGACATCGTGGTGCTGGCC<br>TTCCAGAAGGCCTCCGGAGGTGGTGGATCCGAGGTGCAGCTGGTCGAGTCTGGAGGAGGATTGGTGCAGCCT<br>GGAGGGTCATTGAAACTCTCATGTGCAGCCTCTGGATTCACCTTCAATAAGTACGCCATGAACTGGGTCCGC<br>CAGGCTCCAGGAAAGGGTTTGGAATGGGTTGCTCGCATAAGAAGTAAATATAATAATTATGCAACATATTAT<br>GCCGATTCAGTGAAAGACAGGTTCACCATCTCCAGAGATGATTCAAAAAACACTGCCTATCTACAAATGAAC<br>AACTTGAAGACTGAGGACACTGCCGTGTACTACTGTGTGAGACATGGGAACTTCGGTAATAGCTACATATCC<br>TACTGGGCTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGGAACAGTC<br>ACACTCACTTGTGGCTCCTCGACTGGGGCTGTTACATCTGGCAACTACCCAAACTGGGTCCAACAAAAACCA<br>GGTCAGGCACCCCGTGGTCTAATAGGTGGGACTAAGTTCCTCGCCCCCGGTACTCCTGCCAGATTCTCAGGC<br>TCCCTGCTTGGAGGCAAGGCTGCCCTCACCCTCTCAGGGGTACAGCCAGAGGATGAGGCAGAATATTACTGT<br>GTTCTATGGTACAGCAACCGCTGGGTGTTCGGTGGAGGAACCAAACTGACTGTCCTACATCATCACCATCAT<br>CAT |
| 49 | SA25CD4(1+2)x aCD3 x His tag | artificial | AA | EDICLPRWGCLWEDKKVVLGKKGDTVELTCTASQKKSIQFHWKNSNQIKILGNQGSFLTKGPSKLNDRADSR<br>RSLWDQGNFPLIIKNLKIEDSDTYICEVEDQKEEVQLLVFGLTANSDTHLLQGQSLTLTLESPPGSSPSVQC<br>RSPRGKNIQGGKTLSVSQLELQDSGTWTCTVLQNQKKVEFKIDIVVLAFQKASGGGGSEVQLVESGGGLVQP<br>GGSLKLSCAASGFTFNKYAMNWVRQAPGKGLEWVARIRSKYNNYATYYADSVKDRFTISRDDSKNTAYLQMN<br>NLKTEDTAVYYCVRHGNFGNSYISYWAYWGQGTLVTVSSGGGGSGGGGSGGGGSQTVVTQEPSLTVSPGGTV<br>TLTCGSSTGAVTSGNYPNWVQQKPGQAPRGLIGGTKFLAPGTPARFSGSLLGGKAALTLSGVQPEDEAEYYC<br>VLWYSNRWVFGGGTKLTVLHHHHHH |

(continued)

| SEQ ID NO. | DESIGNATION | SOURCE | TYPE | SEQUENCE |
|---|---|---|---|---|
| 50 | CD4(1+2)xaCD3 SA21 x His tag | artificial | NT | AAGAAAGTGGTGCTGGGCAAGAAAGGCGACACCGTGGAACTGACCTGCACCGCCTCCCAGAAGAAGTCCATC CAGTTCCACTGGAAGAACTCCAACCAGATCAAGATCCTGGGCAACCAGGGCAGCTTCCTGACCAAGGGCCCC TCCAAGCTGAACGACCGGGCCGACTCCAGACGGTCCCTGTGGGATCAGGGCAACTTCCCACTGATCATCAAG AACCTGAAGATCGAGGACTCCGACACCTACATCTGCGAGGTGGAAGATCAGAAAGAAGAGGTGCAGCTGCTG GTGTTCGGCCTGACCGCCAACAGCGACACCCATCTGCTGCAGGGCCAGAGCCTGACCCTGACCCTGGAAAGC CCCCCTGGCTCCAGCCCTTCCGTGCAGTGCCGGTCCCCTCGGGGCAAGAACATCCAGGGCGGCAAGACCCTG TCCGTGTCCCAGCTGGAACTGCAGGACAGCGGCACCTGGACCTGTACCGTGCTGCAGAACCAGAAAAAGGTG GAATTCAAGATCGACATCGTGGTGCTGGCCTTCCAGAAGGCTTCCGGAGGTGGTGGATCCGAGGTGCAGCTG GTCGAGTCTGGAGGAGGATTGGTGCAGCCTGGAGGGTCATTGAAACTCTCATGTGCAGCCTCTGGATTCACC TTCAATAAGTACGCCATGAACTGGGTCCGCCAGGCTCCAGGAAAGGGTTTGGAATGGGTTGCTCGCATAAGA AGTAAATATAATAATTATGCAACATATTATGCCGATTCAGTGAAAGACAGGTTCACCATCTCCAGAGATGAT TCAAAAAACACTGCCTATCTACAAATGAACAACTTGAAGACTGAGGACACTGCCGTGTACTACTGTGTGAGA CATGGGAACTTCGGTAATAGCTACATATCCTACTGGGCTTACTGGGGCCAAGGGACTCTGGTCACCGTCTCC TCAGGTGGTGGTGGTTCTGGCGGCGGCGGCTCCGGTGGTGGTGGTTCTCAGACTGTTGTGACTCAGGAACCT TCACTCACCGTATCACCTGGTGGAACAGTCACACTCACTTGTGGCTCCTCGACTGGGGCTGTTACATCTGGC AACTACCCAAACTGGGTCCAACAAAAACCAGGTCAGGCACCCCGTGGTCTAATAGGTGGGACTAAGTTCCTC GCCCCCGGTACTCCTGCCAGATTCTCAGGCTCCCTGCTTGGAGGCAAGGCTGCCCTCACCCTCTCAGGGGTA CAGCCAGAGGATGAGGCAGAATATTACTGTGTTCTATGGTACAGCAACCGCTGGGTGTTCGGTGGAGGAACC AAACTGACTGTCCTACGCCTGATTGAAGATATTTGCCTGCCGCGCTGGGGCTGCCTGTGGGAAGATGATCAT CATCACCATCATCAT |
| 51 | CD4(1+2)xaCD3 SA21 x His tag | artificial | AA | KKVVLGKKGDTVELTCTASQKKSIQFHWKNSNQIKILGNQGSFLTKGPSKLNDRADSRRSLWDQGNFPLIIK NLKIEDSDTYICEVEDQKEEVQLLVFGLTANSDTHLLQGQSLTLTLESPPGSSPSVQCRSPRGKNIQGGKTL SVSQLELQDSGTWTCTVLQNQKKVEFKIDIVVLAFQKASGGGGSEVQLVESGGGLVQPGGSLKLSCAASGFT FNKYAMNWVRQAPGKGLEWVARIRSKYNNYATYYADSVKDRFTISRDDSKNTAYLQMNNLKTEDTAVYYCVR HGNFGNSYISYWAYWGQGTLVTVSSGGGGSGGGGSGGGGSQTVVTQEPSLTVSPGGTVTLTCGSSTGAVTSG NYPNWVQQKPGQAPRGLIGGTKFLAPGTPARFSGSLLGGKAALTLSGVQPEDEAEYYCVLWYSNRWVFGGGT KLTVLRLIEDICLPRWGCLWEDDHHHHHH |
| 52 | CD4(1+2)xaCD3 SA21 | artificial | AA | KKVVLGKKGDTVELTCTASQKKSIQFHWKNSNQIKILGNQGSFLTKGPSKLNDRADSRRSLWDQGNFPLIIK NLKIEDSDTYICEVEDQKEEVQLLVFGLTANSDTHLLQGQSLTLTLESPPGSSPSVQCRSPRGKNIQGGKTL SVSQLELQDSGTWTCTVLQNQKKVEFKIDIVVLAFQKASGGGGSEVQLVESGGGLVQPGGSLKLSCAASGFT FNKYAMNWVRQAPGKGLEWVARIRSKYNNYATYYADSVKDRFTISRDDSKNTAYLQMNNLKTEDTAVYYCVR HGNFGNSYISYWAYWGQGTLVTVSSGGGGSGGGGSGGGGSQTVVTQEPSLTVSPGGTVTLTCGSSTGAVTSG |
| | | | | NYPNWVQQKPGQAPRGLIGGTKFLAPGTPARFSGSLLGGKAALTLSGVQPEDEAEYYCVLWYSNRWVFGGGT KLTVLRLIEDICLPRWGCLWEDD |

(continued)

| SEQ ID NO. | DESIGNATION | SOURCE | TYPE | SEQUENCE |
|---|---|---|---|---|
| 53 | CD4(1+2)xaCD3 LSA21 x His tag | artificial | NT | AAGAAAGTGGTGCTGGGCAAGAAAGGCGACACCGTGGAACTGACCTGCACCGCCTCCCAGAAGAAGTCCATC<br>CAGTTCCACTGGAAGAACTCCAACCAGATCAAGATCCTGGGCAACCAGGGCAGCTTCCTGACCAAGGGCCCC<br>TCCAAGCTGAACGACCGGGCCGACTCCAGACGGTCCCTGTGGGATCAGGGCAACTTCCCACTGATCATCAAG<br>AACCTGAAGATCGAGGACTCCGACACCTACATCTGCGAGGTGGAAGATCAGAAAGAAGAGGTGCAGCTGCTG<br>GTGTTCGGCCTGACCGCCAACAGCGACACCCATCTGCTGCAGGGCCAGAGCCTGACCCTGACCCTGGAAAGC<br>CCCCCTGGCTCCAGCCCTTCCGTGCAGTGCCGGTCCCCTCGGGGCAAGAACATCCAGGGCGGCAAGACCCTG<br>TCCGTGTCCCAGCTGGAACTGCAGGACAGCGGCACCTGGACCTGTACCGTGCTGCAGAACCAGAAAAAGGTG<br>GAATTCAAGATCGACATCGTGGTGCTGGCCTTCCAGAAGGCTTCCGGAGGCGGAGGATCTGAAGTGCAGCTG<br>GTGGAATCTGGCGGCGGACTGGTGCAGCCTGGCGGATCTCTGAAGCTGTCTTGTGCCGCCAGCGGCTTCACC<br>TTCAACAAATACGCCATGAACTGGGTGCGACAGGCCCCTGGCAAGGGCCTGGAATGGGTGGCCCGGATCAGA<br>TCCAAGTACAACAACTACGCTACCTACTACGCCGACTCCGTGAAGGACCGGTTCACCATCTCCCGGGACGAC<br>TCCAAGAACACCGCCTACCTGCAGATGAACAACCTGAAAACCGAGGACACCGCCGTGTACTACTGCGTGCGG<br>CACGGCAACTTCGGCAACTCCTACATCAGCTACTGGGCCTACTGGGGCCAGGGCACCCTCGTGACAGTGTCA<br>TCAGGTGGCGGTGGATCTGGGGGAGGCGGTTCAGGCGGAGGGGGATCTCAGACAGTCGTGACCCAGGAACCC<br>TCCCTGACCGTGTCTCCTGGCGGAACCGTGACCCTGACCTGCGGATCTTCTACCGGCGCTGTGACCTCCGGC<br>AACTACCCTAATTGGGTGCAGCAGAAGCCCGGCCAGGCTCCCAGAGGACTGATCGGCGGCACCAAGTTTCTG<br>GCTCCCGGCACCCCTGCCAGATTCTCCGGTTCTCTGCTGGGCGGCAAGGCCGCTCTGACTCTGTCTGGGGTG<br>CAGCCAGAGGACGAGGCCGAGTACTATTGTGTGCTGTGGTACTCCAACCGCTGGGTGTTCGGCGGAGGCACC<br>AAGCTGACAGTGCTGGGTGGCGGAGGCTCTCGGCTGATCGAGGACATCTGCCTGCCTAGATGGGGCTGCCTG<br>TGGGAGGACGACCACCACCATCACCACCAC |
| 54 | CD4(1+2)xaCD3 LSA21 x His tag | artificial | AA | KKVVLGKKGDTVELTCTASQKKSIQFHWKNSNQIKILGNQGSFLTKGPSKLNDRADSRRSLWDQGNFPLIIK<br>NLKIEDSDTYICEVEDQKEEVQLLVFGLTANSDTHLLQGQSLTLTLESPPGSSPSVQCRSPRGKNIQGGKTL<br>SVSQLELQDSGTWTCTVLQNQKKVEFKIDIVVLAFQKASGGGGSEVQLVESGGGLVQPGGSLKLSCAASGFT<br>FNKYAMNWVRQAPGKGLEWVARIRSKYNNYATYYADSVKDRFTISRDDSKNTAYLQMNNLKTEDTAVYYCVR<br>HGNFGNSYISYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYSNRWVFGGGT<br>KLTVLGGGGSRLIEDICLPRWGCLWEDDHHHHHH |
| 55 | CD4(1+2)xaCD3 LSA21 | artificial | AA | KKVVLGKKGDTVELTCTASQKKSIQFHWKNSNQIKILGNQGSFLTKGPSKLNDRADSRRSLWDQGNFPLIIK<br>NLKIEDSDTYICEVEDQKEEVQLLVFGLTANSDTHLLQGQSLTLTLESPPGSSPSVQCRSPRGKNIQGGKTL<br>SVSQLELQDSGTWTCTVLQNQKKVEFKIDIVVLAFQKASGGGGSEVQLVESGGGLVQPGGSLKLSCAASGFT<br>FNKYAMNWVRQAPGKGLEWVARIRSKYNNYATYYADSVKDRFTISRDDSKNTAYLQMNNLKTEDTAVYYCVR<br>HGNFGNSYISYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYSNRWVFGGGT<br>KLTVLGGGGSRLIEDICLPRWGCLWEDD |

(continued)

| SEQ ID NO. | DESIGNATION | SOURCE | TYPE | SEQUENCE |
|---|---|---|---|---|
| 56 | CD4(1+2)xaCD3 LxSA21 x His | artificial | NT | AAGAAAGTGGTGCTGGGCAAGAAAGGCGACACCGTGGAACTGACCTGCACCGCCTCCCAGAAGAAGTCCATC<br>CAGTTCCACTGGAAGAACTCCAACCAGATCAAGATCCTGGGCAACCAGGGCAGCTTCCTGACCAAGGGCCCC<br>TCCAAGCTGAACGACCGGGCCGACTCCAGACGGTCCCTGTGGGATCAGGGCAACTTCCCACTGATCATCAAG |
| | tag | | | AACCTGAAGATCGAGGACTCCGACACCTACATCTGCGAGGTGGAAGATCAGAAAGAAGAGGTGCAGCTGCTG<br>GTGTTCGGCCTGACCGCCAACAGCGACACCCATCTGCTGCAGGGCCAGAGCCTGACCCTGACCCTGGAAAGC<br>CCCCCTGGCTCCAGCCCTTCCGTGCAGTGCCGGTCCCCTCGGGGCAAGAACATCCAGGGCGGCAAGACCCTG<br>TCCGTGTCCCAGCTGGAACTGCAGGACAGCGGCACCTGGACCTGTACCGTGCTGCAGAACCAGAAAAAGGTG<br>GAATTCAAGATCGACATCGTGGTGCTGGCCTTCCAGAAGGCTTCCGGAGGCGGAGGATCTGAAGTGCAGCTG<br>GTGGAATCTGGCGGCGGACTGGTGCAGCCTGGCGGATCTCTGAAGCTGTCTTGTGCCGCCAGCGGCTTCACC<br>TTCAACAAATACGCCATGAACTGGGTGCGACAGGCCCCTGGCAAGGGCCTGGAATGGGTGGCCCGGATCAGA<br>TCCAAGTACAACAACTACGCTACCTACTACGCCGACTCCGTGAAGGACCGGTTCACCATCTCCCGGGACGAC<br>TCCAAGAACACCGCCTACCTGCAGATGAACAACCTGAAAACCGAGGACACCGCCGTGTACTACTGCGTGCGG<br>CACGGCAACTTCGGCAACTCCTACATCAGCTACTGGGCCTACTGGGGCCAGGGCACCCTCGTGACAGTGTCA<br>TCAGGTGGCGGTGGATCTGGGGGAGGCGGTTCAGGCGGAGGGGGATCTCAGACAGTCGTGACCCAGGAACCC<br>TCCCTGACCGTGTCTCCTGGCGGAACCGTGACCCTGACCTGCGGATCTTCTACCGGCGCTGTGACCTCCGGC<br>AACTACCCTAATTGGGTGCAGCAGAAGCCCGGCCAGGCTCCCAGAGGACTGATCGGCGGCACCAAGTTTCTG<br>GCTCCCGGCACCCCTGCCAGATTCTCCGGTTCTCTGCTGGGCGGCAAGGCCGCTCTGACTCTGTCTGGGGTG<br>CAGCCAGAGGACGAGGCCGAGTACTATTGTGTGCTGTGGTACTCCAACCGCTGGGTGTTCGGCGGAGGCACC<br>AAGCTGACAGTGCTGGGTGGCGGAGGTTCTGGCGGGGGAGGCAGTGGGGGGGGAGGATCTAGACTGATCGAG<br>GACATCTGCCTGCCCAGATGGGGCTGCCTGTGGGAGGACGATCACCACCACCATCACCAC |
| 57 | CD4(1+2)xaCD3 LxSA21 x His tag | artificial | AA | KKVVLGKKGDTVELTCTASQKKSIQFHWKNSNQIKILGNQGSFLTKGPSKLNDRADSRRSLWDQGNFPLIIK<br>NLKIEDSDTYICEVEDQKEEVQLLVFGLTANSDTHLLQGQSLTLTLESPPGSSPSVQCRSPRGKNIQGGKTL<br>SVSQLELQDSGTWTCTVLQNQKKVEFKIDIVVLAFQKASGGGGSEVQLVESGGGLVQPGGSLKLSCAASGFT<br>FNKYAMNWVRQAPGKGLEWVARIRSKYNNYATYYADSVKDRFTISRDDSKNTAYLQMNNLKTEDTAVYYCVR<br>HGNFGNSYISYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYSNRWVFGGGT<br>KLTVLGGGGSGGGGSGGGGSRLIEDICLPRWGCLWEDDHHHHHH |
| 58 | CD4(1+2)xaCD3 LxSA21 | artificial | AA | KKVVLGKKGDTVELTCTASQKKSIQFHWKNSNQIKILGNQGSFLTKGPSKLNDRADSRRSLWDQGNFPLIIK<br>NLKIEDSDTYICEVEDQKEEVQLLVFGLTANSDTHLLQGQSLTLTLESPPGSSPSVQCRSPRGKNIQGGKTL<br>SVSQLELQDSGTWTCTVLQNQKKVEFKIDIVVLAFQKASGGGGSEVQLVESGGGLVQPGGSLKLSCAASGFT<br>FNKYAMNWVRQAPGKGLEWVARIRSKYNNYATYYADSVKDRFTISRDDSKNTAYLQMNNLKTEDTAVYYCVR<br>HGNFGNSYISYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYSNRWVFGGGT<br>KLTVLGGGGSGGGGSGGGGSRLIEDICLPRWGCLWEDD |

(continued)

| SEQ ID NO. | DESIGNATION | SOURCE | TYPE | SEQUENCE |
|---|---|---|---|---|
| 59 | CD4(1+2)xaCD3 SA25 x His tag | artificial | NT | AAGAAAGTGGTGCTGGGCAAGAAAGGCGACACCGTGGAACTGACCTGCACCGCCTCCCAGAAGAAGTCCATC<br>CAGTTCCACTGGAAGAACTCCAACCAGATCAAGATCCTGGGCAACCAGGGCAGCTTCCTGACCAAGGGCCCC<br>TCCAAGCTGAACGACCGGGCCGACTCCAGACGGTCCCTGTGGGATCAGGGCAACTTCCCACTGATCATCAAG<br>AACCTGAAGATCGAGGACTCCGACACCTACATCTGCGAGGTGGAAGATCAGAAAGAAGAGGTGCAGCTGCTG<br>GTGTTCGGCCTGACCGCCAACAGCGACACCCATCTGCTGCAGGGCCAGAGCCTGACCCTGACCCTGGAAAGC<br>CCCCCTGGCTCCAGCCCTTCCGTGCAGTGCCGGTCCCCTCGGGGCAAGAACATCCAGGGCGGCAAGACCCTG<br>TCCGTGTCCCAGCTGGAACTGCAGGACAGCGGCACCTGGACCTGTACCGTGCTGCAGAACCAGAAAAAGGTG<br>GAATTCAAGATCGACATCGTGGTGCTGGCCTTCCAGAAGGCTTCCGGAGGCGGAGGCTCTGAGGTGCAGCTG |
| | | | | GTGGAAAGTGGCGGCGGACTGGTGCAGCCTGGCGGCTCCCTGAAGCTGTCTTGCGCCGCCAGCGGCTTCACC<br>TTCAACAAATACGCCATGAACTGGGTCCGACAGGCTCCTGGCAAGGGCCTGGAATGGGTGGCCCGGATTCGG<br>TCCAAGTACAACAACTACGCCACCTACTACGCCGACTCCGTGAAGGACCGGTTCACCATCAGCCGGGACGAC<br>TCCAAGAACACCGCCTACCTGCAGATGAACAACCTGAAAACCGAGGACACCGCCGTGTACTACTGCGTGCGG<br>CACGGCAACTTCGGCAACTCCTACATCAGCTACTGGGCCTACTGGGGCCAGGGCACCCTGGTGACAGTGTCC<br>TCTGGCGGAGGCGGATCTGGGGGCGGAGGATCAGGCGGGGGAGGATCCCAGACCGTGGTGACACAGGAACCC<br>TCCCTGACCGTCTCTCCTGGGGGCACCGTGACCCTGACCTGCGGATCTTCCACCGGCGCTGTGACTAGTGGC<br>AACTACCCCAACTGGGTGCAGCAGAAGCCCGGCCAGGCCCCTAGAGGCCTGATCGGCGGCACCAAGTTTCTG<br>GCTCCCGGCACCCCTGCCCGGTTCTCTGGATCTCTGCTGGGCGGCAAGGCCGCTCTGACACTGTCTGGCGTG<br>CAGCCTGAGGACGAGGCCGAGTACTATTGTGTGCTGTGGTACTCCAACAGATGGGTGTTCGGCGGAGGCACC<br>AAGCTGACCGTGCTGGAAGATATCTGCCTGCCCAGATGGGGCTGCCTGTGGGAGGACCACCACCACCATCAC<br>CAC |
| 60 | CD4(1+2)xaCD3 SA25 x His tag | artificial | AA | KKVVLGKKGDTVELTCTASQKKSIQFHWKNSNQIKILGNQGSFLTKGPSKLNDRADSRRSLWDQGNFPLIIK<br>NLKIEDSDTYICEVEDQKEEVQLLVFGLTANSDTHLLQGQSLTLTLESPPGSSPSVQCRSPRGKNIQGGKTL<br>SVSQLELQDSGTWTCTVLQNQKKVEFKIDIVVLAFQKASGGGGSEVQLVESGGGLVQPGGSLKLSCAASGFT<br>FNKYAMNWVRQAPGKGLEWVARIRSKYNNYATYYADSVKDRFTISRDDSKNTAYLQMNNLKTEDTAVYYCVR<br>HGNFGNSYISYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYSNRWVFGGGT<br>KLTVLEDICLPRWGCLWEDHHHHHH |
| 61 | CD4(1+2)xaCD3 SA25 | artificial | AA | KKVVLGKKGDTVELTCTASQKKSIQFHWKNSNQIKILGNQGSFLTKGPSKLNDRADSRRSLWDQGNFPLIIK<br>NLKIEDSDTYICEVEDQKEEVQLLVFGLTANSDTHLLQGQSLTLTLESPPGSSPSVQCRSPRGKNIQGGKTL<br>SVSQLELQDSGTWTCTVLQNQKKVEFKIDIVVLAFQKASGGGGSEVQLVESGGGLVQPGGSLKLSCAASGFT<br>FNKYAMNWVRQAPGKGLEWVARIRSKYNNYATYYADSVKDRFTISRDDSKNTAYLQMNNLKTEDTAVYYCVR<br>HGNFGNSYISYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYSNRWVFGGGT<br>KLTVLEDICLPRWGCLWED |

(continued)

| SEQ ID NO. | DESIGNATION | SOURCE | TYPE | SEQUENCE |
|---|---|---|---|---|
| 62 | SA21 LCD4(1+2) xaCD3 x His tag | artificial | NT | CGGCTGATCGAGGACATCTGCCTGCCTAGATGGGGCTGCCTGTGGGAGGATGATGGCGGCGGAGGCTCCAAG AAAGTGGTGCTGGGCAAGAAAGGCGACACCGTGGAACTGACCTGCACCGCCTCCCAGAAGAAGTCCATCCAG TTCCACTGGAAGAACTCCAACCAGATCAAGATCCTGGGCAACCAGGGCAGCTTCCTGACCAAGGGCCCCTCC AAGCTGAACGACCGGGCCGACTCTCGGAGATCCCTGTGGGATCAGGGCAACTTCCCACTGATCATCAAGAAC CTGAAGATCGAGGATTCCGACACCTACATCTGCGAGGTGGAAGATCAGAAAGAAGAGGTGCAGCTGCTGGTG TTCGGCCTGACCGCCAACTCCGATACCCATCTGCTGCAGGGCCAGTCCCTGACCCTGACACTGGAATCTCCA CCCGGCTCCAGCCCTTCCGTGCAGTGCAGATCTCCCAGAGGCAAGAACATCCAGGGCGGCAAGACCCTGTCC GTGTCCCAGCTGGAACTGCAGGACTCTGGCACCTGGACCTGTACCGTGCTGCAGAACCAGAAAAAGGTGGAA TTCAAGATCGACATCGTGGTGCTGGCCTTCCAGAAGGCCTCCGGAGGTGGTGGATCCGAGGTGCAGCTGGTC GAGTCTGGAGGAGGATTGGTGCAGCCTGGAGGGTCATTGAAACTCTCATGTGCAGCCTCTGGATTCACCTTC AATAAGTACGCCATGAACTGGGTCCGCCAGGCTCCAGGAAAGGGTTTGGAATGGGTTGCTCGCATAAGAAGT AAATATAATAATTATGCAACATATTATGCCGATTCAGTGAAAGACAGGTTCACCATCTCCAGAGATGATTCA AAAAACACTGCCTATCTACAAATGAACAACTTGAAGACTGAGGACACTGCCGTGTACTACTGTGTGAGACAT |
| | | | | GGGAACTTCGGTAATAGCTACATATCCTACTGGGCTTACTGGGGCCAAGGGACTCTGGTCACCGTCTCCTCA GGTGGTGGTGGTTCTGGCGGCGGCGGCTCCGGTGGTGGTGGTTCTCAGACTGTTGTGACTCAGGAACCTTCA CTCACCGTATCACCTGGTGGAACAGTCACACTCACTTGTGGCTCCTCGACTGGGGCTGTTACATCTGGCAAC TACCCAAACTGGGTCCAACAAAAACCAGGTCAGGCACCCCGTGGTCTAATAGGTGGGACTAAGTTCCTCGCC CCCGGTACTCCTGCCAGATTCTCAGGCTCCCTGCTTGGAGGCAAGGCTGCCCTCACCCTCTCAGGGGTACAG CCAGAGGATGAGGCAGAATATTACTGTGTTCTATGGTACAGCAACCGCTGGGTGTTCGGTGGAGGAACCAAA CTGACTGTCCTACATCATCACCATCATCAT |
| 63 | SA21 LCD4(1+2) xaCD3 x His tag | artificial | AA | RLIEDICLPRWGCLWEDDGGGGSKKVVLGKKGDTVELTCTASQKKSIQFHWKNSNQIKILGNQGSFLTKGPS KLNDRADSRRSLWDQGNFPLIIKNLKIEDSDTYICEVEDQKEEVQLLVFGLTANSDTHLLQGQSLTLTLESP PGSSPSVQCRSPRGKNIQGGKTLSVSQLELQDSGTWTCTVLQNQKKVEFKIDIVVLAFQKASGGGGSEVQLV ESGGGLVQPGGSLKLSCAASGFTFNKYAMNWVRQAPGKGLEWVARIRSKYNNYATYYADSVKDRFTISRDDS KNTAYLQMNNLKTEDTAVYYCVRHGNFGNSYISYWAYWGQGTLVTVSSGGGGSGGGGSGGGGSQTVVTQEPS LTVSPGGTVTLTCGSSTGAVTSGNYPNWVQQKPGQAPRGLIGGTKFLAPGTPARFSGSLLGGKAALTLSGVQ PEDEAEYYCVLWYSNRWVFGGGTKLTVLHHHHHH |

(continued)

| SEQ ID NO. | DESIGNATION | SOURCE | TYPE | SEQUENCE |
|---|---|---|---|---|
| 64 | SA21 LxCD4(1+ 2) xaCD3 x His tag | artificial | NT | CGGCTGATCGAGGACATCTGCCTGCCTAGATGGGGCTGCCTGTGGGAGGATGATGGCGGCGGAGGATCTGGC<br>GGAGGTGGAAGCGGAGGGGGCGGATCCAAAAAGGTGGTGCTGGGCAAGAAAGGCGACACCGTGGAACTGACC<br>TGCACCGCCTCCCAGAAGAAGTCCATCCAGTTCCACTGGAAGAACTCCAACCAGATCAAGATCCTGGGCAAC<br>CAGGGCAGCTTCCTGACCAAGGGCCCCTCCAAGCTGAACGACCGGGCCGACTCTCGGAGATCCCTGTGGGAT<br>CAGGGCAACTTCCCACTGATCATCAAGAACCTGAAGATCGAGGATTCCGACACCTACATCTGCGAAGTGGAA<br>GATCAGAAAGAAGAGGTGCAGCTGCTGGTGTTCGGCCTGACCGCCAACTCCGATACCCATCTGCTGCAGGGC<br>CAGTCCCTGACCCTGACACTGGAATCTCCACCCGGCTCCAGCCCTTCCGTGCAGTGCAGATCTCCCAGAGGC<br>AAGAACATCCAGGGCGGCAAGACCCTGTCCGTGTCCCAGCTGGAACTGCAGGACTCTGGCACCTGGACCTGT<br>ACCGTGCTGCAGAACCAGAAAAAAGTGGAATTCAAGATCGACATCGTGGTGCTGGCCTTCCAGAAGGCCTCC<br>GGAGGTGGTGGATCCGAGGTGCAGCTGGTCGAGTCTGGAGGAGGATTGGTGCAGCCTGGAGGGTCATTGAAA<br>CTCTCATGTGCAGCCTCTGGATTCACCTTCAATAAGTACGCCATGAACTGGGTCCGCCAGGCTCCAGGAAAG<br>GGTTTGGAATGGGTTGCTCGCATAAGAAGTAAATATAATAATTATGCAACATATTATGCCGATTCAGTGAAA<br>GACAGGTTCACCATCTCCAGAGATGATTCAAAAAACACTGCCTATCTACAAATGAACAACTTGAAGACTGAG<br>GACACTGCCGTGTACTACTGTGTGAGACATGGGAACTTCGGTAATAGCTACATATCCTACTGGGCTTACTGG<br>GGCCAAGGGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCACTTGTGGC<br>TCCTCGACTGGGGCTGTTACATCTGGCAACTACCCAAACTGGGTCCAACAAAAACCAGGTCAGGCACCCCGT<br>GGTCTAATAGGTGGGACTAAGTTCCTCGCCCCCGGTACTCCTGCCAGATTCTCAGGCTCCCTGCTTGGAGGC<br>AAGGCTGCCCTCACCCTCTCAGGGGTACAGCCAGAGGATGAGGCAGAATATTACTGTGTTCTATGGTACAGC<br>AACCGCTGGGTGTTCGGTGGAGGAACCAAACTGACTGTCCTACATCATCACCATCATCAT |
| 65 | SA21 LxCD4(1+ 2) xaCD3 x His tag | artificial | AA | RLIEDICLPRWGCLWEDDGGGGSGGGGSGGGGSKKVVLGKKGDTVELTCTASQKKSIQFHWKNSNQIKILGN<br>QGSFLTKGPSKLNDRADSRRSLWDQGNFPLIIKNLKIEDSDTYICEVEDQKEEVQLLVFGLTANSDTHLLQG<br>QSLTLTLESPPGSSPSVQCRSPRGKNIQGGKTLSVSQLELQDSGTWTCTVLQNQKKVEFKIDIVVLAFQKAS<br>GGGGSEVQLVESGGGLVQPGGSLKLSCAASGFTFNKYAMNWVRQAPGKGLEWVARIRSKYNNYATYYADSVK<br>DRFTISRDDSKNTAYLQMNNLKTEDTAVYYCVRHGNFGNSYISYWAYWGQGTLVTVSSGGGGSGGGGSGGGG |
| | | | | SQTVVTQEPSLTVSPGGTVTLTCGSSTGAVTSGNYPNWVQQKPGQAPRGLIGGTKFLAPGTPARFSGSLLGG<br>KAALTLSGVQPEDEAEYYCVLWYSNRWVFGGGTKLTVLHHHHHH |

(continued)

| SEQ ID NO. | DESIGNATION | SOURCE | TYPE | SEQUENCE |
|---|---|---|---|---|
| 66 | CD4(1+2)SA21x aCD3 x His tag | artificial | NT | AAGAAAGTGGTGCTGGGCAAGAAAGGCGACACCGTGGAACTGACCTGCACCGCCAGCCAGAAGAAGTCCATC<br>CAGTTCCACTGGAAGAACAGCAACCAGATCAAGATCCTGGGCAACCAGGGCAGCTTCCTGACCAAGGGCCCC<br>AGCAAGCTGAACGACAGAGCCGACTCTCGGCGGAGCCTGTGGGACCAGGGCAATTTCCCACTGATCATCAAG<br>AACCTGAAGATCGAGGACAGCGACACCTACATCTGCGAGGTGGAAGATCAGAAAGAAGAGGTGCAGCTGCTG<br>GTGTTCGGCCTGACCGCCAACTCCGACACCCATCTGCTGCAGGGCCAGAGCCTGACCCTGACACTGGAAAGC<br>CCTCCAGGCAGCAGCCCCAGCGTGCAGTGTAGAAGCCCCAGAGGCAAGAACATCCAGGGCGGCAAGACCCTG<br>AGCGTGTCCCAGCTGGAACTGCAGGATAGCGGCACCTGGACCTGTACCGTGCTGCAGAACCAGAAAAAGGTG<br>GAGTTCAAGATCGACATCGTGGTGCTGGCCTTCCAGAAGGCCCGGCTGATCGAGGATATCTGCCTGCCCAGA<br>TGGGGCTGTCTGTGGGAGGACGACGAAGTGCAGCTGGTGGAATCTGGCGGCGGACTGGTGCAGCCTGGCGGA<br>TCTCTGAAGCTGAGCTGTGCCGCCAGCGGCTTCACCTTCAACAAATACGCCATGAACTGGGTGCGCCAGGCC<br>CCTGGCAAAGGCCTGGAATGGGTGGCCCGGATCAGAAGCAAGTACAACAACTATGCCACCTACTACGCCGAC<br>AGCGTGAAGGACCGGTTCACCATCAGCAGGGACGACTCCAAGAACACCGCCTACCTGCAGATGAACAACCTG<br>AAAACCGAGGATACCGCCGTGTACTACTGCGTGCGGCACGGCAACTTCGGCAACAGCTACATCAGCTACTGG<br>GCCTACTGGGGCCAGGGCACACTCGTGACAGTGTCTAGCGGAGGCGGAGGATCAGGCGGCGGAGGAAGTGGC<br>GGAGGGGGATCTCAGACAGTCGTGACCCAGGAACCCAGCCTGACCGTGTCTCCTGGCGGAACCGTGACACTG<br>ACATGCGGCAGCTCTACAGGCGCCGTGACCAGCGGCAACTACCCTAATTGGGTGCAGCAGAAGCCCGGACAG<br>GCCCCAAGAGGACTGATCGGCGGCACCAAGTTTCTGGCTCCCGGCACCCCTGCCAGATTCAGCGGCTCACTG<br>CTGGGAGGAAAGGCCGCCCTGACTCTGTCTGGGGTGCAGCCAGAGGATGAGGCCGAGTACTATTGTGTGCTG<br>TGGTACAGCAACCGCTGGGTGTTCGGAGGCGGCACAAAGCTGACAGTGCTGCACCACCACCATCACCAC |
| 67 | CD4(1+2)SA21x aCD3 x His tag | artificial | AA | KKVVLGKKGDTVELTCTASQKKSIQFHWKNSNQIKILGNQGSFLTKGPSKLNDRADSRRSLWDQGNFPLIIK<br>NLKIEDSDTYICEVEDQKEEVQLLVFGLTANSDTHLLQGQSLTLTLESPPGSSPSVQCRSPRGKNIQGGKTL<br>SVSQLELQDSGTWTCTVLQNQKKVEFKIDIVVLAFQKARLIEDICLPRWGCLWEDDEVQLVESGGGLVQPGG<br>SLKLSCAASGFTFNKYAMNWVRQAPGKGLEWVARIRSKYNNYATYYADSVKDRFTISRDDSKNTAYLQMNNL<br>KTEDTAVYYCVRHGNFGNSYISYWAYWGQGTLVTVSSGGGGSGGGGSGGGGSQTVVTQEPSLTVSPGGTVTL<br>TCGSSTGAVTSGNYPNWVQQKPGQAPRGLIGGTKFLAPGTPARFSGSLLGGKAALTLSGVQPEDEAEYYCVL<br>WYSNRWVFGGGTKLTVLHHHHHH |

(continued)

| SEQ ID NO. | DESIGNATION | SOURCE | TYPE | SEQUENCE |
|---|---|---|---|---|
| 68 | CD4(1+2)LSA21 LxaCD3 x His tag | artificial | NT | AAGAAAGTGGTGCTGGGCAAGAAAGGCGACACCGTGGAACTGACCTGCACCGCCAGCCAGAAGAAGTCCATC<br>CAGTTCCACTGGAAGAACAGCAACCAGATCAAGATCCTGGGCAACCAGGGCAGCTTCCTGACCAAGGGCCCC<br>AGCAAGCTGAACGACAGAGCCGACTCTCGGCGGAGCCTGTGGGACCAGGGCAATTTCCCACTGATCATCAAG<br>AACCTGAAGATCGAGGACAGCGACACCTACATCTGCGAGGTGGAAGATCAGAAAGAAGAGGTGCAGCTGCTG<br>GTGTTCGGCCTGACCGCCAACTCCGACACCCATCTGCTGCAGGGCCAGAGCCTGACCCTGACACTGGAAAGC<br>CCTCCAGGCAGCAGCCCCAGCGTGCAGTGTAGAAGCCCCAGAGGCAAGAACATCCAGGGCGGCAAGACCCTG<br>AGCGTGTCCCAGCTGGAACTGCAGGATAGCGGCACCTGGACCTGTACCGTGCTGCAGAACCAGAAAAAGGTG<br>GAGTTCAAGATCGACATCGTGGTGCTGGCCTTCCAGAAAGCCGGCGGAGGCGGCTCTAGACTGATCGAGGAT<br>ATCTGCCTGCCCAGATGGGGCTGTCTGTGGGAGGACGATTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATTCACCTTC<br>AATAAGTACGCCATGAACTGGGTCCGCCAGGCTCCAGGAAAGGGTTTGGAATGGGTTGCTCGCATAAGAAGT |
| | | | | AAATATAATAATTATGCAACATATTATGCCGATTCAGTGAAAGACAGGTTCACCATCTCCAGAGATGATTCA<br>AAAAACACTGCCTATCTACAAATGAACAACTTGAAGACTGAGGACACTGCCGTGTACTACTGTGTGAGACAT<br>GGGAACTTCGGTAATAGCTACATATCCTACTGGGCTTACTGGGGCCAAGGGACTCTGGTCACCGTCTCCTCA<br>GGTGGTGGTGGTTCTGGCGGCGGCGGCTCCGGTGGTGGTGGTTCTCAGACTGTTGTGACTCAGGAACCTTCA<br>CTCACCGTATCACCTGGTGGAACAGTCACACTCACTTGTGGCTCCTCGACTGGGGCTGTTACATCTGGCAAC<br>TACCCAAACTGGGTCCAACAAAAACCAGGTCAGGCACCCCGTGGTCTAATAGGTGGGACTAAGTTCCTCGCC<br>CCCGGTACTCCTGCCAGATTCTCAGGCTCCCTGCTTGGAGGCAAGGCTGCCCTCACCCTCTCAGGGGTACAG<br>CCAGAGGATGAGGCAGAATATTACTGTGTTCTATGGTACAGCAACCGCTGGGTGTTCGGTGGAGGAACCAAA<br>CTGACTGTCCTACATCATCACCATCATCAT |
| 69 | CD4(1+2)LSA21 LxaCD3 x His tag | artificial | AA | KKVVLGKKGDTVELTCTASQKKSIQFHWKNSNQIKILGNQGSFLTKGPSKLNDRADSRRSLWDQGNFPLIIK<br>NLKIEDSDTYICEVEDQKEEVQLLVFGLTANSDTHLLQGQSLTLTLESPPGSSPSVQCRSPRGKNIQGGKTL<br>SVSQLELQDSGTWTCTVLQNQKKVEFKIDIVVLAFQKAGGGGSRLIEDICLPRWGCLWEDDSGGGGSEVQLV<br>ESGGGLVQPGGSLKLSCAASGFTFNKYAMNWVRQAPGKGLEWVARIRSKYNNYATYYADSVKDRFTISRDDS<br>KNTAYLQMNNLKTEDTAVYYCVRHGNFGNSYISYWAYWGQGTLVTVSSGGGGSGGGGSGGGGSQTVVTQEPS<br>LTVSPGGTVTLTCGSSTGAVTSGNYPNWVQQKPGQAPRGLIGGTKFLAPGTPARFSGSLLGGKAALTLSGVQ<br>PEDEAEYYCVLWYSNRWVFGGGTKLTVLHHHHHH |

(continued)

| SEQ ID NO. | DESIGNATION | SOURCE | TYPE | SEQUENCE |
|---|---|---|---|---|
| 70 | CD4(1+2)LxSA2 1LxaCD3 x His tag | artificial | NT | AAGAAAGTGGTGCTGGGCAAGAAAGGCGACACCGTGGAACTGACCTGCACCGCCAGCCAGAAGAAGTCCATC CAGTTCCACTGGAAGAACAGCAACCAGATCAAGATCCTGGGCAACCAGGGCAGCTTCCTGACCAAGGGCCCC AGCAAGCTGAACGACAGAGCCGACTCTCGGCGGAGCCTGTGGGACCAGGGCAATTTCCCACTGATCATCAAG AACCTGAAGATCGAGGACAGCGACACCTACATCTGCGAGGTGGAAGATCAGAAAGAAGAGGTGCAGCTGCTG GTGTTCGGCCTGACCGCCAACTCCGACACCCATCTGCTGCAGGGCCAGAGCCTGACCCTGACACTGGAAAGC CCTCCAGGCAGCAGCCCCAGCGTGCAGTGTAGAAGCCCCAGAGGCAAGAACATCCAGGGCGGCAAGACCCTG AGCGTGTCCCAGCTGGAACTGCAGGATAGCGGCACCTGGACCTGTACCGTGCTGCAGAACCAGAAAAAGGTG GAGTTCAAGATCGACATCGTGGTGCTGGCCTTCCAGAAAGCCGGCGGAGGCGGATCTGGCGGCGGAGGATCT GGGGGAGGCGGCTCTAGACTGATCGAGGATATCTGCCTGCCCAGATGGGGCTGTCTGTGGGAGGATGATTCT GGCGGAGGGGGAAGTGGGGGGGGAGGATCCGGAGGTGGTGGATCCGAGGTGCAGCTGGTCGAGTCTGGAGGA GGATTGGTGCAGCCTGGAGGGTCATTGAAACTCTCATGTGCAGCCTCTGGATTCACCTTCAATAAGTACGCC ATGAACTGGGTCCGCCAGGCTCCAGGAAAGGGTTTGGAATGGGTTGCTCGCATAAGAAGTAAATATAATAAT TATGCAACATATTATGCCGATTCAGTGAAAGACAGGTTCACCATCTCCAGAGATGATTCAAAAAACACTGCC TATCTACAAATGAACAACTTGAAGACTGAGGACACTGCCGTGTACTACTGTGTGAGACATGGGAACTTCGGT AATAGCTACATATCCTACTGGGCTTACTGGGGCCAAGGGACTCTGGTCACCGTCTCCTCAGGTGGTGGTGGT TCTGGCGGCGGCGGCTCCGGTGGTGGTGGTTCTCAGACTGTTGTGACTCAGGAACCTTCACTCACCGTATCA CCTGGTGGAACAGTCACACTCACTTGTGGCTCCTCGACTGGGGCTGTTACATCTGGCAACTACCCAAACTGG GTCCAACAAAAACCAGGTCAGGCACCCCGTGGTCTAATAGGTGGGACTAAGTTCCTCGCCCCCGGTACTCCT GCCAGATTCTCAGGCTCCCTGCTTGGAGGCAAGGCTGCCCTCACCCTCTCAGGGGTACAGCCAGAGGATGAG GCAGAATATTACTGTGTTCTATGGTACAGCAACCGCTGGGTGTTCGGTGGAGGAACCAAACTGACTGTCCTA CATCATCACCATCATCAT |
| 71 | CD4(1+2)LxSA2 1LxaCD3 x His | artificial | AA | KKVVLGKKGDTVELTCTASQKKSIQFHWKNSNQIKILGNQGSFLTKGPSKLNDRADSRRSLWDQGNFPLIIK NLKIEDSDTYICEVEDQKEEVQLLVFGLTANSDTHLLQGQSLTLTLESPPGSSPSVQCRSPRGKNIQGGKTL |
| | tag | | | SVSQLELQDSGTWTCTVLQNQKKVEFKIDIVVLAFQKAGGGGSGGGGSGGGGSRLIEDICLPRWGCLWEDDS GGGGSGGGGSGGGGSEVQLVESGGGLVQPGGSLKLSCAASGFTFNKYAMNWVRQAPGKGLEWVARIRSKYNN YATYYADSVKDRFTISRDDSKNTAYLQMNNLKTEDTAVYYCVRHGNFGNSYISYWAYWGQGTLVTVSSGGGG SGGGGSGGGGSQTVVTQEPSLTVSPGGTVTLTCGSSTGAVTSGNYPNWVQQKPGQAPRGLIGGTKFLAPGTP ARFSGSLLGGKAALTLSGVQPEDEAEYYCVLWYSNRWVFGGGTKLTVLHHHHHH |

(continued)

| SEQ ID NO. | DESIGNATION | SOURCE | TYPE | SEQUENCE |
|---|---|---|---|---|
| 72 | B12HL | artificial | NT | CAAGTGCAGCTGGTGCAGTCTGGCGCCGAAGTGAAGAAACCTGGCGCCTCCGTGAAGGTGTCCTGCCAGGCC<br>TCCGGCTACCGGTTCTCCAACTTCGTGATCCACTGGGTGCGACAGGCCCCTGGCCAGAGATTCGAGTGGATG<br>GGCTGGATCAACCCCTACAACGGCAACAAAGAGTTCTCCGCCAAGTTCCAGGACAGAGTGACCTTCACCGCC<br>GACACCTCCGCCAACACCGCCTACATGGAACTGCGGTCCCTGAGAAGCGCCGACACCGCCGTGTACTACTGC<br>GCCAGAGTGGGCCCCTACTCCTGGGACGACTCCCCCCAGGACAACTACTACATGGACGTGTGGGGCAAGGGC<br>ACCACCGTGATCGTGTCCTCTGGCGGCGGAGGATCTGGCGGAGGCGGAAGTGGCGGAGGGGGCTCTGAGATC<br>GTGCTGACCCAGTCCCCCGGCACACTGTCTCTGAGCCCTGGCGAGCGGGCCACCTTCTCTTGCCGGTCCTCC<br>CACTCCATCCGGTCCAGACGGGTGGCCTGGTATCAGCACAAGCCAGGCCAGGCTCCTCGGCTGGTGATCCAC<br>GGCGTGTCCAACCGGGCCTCCGGCATCTCCGACAGATTCAGCGGCTCCGGCAGCGGCACCGACTTCACCCTG<br>ACCATCACCCGCGTGGAACCCGAGGACTTCGCCCTGTACTATTGCCAGGTGTACGGCGCCTCCTCCTACACC<br>TTCGGCCAGGGCACTAAGCTGGAACGGAAG |
| 73 | B12HL | artificial | AA | QVQLVQSGAEVKKPGASVKVSCQASGYRFSNFVIHWVRQAPGQRFEWMGWINPYNGNKEFSAKFQDRVTFTA<br>DTSANTAYMELRSLRSADTAVYYCARVGPYSWDDSPQDNYYMDVWGKGTTVIVSSGGGGSGGGGSGGGGSEI<br>VLTQSPGTLSLSPGERATFSCRSSHSIRSRRVAWYQHKPGQAPRLVIHGVSNRASGISDRFSGSGSGTDFTL<br>TITRVEPEDFALYYCQVYGASSYTFGQGTKLERK |
| 74 | B12HLxaCD3xS A21 x His tag | artificial | NT | CAAGTGCAGCTGGTGCAGTCTGGCGCCGAAGTGAAGAAACCTGGCGCCTCCGTGAAGGTGTCCTGCCAGGCC<br>TCCGGCTACCGGTTCTCCAACTTCGTGATCCACTGGGTGCGACAGGCCCCTGGCCAGAGATTCGAGTGGATG<br>GGCTGGATCAACCCCTACAACGGCAACAAAGAGTTCTCCGCCAAGTTCCAGGACAGAGTGACCTTCACCGCC<br>GACACCTCCGCCAACACCGCCTACATGGAACTGCGGTCCCTGAGAAGCGCCGACACCGCCGTGTACTACTGC<br>GCCAGAGTGGGCCCCTACTCCTGGGACGACTCCCCCCAGGACAACTACTACATGGACGTGTGGGGCAAGGGC<br>ACCACCGTGATCGTGTCCTCTGGCGGCGGAGGATCTGGCGGAGGCGGAAGTGGCGGAGGGGGCTCTGAGATC<br>GTGCTGACCCAGTCCCCCGGCACACTGTCTCTGAGCCCTGGCGAGCGGGCCACCTTCTCTTGCCGGTCCTCC<br>CACTCCATCCGGTCCAGACGGGTGGCCTGGTATCAGCACAAGCCAGGCCAGGCTCCTCGGCTGGTGATCCAC<br>GGCGTGTCCAACCGGGCCTCCGGCATCTCCGACAGATTCAGCGGCTCCGGCAGCGGCACCGACTTCACCCTG<br>ACCATCACCCGCGTGGAACCCGAGGACTTCGCCCTGTACTATTGCCAGGTGTACGGCGCCTCCTCCTACACC<br>TTCGGCCAGGGCACTAAGCTGGAACGGAAGTCCGGAGGTGGTGGATCCGAGGTGCAGCTGGTCGAGTCTGGA<br>GGAGGATTGGTGCAGCCTGGAGGGTCATTGAAACTCTCATGTGCAGCCTCTGGATTCACCTTCAATAAGTAC<br>GCCATGAACTGGGTCCGCCAGGCTCCAGGAAAGGGTTTGGAATGGGTTGCTCGCATAAGAAGTAAATATAAT<br>AATTATGCAACATATTATGCCGATTCAGTGAAAGACAGGTTCACCATCTCCAGAGATGATTCAAAAAACACT<br>GCCTATCTACAAATGAACAACTTGAAGACTGAGGACACTGCCGTGTACTACTGTGTGAGACATGGGAACTTC<br>GGTAATAGCTACATATCCTACTGGGCTTACTGGGGCCAAGGGACTCTGGTCACCGTCTCCTCAGGTGGTGGT<br>GGTTCTGGCGGCGGCGGCTCCGGTGGTGGTGGTTCTCAGACTGTTGTGACTCAGGAACCTTCACTCACCGTA<br>TCACCTGGTGGAACAGTCACACTCACTTGTGGCTCCTCGACTGGGGCTGTTACATCTGGCAACTACCCAAAC<br>TGGGTCCAACAAAAACCAGGTCAGGCACCCCGTGGTCTAATAGGTGGGACTAAGTTCCTCGCCCCCGGTACT |

(continued)

| SEQ ID NO. | DESIGNATION | SOURCE | TYPE | SEQUENCE |
|---|---|---|---|---|
| | | | | CCTGCCAGATTCTCAGGCTCCCTGCTTGGAGGCAAGGCTGCCCTCACCCTCTCAGGGGTACAGCCAGAGGAT GAGGCAGAATATTACTGTGTTCTATGGTACAGCAACCGCTGGGTGTTCGGTGGAGGAACCAAACTGACTGTC CTACGCCTGATTGAAGATATTTGCCTGCCGCGCTGGGGCTGCCTGTGGGAAGATGATCATCATCACCATCAT CAT |
| 75 | B12HLxaCD3xS A21 x His tag | artificial | AA | QVQLVQSGAEVKKPGASVKVSCQASGYRFSNFVIHWVRQAPGQRFEWMGWINPYNGNKEFSAKFQDRVTFTA DTSANTAYMELRSLRSADTAVYYCARVGPYSWDDSPQDNYYMDVWGKGTTVIVSSGGGGSGGGGSGGGGSEI VLTQSPGTLSLSPGERATFSCRSSHSIRSRRVAWYQHKPGQAPRLVIHGVSNRASGISDRFSGSGSGTDFTL TITRVEPEDFALYYCQVYGASSYTFGQGTKLERKSGGGGSEVQLVESGGGLVQPGGSLKLSCAASGFTFNKY AMNWVRQAPGKGLEWVARIRSKYNNYATYYADSVKDRFTISRDDSKNTAYLQMNNLKTEDTAVYYCVRHGNF GNSYISYWAYWGQGTLVTVSSGGGGSGGGGSGGGGSQTVVTQEPSLTVSPGGTVTLTCGSSTGAVTSGNYPN WVQQKPGQAPRGLIGGTKFLAPGTPARFSGSLLGGKAALTLSGVQPEDEAEYYCVLWYSNRWVFGGGTKLTV LRLIEDICLPRWGCLWEDDHHHHHH |
| 76 | B12HLxaCD3xS A21 | artificial | AA | QVQLVQSGAEVKKPGASVKVSCQASGYRFSNFVIHWVRQAPGQRFEWMGWINPYNGNKEFSAKFQDRVTFTA DTSANTAYMELRSLRSADTAVYYCARVGPYSWDDSPQDNYYMDVWGKGTTVIVSSGGGGSGGGGSGGGGSEI VLTQSPGTLSLSPGERATFSCRSSHSIRSRRVAWYQHKPGQAPRLVIHGVSNRASGISDRFSGSGSGTDFTL TITRVEPEDFALYYCQVYGASSYTFGQGTKLERKSGGGGSEVQLVESGGGLVQPGGSLKLSCAASGFTFNKY AMNWVRQAPGKGLEWVARIRSKYNNYATYYADSVKDRFTISRDDSKNTAYLQMNNLKTEDTAVYYCVRHGNF GNSYISYWAYWGQGTLVTVSSGGGGSGGGGSGGGGSQTVVTQEPSLTVSPGGTVTLTCGSSTGAVTSGNYPN WVQQKPGQAPRGLIGGTKFLAPGTPARFSGSLLGGKAALTLSGVQPEDEAEYYCVLWYSNRWVFGGGTKLTV LRLIEDICLPRWGCLWEDD |
| 77 | B12LH | artificial | NT | GAGATCGTGCTGACCCAGTCCCCCGGCACACTGTCTCTGAGCCCTGGCGAGCGGGCCACCTTCTCTTGCCGG TCCTCCCACTCCATCCGGTCCAGACGGGTGGCCTGGTATCAGCACAAGCCAGGCCAGGCCCCTCGGCTGGTG ATCCACGGCGTGTCCAACCGGGCCTCCGGCATCTCCGACAGATTCTCCGGCTCCGGCAGCGGCACCGACTTC ACCCTGACCATCACCCGCGTGGAACCCGAGGACTTCGCCCTGTACTACTGCCAGGTGTACGGCGCCTCCTCC TACACCTTCGGCCAGGGCACCAAGCTGGAAAGAAAGGGCGGAGGCGGCTCTGGTGGCGGAGGAAGTGGAGGC GGAGGATCTCAGGTGCAGCTGGTGCAGTCTGGCGCCGAAGTGAAGAAACCTGGCGCCTCCGTGAAGGTGTCC TGCCAGGCCAGCGGCTACCGGTTCTCCAACTTCGTGATCCACTGGGTGCGACAGGCACCTGGCCAGAGATTC GAGTGGATGGGCTGGATCAACCCCTACAACGGCAACAAAGAGTTCTCCGCCAAGTTCCAGGACAGAGTGACC TTCACCGCCGACACCTCCGCCAACACCGCCTACATGGAACTGCGGTCCCTGAGAAGCGCCGACACCGCTGTG TACTACTGTGCCAGAGTGGGCCCCTACTCCTGGGACGACTCCCCCCAGGACAACTACTACATGGACGTGTGG GGCAAGGGCACTACCGTGATCGTGTCTTCC |

(continued)

| SEQ ID NO. | DESIGNATION | SOURCE | TYPE | SEQUENCE |
|---|---|---|---|---|
| 78 | B12LH | artificial | AA | EIVLTQSPGTLSLSPGERATFSCRSSHSIRSRRVAWYQHKPGQAPRLVIHGVSNRASGISDRFSGSGSGTDF<br>TLTITRVEPEDFALYYCQVYGASSYTFGQGTKLERKGGGGSGGGGSGGGGSQVQLVQSGAEVKKPGASVKVS<br>CQASGYRFSNFVIHWVRQAPGQRFEWMGWINPYNGNKEFSAKFQDRVTFTADTSANTAYMELRSLRSADTAV<br>YYCARVGPYSWDDSPQDNYYMDVWGKGTTVIVSS |
| 79 | B12LHxaCD3xS A21 x His tag | artificial | NT | GAGATCGTGCTGACCCAGTCCCCCGGCACACTGTCTCTGAGCCCTGGCGAGCGGGCCACCTTCTCTTGCCGG<br>TCCTCCCACTCCATCCGGTCCAGACGGGTGGCCTGGTATCAGCACAAGCCAGGCCAGGCCCCTCGGCTGGTG<br>ATCCACGGCGTGTCCAACCGGGCCTCCGGCATCTCCGACAGATTCTCCGGCTCCGGCAGCGGCACCGACTTC<br>ACCCTGACCATCACCCGCGTGGAACCCGAGGACTTCGCCCTGTACTACTGCCAGGTGTACGGCGCCTCCTCC |
| | | | | TACACCTTCGGCCAGGGCACCAAGCTGGAAAGAAAGGGCGGAGGCGGCTCTGGTGGCGGAGGAAGTGGAGGC<br>GGAGGATCTCAGGTGCAGCTGGTGCAGTCTGGCGCCGAAGTGAAGAAACCTGGCGCCTCCGTGAAGGTGTCC<br>TGCCAGGCCAGCGGCTACCGGTTCTCCAACTTCGTGATCCACTGGGTGCGACAGGCACCTGGCCAGAGATTC<br>GAGTGGATGGGCTGGATCAACCCCTACAACGGCAACAAAGAGTTCTCCGCCAAGTTCCAGGACAGAGTGACC<br>TTCACCGCCGACACCTCCGCCAACACCGCCTACATGGAACTGCGGTCCCTGAGAAGCGCCGACACCGCTGTG<br>TACTACTGTGCCAGAGTGGGCCCCTACTCCTGGGACGACTCCCCCCAGGACAACTACTACATGGACGTGTGG<br>GGCAAGGGCACTACCGTGATCGTGTCTTCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAAGTACGCC<br>ATGAACTGGGTCCGCCAGGCTCCAGGAAAGGGTTTGGAATGGGTTGCTCGCATAAGAAGTAAATATAATAAT<br>TATGCAACATATTATGCCGATTCAGTGAAAGACAGGTTCACCATCTCCAGAGATGATTCAAAAAACACTGCC<br>TATCTACAAATGAACAACTTGAAGACTGAGGACACTGCCGTGTACTACTGTGTGAGACATGGGAACTTCGGT<br>AATAGCTACATATCCTACTGGGCTTACTGGGGCCAAGGGACTCTGGTCACCGTCTCCTCAGGTGGTGGTGGT<br>TCTGGCGGCGGCGGCTCCGGTGGTGGTGGTTCTCAGACTGTTGTGACTCAGGAACCTTCACTCACCGTATCA<br>CCTGGTGGAACAGTCACACTCACTTGTGGCTCCTCGACTGGGGCTGTTACATCTGGCAACTACCCAAACTGG<br>GTCCAACAAAAACCAGGTCAGGCACCCCGTGGTCTAATAGGTGGGACTAAGTTCCTCGCCCCCGGTACTCCT<br>GCCAGATTCTCAGGCTCCCTGCTTGGAGGCAAGGCTGCCCTCACCCTCTCAGGGGTACAGCCAGAGGATGAG<br>GCAGAATATTACTGTGTTCTATGGTACAGCAACCGCTGGGTGTTCGGTGGAGGAACCAAACTGACTGTCCTA<br>CGCCTGATTGAAGATATTTGCCTGCCGCGCTGGGGCTGCCTGTGGGAAGATGATCATCATCACCATCATCAT |
| 80 | B12LHxaCD3xS A21 x His tag | artificial | AA | EIVLTQSPGTLSLSPGERATFSCRSSHSIRSRRVAWYQHKPGQAPRLVIHGVSNRASGISDRFSGSGSGTDF<br>TLTITRVEPEDFALYYCQVYGASSYTFGQGTKLERKGGGGSGGGGSGGGGSQVQLVQSGAEVKKPGASVKVS<br>CQASGYRFSNFVIHWVRQAPGQRFEWMGWINPYNGNKEFSAKFQDRVTFTADTSANTAYMELRSLRSADTAV<br>YYCARVGPYSWDDSPQDNYYMDVWGKGTTVIVSSGGGGSEVQLVESGGGLVQPGGSLKLSCAASGFTFNKYA<br>MNWVRQAPGKGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGAVTSGNYPNW<br>VQQKPGQAPRGLIGGTKFLAPGTPARFSGSLLGGKAALTLSGVQPEDEAEYYCVLWYSNRWVFGGGTKLTVL<br>RLIEDICLPRWGCLWEDDHHHHHH |

(continued)

| SEQ ID NO. | DESIGNATION | SOURCE | TYPE | SEQUENCE |
|---|---|---|---|---|
| 81 | B12LHxaCD3xS A21 | artificial | AA | EIVLTQSPGTLSLSPGERATFSCRSSHSIRSRRVAWYQHKPGQAPRLVIHGVSNRASGISDRFSGSGSGTDF TLTITRVEPEDFALYYCQVYGASSYTFGQGTKLERKGGGGSGGGGSGGGGSQVQLVQSGAEVKKPGASVKVS CQASGYRFSNFVIHWVRQAPGQRFEWMGWINPYNGNKEFSAKFQDRVTFTADTSANTAYMELRSLRSADTAV YYCARVGPYSWDDSPQDNYYMDVWGKGTTVIVSSGGGGSEVQLVESGGGLVQPGGSLKLSCAASGFTFNKYA MNWVRQAPGKGLEWVARIRSKYNNYATYYADSVKDRFTISRDDSKNTAYLQMNNLKTEDTAVYYCVRHGNFG NSYISYWAYWGQGTLVTVSSGGGGSGGGGSGGGGSQTVVTQEPSLTVSPGGTVTLTCGSSTGAVTSGNYPNW VQQKPGQAPRGLIGGTKFLAPGTPARFSGSLLGGKAALTLSGVQPEDEAEYYCVLWYSNRWVFGGGTKLTVL RLIEDICLPRWGCLWEDD |
| 82 | VRC01HL | artificial | NT | CAGGTGCAGCTGGTGCAGTCTGGCGGCCAGATGAAGAAACCCGGCGAGAGCATGCGGATCAGCTGCCGGGCC TCTGGATACGAGTTCATCGACAGCACTCTGAACTGGATCCGGCTGGCCCCTGGCAAGAGGCCCGAGTGGATG GGCTGGCTGAAGCCCAGAGGCGGAGCCGTGAACTACGCCAGACCTCTGCAGGGCAGAGTGACCATGACCCGG GACGTCTACAGCGATACCGCCTTCCTGGAACTGCGGAGCCTGACCGTGGACGATACCGCCGTGTACTTCTGT ACTCGGGGCAAGAACGCCGACTACAACTGGGACTTCGAGCACTGGGGCAGAGGCACCCCCGTGATCGTGTCT |
| | | | | AGCGGAGGCGGAGGATCTGGCGGCGGAGGCTCTGGGGGAGGCGGAAGCGAGATCGTGCTGACCCAGAGCCCT GGCACCCTGAGCCTGTCTCCCGGCGAAACCGCCATCATCAGCTGCAGAACCAGCCAGTACGGCAGCCTGGCC TGGTATCAGCAGAGGCCAGGCCAGGCCCCCAGACTCGTGATCTACAGTGGAAGCACCAGAGCCGCCGGAATC CCCGACCGGTTCTCTGGTTCCAGATGGGGCCCTGACTACAACCTGACCATCAGCAACCTGGAAAGCGGCGAC TTCGGCGTGTACTACTGCCAGCAGTACGAGTTCTTCGGCCAGGGCACCAAGGTCCAGGTGGACATCAAG |
| 83 | VRC01HL | artificial | AA | QVQLVQSGGQMKKPGESMRISCRASGYEFIDSTLNWIRLAPGKRPEWMGWLKPRGGAVNYARPLQGRVTMTR DVYSDTAFLELRSLTVDDTAVYFCTRGKNADYNWDFEHWGRGTPVIVSSGGGGSGGGGSGGGGSEIVLTQSP GTLSLSPGETAIISCRTSQYGSLAWYQQRPGQAPRLVIYSGSTRAAGIPDRFSGSRWGPDYNLTISNLESGD FGVYYCQQYEFFGQGTKVQVDIK |

(continued)

| SEQ ID NO. | DESIGNATION | SOURCE | TYPE | SEQUENCE |
|---|---|---|---|---|
| 84 | VRC01HLxaCD 3xSA21 x His tag | artificial | NT | CAGGTGCAGCTGGTGCAGTCTGGCGGCCAGATGAAGAAACCCGGCGAGAGCATGCGGATCAGCTGCCGGGCC<br>TCTGGATACGAGTTCATCGACAGCACTCTGAACTGGATCCGGCTGGCCCCTGGCAAGAGGCCCGAGTGGATG<br>GGCTGGCTGAAGCCCAGAGGCGGAGCCGTGAACTACGCCAGACCTCTGCAGGGCAGAGTGACCATGACCCGG<br>GACGTCTACAGCGATACCGCCTTCCTGGAACTGCGGAGCCTGACCGTGGACGATACCGCCGTGTACTTCTGT<br>ACTCGGGGCAAGAACGCCGACTACAACTGGGACTTCGAGCACTGGGGCAGAGGCACCCCCGTGATCGTGTCT<br>AGCGGAGGCGGAGGATCTGGCGGCGGAGGCTCTGGGGGAGGCGGAAGCGAGATCGTGCTGACCCAGAGCCCT<br>GGCACCCTGAGCCTGTCTCCCGGCGAAACCGCCATCATCAGCTGCAGAACCAGCCAGTACGGCAGCCTGGCC<br>TGGTATCAGCAGAGGCCAGGCCAGGCCCCCAGACTCGTGATCTACAGTGGAAGCACCAGAGCCGCCGGAATC<br>CCCGACCGGTTCTCTGGTTCCAGATGGGGCCCTGACTACAACCTGACCATCAGCAACCTGGAAAGCGGCGAC<br>TTCGGCGTGTACTACTGCCAGCAGTACGAGTTCTTCGGCCAGGGCACCAAGGTCCAGGTGGACATCAAGTCC<br>GGAGGTGGTGGATCCGAGGTGCAGCTGGTCGAGTCTGGAGGAGGATTGGTGCAGCCTGGAGGGTCATTGAAA<br>CTCTCATGTGCAGCCTCTGGATTCACCTTCAATAAGTACGCCATGAACTGGGTCCGCCAGGCTCCAGGAAAG<br>GGTTTGGAATGGGTTGCTCGCATAAGAAGTAAATATAATAATTATGCAACATATTATGCCGATTCAGTGAAA<br>GACAGGTTCACCATCTCCAGAGATGATTCAAAAAACACTGCCTATCTACAAATGAACAACTTGAAGACTGAG<br>GACACTGCCGTGTACTACTGTGTGAGACATGGGAACTTCGGTAATAGCTACATATCCTACTGGGCTTACTGG<br>GGCCAAGGGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCACTTGTGGC<br>TCCTCGACTGGGGCTGTTACATCTGGCAACTACCCAAACTGGGTCCAACAAAAACCAGGTCAGGCACCCCGT<br>GGTCTAATAGGTGGGACTAAGTTCCTCGCCCCCGGTACTCCTGCCAGATTCTCAGGCTCCCTGCTTGGAGGC<br>AAGGCTGCCCTCACCCTCTCAGGGGTACAGCCAGAGGATGAGGCAGAATATTACTGTGTTCTATGGTACAGC<br>AACCGCTGGGTGTTCGGTGGAGGAACCAAACTGACTGTCCTACGCCTGATTGAAGATATTTGCCTGCCGCGC<br>TGGGGCTGCCTGTGGGAAGATGATCATCATCACCATCATCAT |
| 85 | VRC01HLxaCD 3xSA21 x His tag | artificial | AA | QVQLVQSGGQMKKPGESMRISCRASGYEFIDSTLNWIRLAPGKRPEWMGWLKPRGGAVNYARPLQGRVTMTR<br>DVYSDTAFLELRSLTVDDTAVYFCTRGKNADYNWDFEHWGRGTPVIVSSGGGGSGGGGSGGGGSEIVLTQSP<br>GTLSLSPGETAIISCRTSQYGSLAWYQQRPGQAPRLVIYSGSTRAAGIPDRFSGSRWGPDYNLTISNLESGD<br>FGVYYCQQYEFFGQGTKVQVDIKSGGGGSEVQLVESGGGLVQPGGSLKLSCAASGFTFNKYAMNWVRQAPGK<br>GLEWVARIRSKYNNYATYYADSVKDRFTISRDDSKNTAYLQMNNLKTEDTAVYYCVRHGNFGNSYISYWAYW<br>GQGTLVTVSSGGGGSGGGGSGGGGSQTVVTQEPSLTVSPGGTVTLTCGSSTGAVTSGNYPNWVQQKPGQAPR<br>GLIGGTKFLAPGTPARFSGSLLGGKAALTLSGVQPEDEAEYYCVLWYSNRWVFGGGTKLTVLRLIEDICLPR<br>WGCLWEDDHHHHHH |

(continued)

| SEQ ID NO. | DESIGNATION | SOURCE | TYPE | SEQUENCE |
|---|---|---|---|---|
| 86 | VRC01HLxaCD 3xSA21 | artificial | AA | QVQLVQSGGQMKKPGESMRISCRASGYEFIDSTLNWIRLAPGKRPEWMGWLKPRGGAVNYARPLQGRVTMTR DVYSDTAFLELRSLTVDDTAVYFCTRGKNADYNWDFEHWGRGTPVIVSSGGGGSGGGGSGGGGSEIVLTQSP GTLSLSPGETAIISCRTSQYGSLAWYQQRPGQAPRLVIYSGSTRAAGIPDRFSGSRWGPDYNLTISNLESGD FGVYYCQQYEFFGQGTKVQVDIKSGGGGSEVQLVESGGGLVQPGGSLKLSCAASGFTFNKYAMNWVRQAPGK GLEWVARIRSKYNNYATYYADSVKDRFTISRDDSKNTAYLQMNNLKTEDTAVYYCVRHGNFGNSYISYWAYW GQGTLVTVSSGGGGSGGGGSGGGGSQTVVTQEPSLTVSPGGTVTLTCGSSTGAVTSGNYPNWVQQKPGQAPR GLIGGTKFLAPGTPARFSGSLLGGKAALTLSGVQPEDEAEYYCVLWYSNRWVFGGGTKLTVLRLIEDICLPR WGCLWEDD |
| 87 | VRC01 LH | artificial | NT | GAGATCGTGCTGACCCAGAGCCCCGGCACCCTGAGCCTGTCTCCAGGCGAGACAGCCATCATCAGCTGCCGG ACCAGCCAGTACGGCAGCCTGGCTTGGTATCAGCAGAGGCCAGGACAGGCCCCCAGACTCGTGATCTACTCT GGAAGCACCAGAGCCGCCGGAATCCCCGACCGGTTCTCTGGATCCCGCTGGGGCCCTGACTACAACCTGACC ATCAGCAACCTGGAAAGCGGCGACTTCGGCGTGTACTACTGCCAGCAGTACGAGTTCTTCGGCCAGGGCACC AAGGTCCAGGTGGACATCAAGGGCGGAGGCGGATCTGGCGGAGGAGGAAGCGGAGGCGGAGGATCTCAGGTG CAGCTGGTGCAGTCTGGCGGCCAGATGAAGAAACCCGGCGAGAGCATGCGGATCAGCTGCAGAGCCTCTGGA TACGAGTTCATCGACAGCACTCTGAACTGGATCCGGCTGGCCCCTGGCAAGAGGCCCGAGTGGATGGGCTGG CTGAAGCCCAGAGGCGGAGCCGTGAACTACGCCAGACCTCTGCAGGGCAGAGTGACCATGACCCGGGACGTC TACAGCGATACCGCCTTCCTGGAACTGCGGAGCCTGACCGTGGACGATACCGCCGTGTACTTCTGTACTCGG GGCAAGAACGCCGACTACAACTGGGACTTCGAGCACTGGGGCAGAGGCACCCCCGTGATCGTGTCCTCC |
| 88 | VRC01 LH | artificial | AA | EIVLTQSPGTLSLSPGETAIISCRTSQYGSLAWYQQRPGQAPRLVIYSGSTRAAGIPDRFSGSRWGPDYNLT ISNLESGDFGVYYCQQYEFFGQGTKVQVDIKGGGGSGGGGSGGGGSQVQLVQSGGQMKKPGESMRISCRASG YEFIDSTLNWIRLAPGKRPEWMGWLKPRGGAVNYARPLQGRVTMTRDVYSDTAFLELRSLTVDDTAVYFCTR GKNADYNWDFEHWGRGTPVIVSS |

(continued)

| SEQ ID NO. | DESIGNATION | SOURCE | TYPE | SEQUENCE |
|---|---|---|---|---|
| 89 | VRC01LHxaCD 3xSA21 x His tag | artificial | NT | GAGATCGTGCTGACCCAGAGCCCCGGCACCCTGAGCCTGTCTCCAGGCGAGACAGCCATCATCAGCTGCCGG<br>ACCAGCCAGTACGGCAGCCTGGCTTGGTATCAGCAGAGGCCAGGACAGGCCCCCAGACTCGTGATCTACTCT<br>GGAAGCACCAGAGCCGCCGGAATCCCCGACCGGTTCTCTGGATCCCGCTGGGGCCCTGACTACAACCTGACC<br>ATCAGCAACCTGGAAAGCGGCGACTTCGGCGTGTACTACTGCCAGCAGTACGAGTTCTTCGGCCAGGGCACC<br>AAGGTCCAGGTGGACATCAAGGGCGGAGGCGGATCTGGCGGAGGAGGAAGCGGAGGCGGAGGATCTCAGGTG<br>CAGCTGGTGCAGTCTGGCGGCCAGATGAAGAAACCCGGCGAGAGCATGCGGATCAGCTGCAGAGCCTCTGGA<br>TACGAGTTCATCGACAGCACTCTGAACTGGATCCGGCTGGCCCCTGGCAAGAGGCCCGAGTGGATGGGCTGG<br>CTGAAGCCCAGAGGCGGAGCCGTGAACTACGCCAGACCTCTGCAGGGCAGAGTGACCATGACCCGGGACGTC<br>TACAGCGATACCGCCTTCCTGGAACTGCGGAGCCTGACCGTGGACGATACCGCCGTGTACTTCTGTACTCGG<br>GGCAAGAACGCCGACTACAACTGGGACTTCGAGCACTGGGGCAGAGGCACCCCCGTGATCGTGTCCTCCGGA<br>GGTGGTGGATCCGAGGTGCAGCTGGTCGAGTCTGGAGGAGGATTGGTGCAGCCTGGAGGGTCATTGAAACTC<br>TCATGTGCAGCCTCTGGATTCACCTTCAATAAGTACGCCATGAACTGGGTCCGCCAGGCTCCAGGAAAGGGT<br>TTGGAATGGGTTGCTCGCATAAGAAGTAAATATAATAATTATGCAACATATTATGCCGATTCAGTGAAAGAC<br>AGGTTCACCATCTCCAGAGATGATTCAAAAAACACTGCCTATCTACAAATGAACAACTTGAAGACTGAGGAC<br>ACTGCCGTGTACTACTGTGTGAGACATGGGAACTTCGGTAATAGCTACATATCCTACTGGGCTTACTGGGGC<br>CAAGGGACTCTGGTCACCGTCTCCTCAGGTGGTGGTGGTTCTGGCGGCGGCGGCTCCGGTGGTGGTGGTTCT<br>CAGACTGTTGTGACTCAGGAACCTTCACTCACCGTATCACCTGGTGGAACAGTCACACTCACTTGTGGCTCC |
| | | | | TCGACTGGGGCTGTTACATCTGGCAACTACCCAAACTGGGTCCAACAAAAACCAGGTCAGGCACCCCGTGGT<br>CTAATAGGTGGGACTAAGTTCCTCGCCCCCGGTACTCCTGCCAGATTCTCAGGCTCCCTGCTTGGAGGCAAG<br>GCTGCCCTCACCCTCTCAGGGGTACAGCCAGAGGATGAGGCAGAATATTACTGTGTTCTATGGTACAGCAAC<br>CGCTGGGTGTTCGGTGGAGGAACCAAACTGACTGTCCTACGCCTGATTGAAGATATTTGCCTGCCGCGCTGG<br>GGCTGCCTGTGGGAAGATGATCATCATCACCATCATCAT |
| 90 | VRC01LHxaCD 3xSA21 x His tag | artificial | AA | EIVLTQSPGTLSLSPGETAIISCRTSQYGSLAWYQQRPGQAPRLVIYSGSTRAAGIPDRFSGSRWGPDYNLT<br>ISNLESGDFGVYYCQQYEFFGQGTKVQVDIKGGGGSGGGGSGGGGSQVQLVQSGGQMKKPGESMRISCRASG<br>YEFIDSTLNWIRLAPGKRPEWMGWLKPRGGAVNYARPLQGRVTMTRDVYSDTAFLELRSLTVDDTAVYFCTR<br>GKNADYNWDFEHWGRGTPVIVSSGGGGSEVQLVESGGGLVQPGGSLKLSCAASGFTFNKYAMNWVRQAPGKG<br>LEWVARIRSKYNNYATYYADSVKDRFTISRDDSKNTAYLQMNNLKTEDTAVYYCVRHGNFGNSYISYWAYWG<br>QGTLVTVSSGGGGSGGGGSGGGGSQTVVTQEPSLTVSPGGTVTLTCGSSTGAVTSGNYPNWVQQKPGQAPRG<br>LIGGTKFLAPGTPARFSGSLLGGKAALTLSGVQPEDEAEYYCVLWYSNRWVFGGGTKLTVLRLIEDICLPRW<br>GCLWEDDHHHHHH |

(continued)

| SEQ ID NO. | DESIGNATION | SOURCE | TYPE | SEQUENCE |
|---|---|---|---|---|
| 91 | VRC01LHxaCD 3xSA21 | artificial | AA | EIVLTQSPGTLSLSPGETAIISCRTSQYGSLAWYQQRPGQAPRLVIYSGSTRAAGIPDRFSGSRWGPDYNLT<br>ISNLESGDFGVYYCQQYEFFGQGTKVQVDIKGGGGSGGGGSGGGGSQVQLVQSGGQMKKPGESMRISCRASG<br>YEFIDSTLNWIRLAPGKRPEWMGWLKPRGGAVNYARPLQGRVTMTRDVYSDTAFLELRSLTVDDTAVYFCTR<br>GKNADYNWDFEHWGRGTPVIVSSGGGGSEVQLVESGGGLVQPGGSLKLSCAASGFTFNKYAMNWVRQAPGKG<br>LEWVARIRSKYNNYATYYADSVKDRFTISRDDSKNTAYLQMNNLKTEDTAVYYCVRHGNFGNSYISYWAYWG<br>QGTLVTVSSGGGGSGGGGSGGGGSQTVVTQEPSLTVSPGGTVTLTCGSSTGAVTSGNYPNWVQQKPGQAPRG<br>LIGGTKFLAPGTPARFSGSLLGGKAALTLSGVQPEDEAEYYCVLWYSNRWVFGGGTKLTVLRLIEDICLPRW<br>GCLWEDD |
| 92 | 4E10HL | artificial | NT | CAGGTGCAGCTGGTGCAGTCTGGCGCCGAAGTGAAAAGACCCGGCAGCAGCGTGACCGTGTCCTGCAAAGCT<br>AGCGGCGGCAGCTTCAGCACCTACGCCCTGTCTTGGGTGCGCCAGGCTCCTGGCAGAGGCCTGGAATGGATG<br>GGCGGAGTGATCCCCCTGCTGACCATCACCAACTACGCCCCCAGATTCCAGGGCCGGATCACCATCACCGCC<br>GACAGAAGCACCAGCACCGCCTACCTGGAACTGAACAGCCTGAGGCCCGAGGACACCGCCGTGTACTACTGT<br>GCCAGAGAGGGCACAACAGGCTGGGGCTGGCTGGGCAAACCTATCGGAGCCTTTGCCCACTGGGGCCAGGGC<br>ACACTCGTGACAGTGTCTAGTGGCGGCGGAGGATCTGGCGGAGGCGGAAGTGGGGGAGGCGGCTCTGAAATC<br>GTGCTGACACAGAGCCCAGGCACCCAGTCTCTGAGCCCTGGCGAAAGAGCCACCCTGAGCTGTAGAGCCAGC<br>CAGAGCGTGGGCAACAACAAGCTGGCCTGGTATCAGCAGCGGCCAGGCCAGGCACCTCGGCTGCTGATCTAT<br>GGCGCCAGCAGCAGACCTAGCGGCGTGGCCGATAGATTTTCCGGCTCTGGCAGCGGCACCGACTTCACCCTG<br>ACCATCTCCAGACTGGAACCCGAGGACTTTGCCGTGTATTATTGCCAGCAGTACGGCCAGAGCCTGAGCACC<br>TTTGGACAGGGCACCAAGGTGGAAGTGAAG |
| 93 | 4E10HL | artificial | AA | QVQLVQSGAEVKRPGSSVTVSCKASGGSFSTYALSWVRQAPGRGLEWMGGVIPLLTITNYAPRFQGRITITA<br>DRSTSTAYLELNSLRPEDTAVYYCAREGTTGWGWLGKPIGAFAHWGQGTLVTVSSGGGGSGGGGSGGGGSEI<br>VLTQSPGTQSLSPGERATLSCRASQSVGNNKLAWYQQRPGQAPRLLIYGASSRPSGVADRFSGSGSGTDFTL<br>TISRLEPEDFAVYYCQQYGQSLSTFGQGTKVEVK |
| 94 | 4E10HLxaCD3x SA21 x His tag | artificial | NT | CAGGTGCAGCTGGTGCAGTCTGGCGCCGAAGTGAAAAGACCCGGCAGCAGCGTGACCGTGTCCTGCAAAGCT<br>AGCGGCGGCAGCTTCAGCACCTACGCCCTGTCTTGGGTGCGCCAGGCTCCTGGCAGAGGCCTGGAATGGATG<br>GGCGGAGTGATCCCCCTGCTGACCATCACCAACTACGCCCCCAGATTCCAGGGCCGGATCACCATCACCGCC |

(continued)

| SEQ ID NO. | DESIGNATION | SOURCE | TYPE | SEQUENCE |
|---|---|---|---|---|
| | | | | GACAGAAGCACCAGCACCGCCTACCTGGAACTGAACAGCCTGAGGCCCGAGGACACCGCCGTGTACTACTGT<br>GCCAGAGAGGGCACAACAGGCTGGGGCTGGCTGGGCAAACCTATCGGAGCCTTTGCCCACTGGGGCCAGGGC<br>ACACTCGTGACAGTGTCTAGTGGCGGCGGAGGATCTGGCGGAGGCGGAAGTGGGGGAGGCGGCTCTGAAATC<br>GTGCTGACACAGAGCCCAGGCACCCAGTCTCTGAGCCCTGGCGAAAGAGCCACCCTGAGCTGTAGAGCCAGC<br>CAGAGCGTGGGCAACAACAAGCTGGCCTGGTATCAGCAGCGGCCAGGCCAGGCACCTCGGCTGCTGATCTAT<br>GGCGCCAGCAGCAGACCTAGCGGCGTGGCCGATAGATTTTCCGGCTCTGGCAGCGGCACCGACTTCACCCTG<br>ACCATCTCCAGACTGGAACCCGAGGACTTTGCCGTGTATTATTGCCAGCAGTACGGCCAGAGCCTGAGCACC<br>TTTGGACAGGGCACCAAGGTGGAAGTGAAGTCCGGAGGTGGTGGATCCGAGGTGCAGCTGGTCGAGTCTGGA<br>GGAGGATTGGTGCAGCCTGGAGGGTCATTGAAACTCTCATGTGCAGCCTCTGGATTCACCTTCAATAAGTAC<br>GCCATGAACTGGGTCCGCCAGGCTCCAGGAAAGGGTTTGGAATGGGTTGCTCGCATAAGAAGTAAATATAAT<br>AATTATGCAACATATTATGCCGATTCAGTGAAAGACAGGTTCACCATCTCCAGAGATGATTCAAAAAACACT<br>GCCTATCTACAAATGAACAACTTGAAGACTGAGGACACTGCCGTGTACTACTGTGTGAGACATGGGAACTTC<br>GGTAATAGCTACATATCCTACTGGGCTTACTGGGGCCAAGGGACTCTGGTCACCGTCTCCTCAGGTGGTGGT<br>GGTTCTGGCGGCGGCGGCTCCGGTGGTGGTGGTTCTCAGACTGTTGTGACTCAGGAACCTTCACTCACCGTA<br>TCACCTGGTGGAACAGTCACACTCACTTGTGGCTCCTCGACTGGGGCTGTTACATCTGGCAACTACCCAAAC<br>TGGGTCCAACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCCAGAGGAT<br>GAGGCAGAATATTACTGTGTTCTATGGTACAGCAACCGCTGGGTGTTCGGTGGAGGAACCAAACTGACTGTC<br>CTACGCCTGATTGAAGATATTTGCCTGCCGCGCTGGGGCTGCCTGTGGGAAGATGATCATCATCACCATCAT<br>CAT |
| 95 | 4E10HLxaCD3x SA21 x His tag | artificial | AA | QVQLVQSGAEVKRPGSSVTVSCKASGGSFSTYALSWVRQAPGRGLEWMGGVIPLLTITNYAPRFQGRITITA<br>DRSTSTAYLELNSLRPEDTAVYYCAREGTTGWGWLGKPIGAFAHWGQGTLVTVSSGGGGSGGGGSGGGGSEI<br>VLTQSPGTQSLSPGERATLSCRASQSVGNNKLAWYQQRPGQAPRLLIYGASSRPSGVADRFSGSGSGTDFTL<br>TISRLEPEDFAVYYCQQYGQSLSTFGQGTKVEVKSGGGGSEVQLVESGGGLVQPGGSLKLSCAASGFTFNKY<br>AMNWVRQAPGKGLEWVARIRSKYNNYATYYADSVKDRFTISRDDSKNTAYLQMNNLKTEDTAVYYCVRHGNF<br>GNSYISYWAYWGQGTLVTVSSGGGGSGGGGSGGGGSQTVVTQEPSLTVSPGGTVTLTCGSSTGAVTSGNYPN<br>WVQQKPGQAPRGLIGGTKFLAPGTPARFSGSLLGGKAALTLSGVQPEDEAEYYCVLWYSNRWVFGGGTKLTV<br>LRLIEDICLPRWGCLWEDDHHHHHH |

(continued)

| SEQ ID NO. | DESIGNATION | SOURCE | TYPE | SEQUENCE |
|---|---|---|---|---|
| 96 | 4E10HLxaCD3x SA21 | artificial | AA | QVQLVQSGAEVKRPGSSVTVSCKASGGSFSTYALSWVRQAPGRGLEWMGGVIPLLTITNYAPRFQGRITITA DRSTSTAYLELNSLRPEDTAVYYCAREGTTGWGWLGKPIGAFAHWGQGTLVTVSSGGGGSGGGGSGGGGSEI VLTQSPGTQSLSPGERATLSCRASQSVGNNKLAWYQQRPGQAPRLLIYGASSRPSGVADRFSGSGSGTDFTL TISRLEPEDFAVYYCQQYGQSLSTFGQGTKVEVKSGGGGSEVQLVESGGGLVQPGGSLKLSCAASGFTFNKY AMNWVRQAPGKGLEWVARIRSKYNNYATYYADSVKDRFTISRDDSKNTAYLQMNNLKTEDTAVYYCVRHGNF GNSYISYWAYWGQGTLVTVSSGGGGSGGGGSGGGGSQTVVTQEPSLTVSPGGTVTLTCGSSTGAVTSGNYPN WVQQKPGQAPRGLIGGTKFLAPGTPARFSGSLLGGKAALTLSGVQPEDEAEYYCVLWYSNRWVFGGGTKLTV LRLIEDICLPRWGCLWEDD |
| 97 | 4E10LH | artificial | NT | GAAATCGTGCTGACACAGAGCCCAGGCACCCAGTCTCTGAGCCCTGGCGAAAGAGCCACCCTGAGCTGTAGA GCCAGCCAGAGCGTGGGCAACAACAAGCTGGCCTGGTATCAGCAGCGGCCAGGCCAGGCACCTCGGCTGCTG ATCTATGGCGCCAGCAGCAGACCTAGCGGCGTGGCCGATAGATTTTCCGGCTCTGGCAGCGGCACCGACTTC |
| | | | | ACCCTGACCATCTCCAGACTGGAACCCGAGGACTTTGCCGTGTATTATTGCCAGCAGTACGGCCAGAGCCTG AGCACCTTTGGACAGGGCACCAAGGTGGAAGTGAAGGGCGGCGGAGGATCTGGCGGAGGCGGAAGTGGGGGA GGCGGCTCTCAGGTGCAGCTGGTGCAGTCTGGCGCCGAAGTGAAAAGACCCGGCAGCAGCGTGACCGTGTCC TGCAAAGCTAGCGGCGGCAGCTTCAGCACCTACGCCCTGTCTTGGGTGCGCCAGGCTCCTGGCAGAGGCCTG GAATGGATGGGCGGAGTGATCCCCCTGCTGACCATCACCAACTACGCCCCCAGATTCCAGGGCCGGATCACC ATCACCGCCGACAGAAGCACCAGCACCGCCTACCTGGAACTGAACAGCCTGAGGCCCGAGGACACCGCCGTG TACTACTGTGCCAGAGAGGGCACAACAGGCTGGGGCTGGCTGGGCAAACCTATCGGAGCCTTTGCCCACTGG GGCCAGGGCACACTCGTGACAGTGTCTTCC |
| 98 | 4E10LH | artificial | AA | EIVLTQSPGTQSLSPGERATLSCRASQSVGNNKLAWYQQRPGQAPRLLIYGASSRPSGVADRFSGSGSGTDF TLTISRLEPEDFAVYYCQQYGQSLSTFGQGTKVEVKGGGGSGGGGSGGGGSQVQLVQSGAEVKRPGSSVTVS CKASGGSFSTYALSWVRQAPGRGLEWMGGVIPLLTITNYAPRFQGRITITADRSTSTAYLELNSLRPEDTAV YYCAREGTTGWGWLGKPIGAFAHWGQGTLVTVSS |

(continued)

| SEQ ID NO. | DESIGNATION | SOURCE | TYPE | SEQUENCE |
|---|---|---|---|---|
| 99 | 4E10LHxaCD3S A21 x His tag | artificial | NT | GAAATCGTGCTGACACAGAGCCCAGGCACCCAGTCTCTGAGCCCTGGCGAAAGAGCCACCCTGAGCTGTAGA<br>GCCAGCCAGAGCGTGGGCAACAACAAGCTGGCCTGGTATCAGCAGCGGCCAGGCCAGGCACCTCGGCTGCTG<br>ATCTATGGCGCCAGCAGCAGACCTAGCGGCGTGGCCGATAGATTTTCCGGCTCTGGCAGCGGCACCGACTTC<br>ACCCTGACCATCTCCAGACTGGAACCCGAGGACTTTGCCGTGTATTATTGCCAGCAGTACGGCCAGAGCCTG<br>AGCACCTTTGGACAGGGCACCAAGGTGGAAGTGAAGGGCGGCGGAGGATCTGGCGGAGGCGGAAGTGGGGGA<br>GGCGGCTCTCAGGTGCAGCTGGTGCAGTCTGGCGCCGAAGTGAAAAGACCCGGCAGCAGCGTGACCGTGTCC<br>TGCAAAGCTAGCGGCGGCAGCTTCAGCACCTACGCCCTGTCTTGGGTGCGCCAGGCTCCTGGCAGAGGCCTG<br>GAATGGATGGGCGGAGTGATCCCCCTGCTGACCATCACCAACTACGCCCCCAGATTCCAGGGCCGGATCACC<br>ATCACCGCCGACAGAAGCACCAGCACCGCCTACCTGGAACTGAACAGCCTGAGGCCCGAGGACACCGCCGTG<br>TACTACTGTGCCAGAGAGGGCACAACAGGCTGGGGCTGGCTGGGCAAACCTATCGGAGCCTTTGCCCACTGG<br>GGCCAGGGCACACTCGTGACAGTGTCTTCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAAGTACGCC<br>ATGAACTGGGTCCGCCAGGCTCCAGGAAAGGGTTTGGAATGGGTTGCTCGCATAAGAAGTAAATATAATAAT<br>TATGCAACATATTATGCCGATTCAGTGAAAGACAGGTTCACCATCTCCAGAGATGATTCAAAAAACACTGCC<br>TATCTACAAATGAACAACTTGAAGACTGAGGACACTGCCGTGTACTACTGTGTGAGACATGGGAACTTCGGT<br>AATAGCTACATATCCTACTGGGCTTACTGGGGCCAAGGGACTCTGGTCACCGTCTCCTCAGGTGGTGGTGGT<br>TCTGGCGGCGGCGGCTCCGGTGGTGGTGGTTCTCAGACTGTTGTGACTCAGGAACCTTCACTCACCGTATCA<br>CCTGGTGGAACAGTCACACTCACTTGTGGCTCCTCGACTGGGGCTGTTACATCTGGCAACTACCCAAACTGG<br>GTCCAACAAAAACCAGGTCAGGCACCCCGTGGTCTAATAGGTGGGACTAAGTTCCTCGCCCCCGGTACTCCT<br>GCCAGATTCTCAGGCTCCCTGCTTGGAGGCAAGGCTGCCCTCACCCTCTCAGGGGTACAGCCAGAGGATGAG<br>GCAGAATATTACTGTGTTCTATGGTACAGCAACCGCTGGGTGTTCGGTGGAGGAACCAAACTGACTGTCCTA<br>CGCCTGATTGAAGATATTTGCCTGCCGCGCTGGGGCTGCCTGTGGGAAGATGATCATCATCACCATCATCAT |
| 100 | 4E10LHxaCD3S A21 x His tag | artificial | AA | EIVLTQSPGTQSLSPGERATLSCRASQSVGNNKLAWYQQRPGQAPRLLIYGASSRPSGVADRFSGSGSGTDF<br>TLTISRLEPEDFAVYYCQQYGQSLSTFGQGTKVEVKGGGGSGGGGSGGGGSQVQLVQSGAEVKRPGSSVTVS<br>CKASGGSFSTYALSWVRQAPGRGLEWMGGVIPLLTITNYAPRFQGRITITADRSTSTAYLELNSLRPEDTAV<br>YYCAREGTTGWGWLGKPIGAFAHWGQGTLVTVSSGGGGSEVQLVESGGGLVQPGGSLKLSCAASGFTFNKYA<br>MNWVRQAPGKGLEWVARIRSKYNNYATYYADSVKDRFTISRDDSKNTAYLQMNNLKTEDTAVYYCVRHGNFG |
| | | | | NSYISYWAYWGQGTLVTVSSGGGGSGGGGSGGGGSQTVVTQEPSLTVSPGGTVTLTCGSSTGAVTSGNYPNW<br>VQQKPGQAPRGLIGGTKFLAPGTPARFSGSLLGGKAALTLSGVQPEDEAEYYCVLWYSNRWVFGGGTKLTVL<br>RLIEDICLPRWGCLWEDDHHHHHH |

(continued)

| SEQ ID NO. | DESIGNATION | SOURCE | TYPE | SEQUENCE |
|---|---|---|---|---|
| 101 | 4E10LHxaCD3S A21 | artificial | AA | EIVLTQSPGTQSLSPGERATLSCRASQSVGNNKLAWYQQRPGQAPRLLIYGASSRPSGVADRFSGSGSGTDF TLTISRLEPEDFAVYYCQQYGQSLSTFGQGTKVEVKGGGGSGGGGSGGGGSQVQLVQSGAEVKRPGSSVTVS CKASGGSFSTYALSWVRQAPGRGLEWMGGVIPLLTITNYAPRFQGRITITADRSTSTAYLELNSLRPEDTAV YYCAREGTTGWGWLGKPIGAFAHWGQGTLVTVSSGGGGSEVQLVESGGGLVQPGGSLKLSCAASGFTFNKYA MNWVRQAPGKGLEWVARIRSKYNNYATYYADSVKDRFTISRDDSKNTAYLQMNNLKTEDTAVYYCVRHGNFG NSYISYWAYWGQGTLVTVSSGGGGSGGGGSGGGGSQTVVTQEPSLTVSPGGTVTLTCGSSTGAVTSGNYPNW VQQKPGQAPRGLIGGTKFLAPGTPARFSGSLLGGKAALTLSGVQPEDEAEYYCVLWYSNRWVFGGGTKLTVL RLIEDICLPRWGCLWEDD |

## Claims

1. A bispecific antibody comprising at least three binding domains comprised in one polypeptide chain, wherein

   (a) the first domain is a binding domain which is binding to a cell surface molecule on a target cell; and
   (b) the second domain is a binding domain which is binding to the T cell CD3 receptor complex; and
   (c) the third domain is a binding domain which is binding to serum albumin, wherein said third domain is positioned at the C-terminus of said second domain,

   wherein the three domains are on one polypeptide in the order from the N-terminus to the C-terminus

   • the first binding domain;
   • the second binding domain; and
   • the third binding domain.

2. The bispecific antibody according to claim 1, wherein the third domain of the bispecific antibody is an scFv or single domain antibody.

3. The bispecific antibody according to claim 1 or 2, wherein

   (a) the first binding domain is capable of binding to the cell surface molecule on a human and a non-human primate cell;
   (b) the second binding domain is capable of binding to the T cell CD3 receptor complex on a human and a non-human primate cell, and
   (c) the third binding domain is capable of binding to human and non-human primate serum albumin.

4. The bispecific antibody according to any of claims 1 to 3, wherein the third binding domain capable of binding to serum albumin is derived from a combinatorial library or an antibody binding domain.

5. The bispecific antibody according to any of claims 1 to 4, wherein the third binding domain comprises between 10 and 25 aa residues.

6. The bispecific antibody according to claims 1 to 5, wherein the third binding domain capable of binding to serum albumin comprises the amino acid sequence Asp-Xaa-Cys-Leu-Pro-Xaa-Trp-Gly-Cys-Leu-Trp, wherein Xaa is any amino acid.

7. The bispecific antibody according to claims 1 to 5, wherein the third binding domain capable of binding to serum albumin is derived from a CDR of a single domain antibody.

8. The bispecific antibody according to any of claims 1 to 7, wherein the third binding domain is binding to serum albumin with an affinity (KD) of ≤ 500 nM.

9. The bispecific antibody according to any of claims 1 to 8, wherein the molecule consists of a single polypeptide chain.

10. The bispecific antibody according to any of claims 1 to 9, wherein the first binding domain capable of binding to a cell surface molecule is binding to a tumor antigen.

11. The bispecific antibody according to any of claims 1 to 10, wherein the second binding domain capable of binding to the T cell CD3 receptor complex is capable of binding to an epitope of human and Callithrix jacchus, Saguinus oedipus or Saimiri sciureus CD3ε chain, wherein the epitope is part of an amino acid sequence comprised in the group consisting of SEQ ID NOs: 2, 4, 6, or 8 and comprises at least the amino acid sequence Gln-Asp-Gly-Asn-Glu.

12. The bispecific antibody according to any of claims 1 to 6, **characterized by** an amino acid sequence as depicted in SEQ ID NOs: 51, 52, 54, 55, 57, 58, 60, 61, 75, 76, 81, 82, 85, 86, 90, 91, 85, 96, 100 or 101.

13. A nucleic acid sequence encoding a bispecific antibody as defined in any one of claims 1 to 12.

14. A vector comprising a nucleic acid sequence as defined in claim 13.

**15.** A host cell transformed or transfected with, and comprising the nucleic acid sequence as defined in claim 13 or with the vector as defined in claim 14.

**16.** A process for the production of a bispecific antibody according to any one of claims 1 to 12, said process comprising culturing a host cell as defined in claim 15 under conditions allowing the expression of the antibody as defined in any one of claims 1 to 12 and recovering the produced antibody from the culture.

**17.** A pharmaceutical composition comprising a bispecific antibody according to any one of claims 1 to 12, or produced according to the process of claim 16.

**18.** The bispecific antibody according to any one of claims 1 to 12, or produced according to the process of claim 16 for use in the prevention, treatment or amelioration of a disease selected from the group consisting of a proliferative disease, an inflammatory disease, an infectious disease and an autoimmune disease.

**19.** A kit comprising a bispecific antibody as defined in any one of claims 1 to 12, a nucleic acid molecule as defined in claim 13, a vector as defined in claim 14, or a host cell as defined in claim 15.

**Patentansprüche**

**1.** Bispezifischer Antikörper, umfassend mindestens drei Bindungsdomänen, die in einer Polypeptidkette umfasst sind, wobei

(a) die erste Domäne eine Bindungsdomäne ist, die an ein Zelloberflächenmolekül auf einer Zielzelle bindet; und
(b) die zweite Domäne eine Bindungsdomäne ist, die an den T-Zell CD3Rezeptorkomplex bindet; und
(c) die dritte Domäne eine Bindungsdomäne ist, die an Serumalbumin bindet, wobei diese dritte Domäne an dem C-Terminus dieser zweiten Domäne positioniert ist,

wobei die drei Domänen auf einem Polypeptid in der Reihenfolge von dem N-Terminus zu dem C-Terminus sind

• die erste Bindungsdomäne;
• die zweite Bindungsdomäne; und
• die dritte Bindungsdomäne.

**2.** Bispezifischer Antikörper nach Anspruch 1, wobei die dritte Domäne des bispezifischen Antikörpers ein scFv oder Einzeldomänenantikörper ist.

**3.** Bispezifischer Antikörper nach Anspruch 1 oder 2, wobei

(a) die erste Bindungsdomäne in der Lage ist an das Zelloberflächenmolekül auf einer menschlichen und einer nicht-menschlichen Primatenzelle zu binden;
(b) die zweite Bindungsdomäne in der Lage ist an den T-Zellen CD3Rezeptorkomplex auf einer menschlichen und einer nicht-menschlichen Primatenzelle zu binden, und
(c) die dritte Bindungsdomäne in der Lage ist, an menschliches und nicht-menschliches Primatenserumalbumin zu binden.

**4.** Bispezifischer Antikörper nach einem der Ansprüche 1 bis 3, wobei die dritte Bindungsdomäne, die in der Lage ist an Serumalbumin zu binden, aus einer kombinatorischen Bibliothek oder einer Antikörperbindungsdomäne abgeleitet ist.

**5.** Bispezifischer Antikörper nach einem der Ansprüche 1 bis 4, wobei die dritte Bindungsdomäne zwischen 10 und 25 aa Reste umfasst.

**6.** Bispezifischer Antikörper nach Anspruch 1 bis 5, wobei die dritte Bindungsdomäne, die in der Lage ist an Serumalbumin zu binden, die Aminosäuresequenz Asp-Xaa-Cys-Leu-Pro-Xaa-Trp-Gly-Cys-Leu-Trp umfasst, wobei Xaa eine beliebige Aminosäure ist.

**7.** Bispezifischer Antikörper nach Anspruch 1 bis 5, wobei die dritte Bindungsdomäne, die in der Lage ist an Serumalbumin zu binden, von einer CDR eines Einzeldomänenantikörpers abgeleitet ist.

**8.** Bispezifischer Antikörper nach einem der Ansprüche 1 bis 7, wobei die dritte Bindungsdomäne mit einer Affinität (KD) von ≤ 500 nM an Serumalbumin bindet.

**9.** Bispezifischer Antikörper nach einem der Ansprüche 1 bis 8, wobei das Molekül aus einer einzelnen Polypeptidkette besteht.

**10.** Bispezifischer Antikörper nach einem der Ansprüche 1 bis 9, wobei die erste Bindungsdomäne, die in der Lage ist an ein Zelloberflächenmolekül zu binden, an ein Tumorantigen bindet.

**11.** Bispezifischer Antikörper nach einem der Ansprüche 1 bis 10, wobei die zweite Bindungsdomäne, die in der Lage ist an den T-Zell CD3 Rezeptorkomplex zu binden, in der Lage ist an ein Epitop einer menschlichen und Callithrix jacchus, Saguinus oedipus oder Saimiri sciureus CD3ε Kette zu binden, wobei das Epitop Teil einer Aminosäuresequenz ist, die in der Gruppe bestehend aus SEQ ID NOs: 2, 4, 6 oder 8 enthalten ist und mindestens die Aminosäuresequenz Gln-Asp-Gly-Asn-Glu umfasst.

**12.** Bispezifischer Antikörper nach einem der Ansprüche 1 bis 6, **gekennzeichnet durch** eine Aminosäuresequenz wie in SEQ ID NOs: 51, 52, 54, 55, 57, 58, 60, 61, 75, 76, 81, 82, 85, 86, 90, 91, 85, 96, 100 oder 101 dargestellt.

**13.** Nukleinsäuresequenz, die einen bispezifischen Antikörper wie in einem der Ansprüche 1 bis 12 definiert kodiert.

**14.** Vektor, umfassend eine Nukleinsäuresequenz wie in Anspruch 13 definiert.

**15.** Wirtszelle, transformiert oder transfiziert mit der Nukleinsäuresequenz wie in Anspruch 13 definiert oder mit dem Vektor wie in Anspruch 14 definiert.

**16.** Verfahren zur Herstellung eines bispezifischen Antikörpers nach einem der Ansprüche 1 bis 12, wobei dieses Verfahren das Kultivieren einer Wirtszelle wie in Anspruch 15 definiert unter Bedingungen, die die Expression der Antikörperderivate oder Fragmente wie in einem der Ansprüche 1 bis 12 definiert, ermöglichen, und die Rückgewinnung der hergestellten Antikörperderivate oder Fragmente aus der Kultur umfasst.

**17.** Pharmazeutisches Mittel, umfassend einen bispezifischen Antikörper nach einem der Ansprüche 1 bis 12 oder hergestellt gemäß dem Verfahren nach Anspruch 16.

**18.** Bispezifischer Antikörper nach einem der Ansprüche 1 bis 12 oder hergestellt gemäß dem Verfahren nach Anspruch 16 zur Verwendung bei der Prävention, Behandlung oder Verbesserung einer Erkrankung, ausgewählt aus der Gruppe bestehend aus einer proliferativen Erkrankung, einer entzündlichen Erkrankung, einer infektiösen Erkrankung und einer Autoimmunerkrankung.

**19.** Kit, umfassend einen bispezifischen Antikörper nach einem der Ansprüche 1 bis 12, ein Nukleinsäuremolekül wie in Anspruch 13 definiert, einen Vektor wie in Anspruch 14 definiert oder eine Wirtszelle wie in Anspruch 15 definiert.

## Revendications

**1.** Anticorps bispécifique comprenant au moins trois domaines de liaison compris dans une chaîne polypeptidique, dans lequel

(a) le premier domaine est un domaine de liaison qui se lie à une molécule de surface cellulaire sur une cellule cible ; et
(b) le deuxième domaine est un domaine de liaison qui se lie au complexe du récepteur CD3 des lymphocytes T ; et
(c) le troisième domaine est un domaine de liaison qui se lie à l'albumine sérique, ledit troisième domaine est positionné au niveau de l'extrémité C-terminale dudit deuxième domaine,

dans lequel les trois domaines sont sur un polypeptide dans l'ordre de l'extrémité N-terminale à l'extrémité C-terminale

- le premier domaine de liaison ;
- le deuxième domaine de liaison ; et
- le troisième domaine de liaison.

**2.** Anticorps bispécifique selon la revendication 1, dans lequel le troisième domaine de l'anticorps bispécifique est un scFv ou un anticorps à domaine unique.

**3.** Anticorps bispécifique selon la revendication 1 ou 2, dans lequel

(a) le premier domaine de liaison est capable de se lier à la molécule de surface cellulaire sur une cellule de primate humain et non humain ;
(b) le deuxième domaine de liaison est capable de se lier au complexe du récepteur CD3 des lymphocytes T sur une cellule de primate humain et non humain, et
(c) le troisième domaine de liaison est capable de se lier à l'albumine sérique de primate humain et non humain.

**4.** Anticorps bispécifique selon l'une quelconque des revendications 1 à 3, dans lequel le troisième domaine de liaison capable de se lier à l'albumine sérique est dérivé d'une banque combinatoire ou d'un domaine de liaison d'anticorps.

**5.** Anticorps bispécifique selon l'une quelconque des revendications 1 à 4, dans lequel le troisième domaine de liaison comprend entre 10 et 25 résidus aa.

**6.** Anticorps bispécifique selon les revendications 1 à 5, dans lequel le troisième domaine de liaison capable de se lier à l'albumine sérique comprend la séquence d'acides aminés Asp-Xaa-Cys-Leu-Pro-Xaa-Trp-Gly-Cys-Leu-Trp, dans lequel Xaa est n'importe quel acide aminé.

**7.** Anticorps bispécifique selon les revendications 1 à 5, dans lequel le troisième domaine de liaison capable de se lier à l'albumine sérique est dérivé d'une CDR d'un anticorps à domaine unique.

**8.** Anticorps bispécifique selon l'une quelconque des revendications 1 à 7, dans lequel le troisième domaine de liaison se lie à l'albumine sérique avec une affinité (KD) inférieure à 500 nM.

**9.** Anticorps bispécifique selon l'une quelconque des revendications 1 à 8, dans lequel la molécule est constituée d'une seule chaîne polypeptidique.

**10.** Anticorps bispécifique selon l'une quelconque des revendications 1 à 9, dans lequel le premier domaine de liaison capable de se lier à une molécule de surface cellulaire se lie à un antigène tumoral.

**11.** Anticorps bispécifique selon l'une quelconque des revendications 1 à 10, dans lequel le deuxième domaine de liaison capable de se lier au complexe du récepteur CD3 des lymphocytes T est capable de se lier à un épitope de la chaîne CD3ε humaine et de Callithrix jacchus, Saguinus oedipus ou Saimiri sciureus, dans lequel l'épitope fait partie d'une séquence d'acides aminés comprise dans le groupe constitué des SEQ ID NO : 2, 4, 6 ou 8 et comprend au moins la séquence d'acides aminés Gln-Asp-Gly-Asn-Glu.

**12.** Anticorps bispécifique selon l'une quelconque des revendications 1 à 6, **caractérisé par** une séquence d'acides aminés telle que représentée dans les SEQ ID NO : 51, 52, 54, 55, 57, 58, 60, 61, 75, 76, 81, 82, 85, 86, 90, 91, 85, 96, 100 ou 101.

**13.** Séquence d'acide nucléique codant pour un anticorps bispécifique tel que défini dans l'une quelconque des revendications 1 à 12.

**14.** Vecteur comprenant une séquence d'acide nucléique telle que définie dans la revendication 13.

**15.** Cellule hôte transformée ou transfectée avec, et comprenant la séquence d'acide nucléique telle que définie dans la revendication 13 ou avec le vecteur tel que défini dans la revendication 14.

**16.** Procédé pour la production d'un anticorps bispécifique selon l'une quelconque des revendications 1 à 12, ledit procédé comprenant la culture d'une cellule hôte telle que définie dans la revendication 15 dans des conditions permettant l'expression de l'anticorps tel que défini dans l'une quelconque des revendications 1 à 12 et la récupération

de l'anticorps produit à partir de la culture.

**17.** Composition pharmaceutique comprenant un anticorps bispécifique selon l'une quelconque des revendications 1 à 12, ou produit selon le procédé de la revendication 16.

**18.** Anticorps bispécifique selon l'une quelconque des revendications 1 à 12, ou produit selon le procédé de la revendication 16 destiné à être utilisé dans la prévention, le traitement ou l'amélioration d'une maladie choisie dans le groupe constitué d'une maladie proliférative, une maladie inflammatoire, une maladie infectieuse et une maladie auto-immune.

**19.** Kit comprenant un anticorps bispécifique tel que défini dans l'une quelconque des revendications 1 à 12, une molécule d'acide nucléique telle que définie dans la revendication 13, un vecteur tel que défini dans la revendication 14 ou une cellule hôte telle que définie dans la revendication 15.

Figure 1(1)

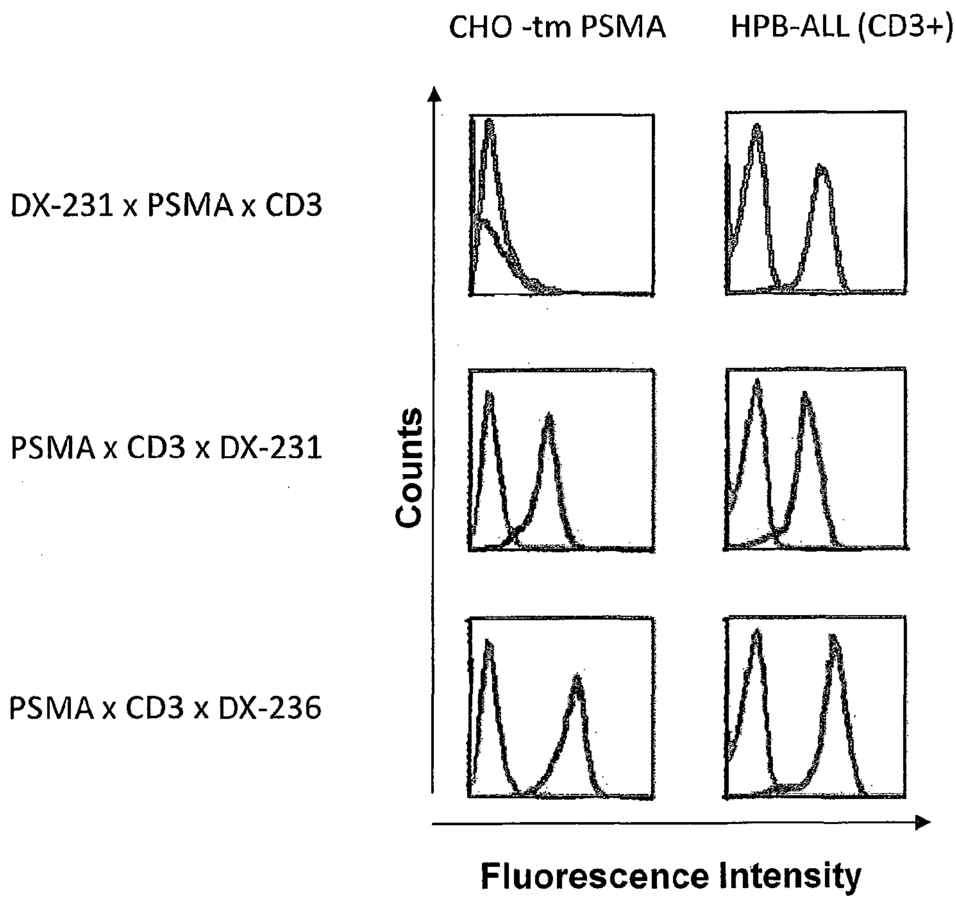
CHO -tm PSMA
HPB-ALL (CD3+)
DX-231 x PSMA x CD3
PSMA x CD3 x DX-231
PSMA x CD3 x DX-236
Counts
Fluorescence Intensity

Figure 1(2)

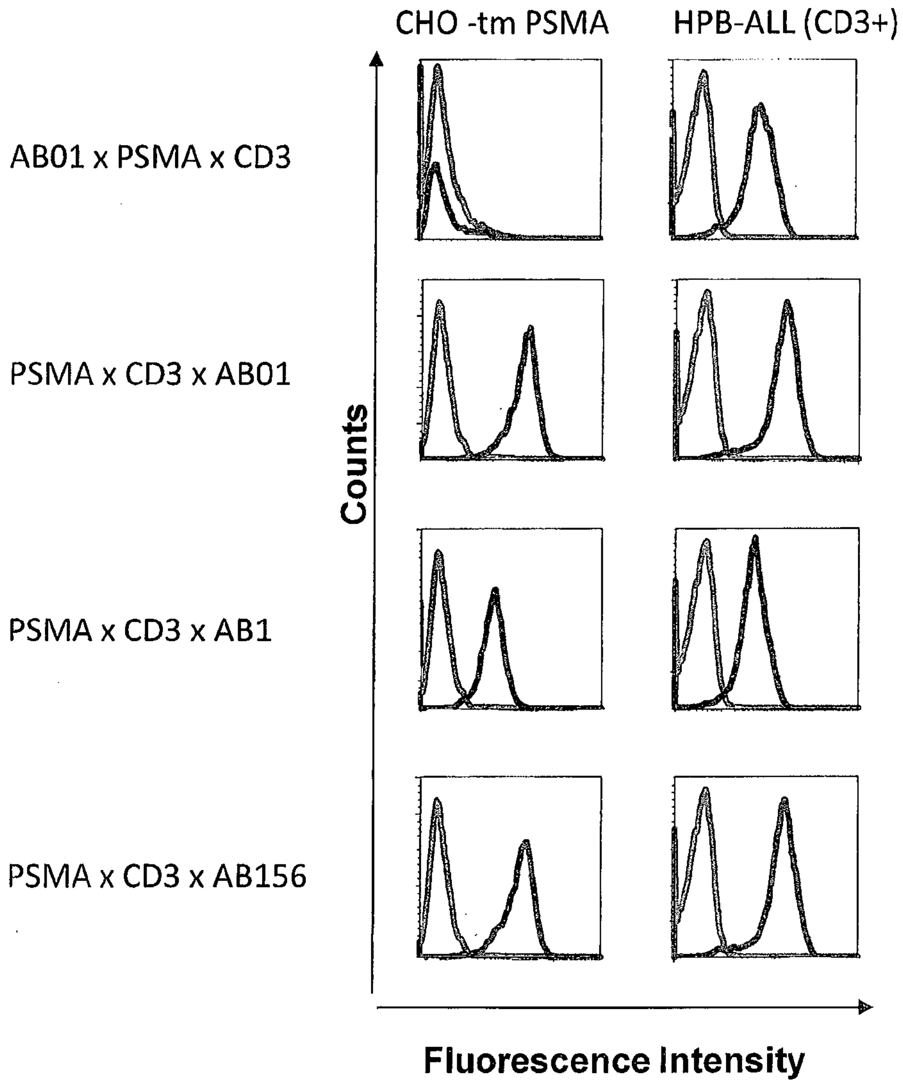
CHO -tm PSMA
HPB-ALL (CD3+)
AB01 x PSMA x CD3
PSMA x CD3 x AB01
PSMA x CD3 x AB1
PSMA x CD3 x AB156
Counts
Fluorescence Intensity

# Figure 1(3)

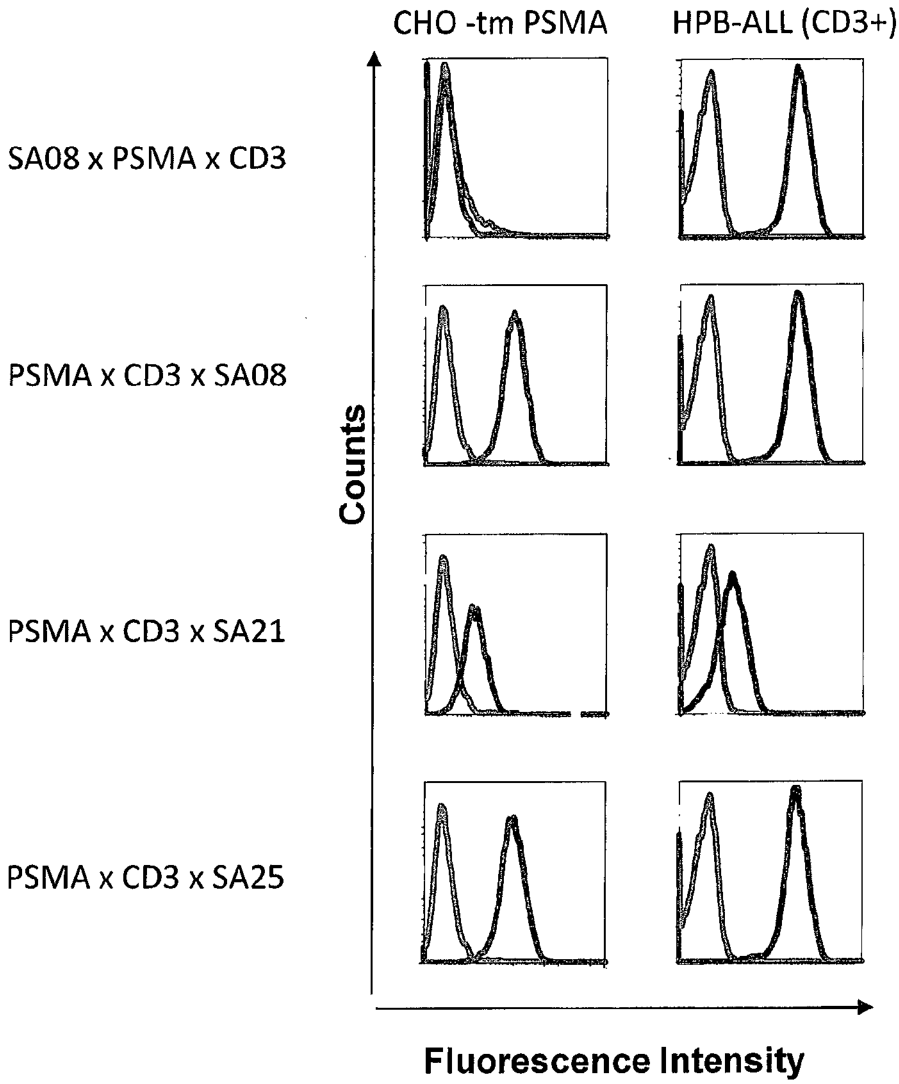
CHO -tm PSMA
HPB-ALL (CD3+)
SA08 x PSMA x CD3
PSMA x CD3 x SA08
PSMA x CD3 x SA21
PSMA x CD3 x SA25
Counts
Fluorescence Intensity

## Figure 2(1)

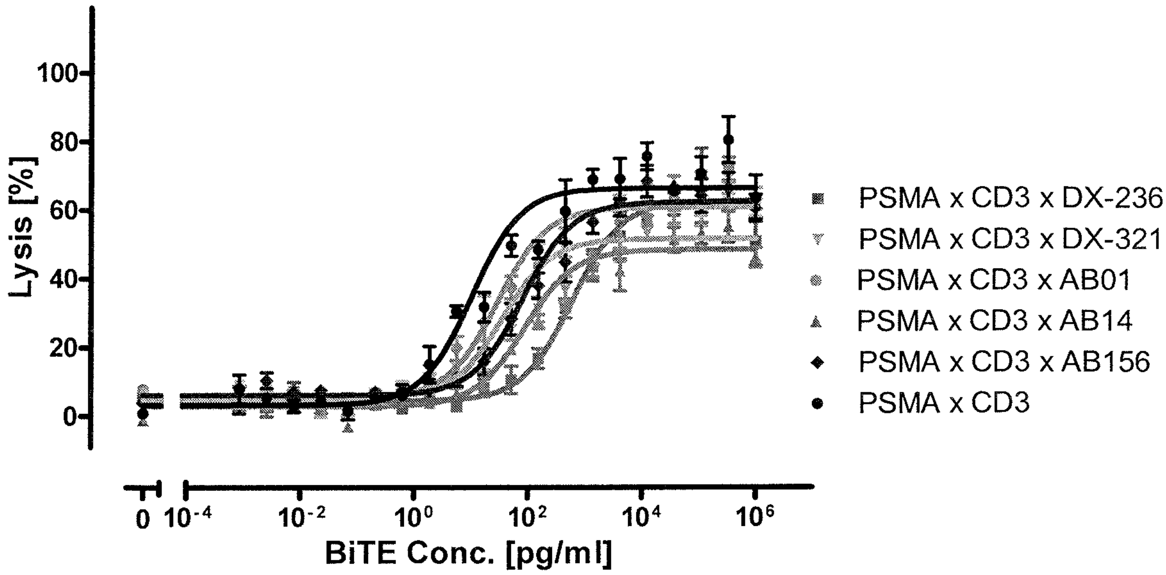


| | PSMA X CD3 x DX236 | PSMA X CD3 x DX-321 | PSMA X CD3 |
|---|---|---|---|
| EC50 [pg/ml] | 599 | 35 | 10 |

| | PSMA X CD3 x AB01 | PSMA X CD3 x AB14 | PSMA X CD3 x AB156 | PSMA X CD3 |
|---|---|---|---|---|
| EC50 [pg/ml] | 29 | 88 | 81 | 10 |

Figure 2(2)

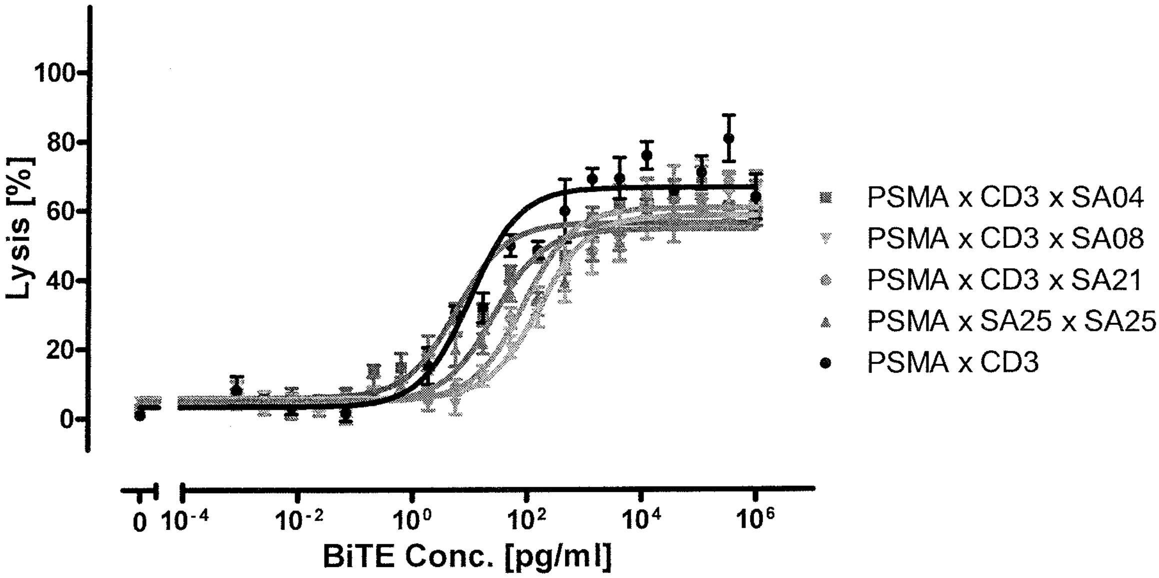


| | PSMA X CD3 x SA04 | PSMA X CD3 x SA08 | PSMA X CD3 x SA21 | PSMA X CD3 x SA25 |
|---|---|---|---|---|
| EC50 [pg/ml] | 5.8 | 164 | 87 | 26 |

# Figure 3

EpCAM BiTE

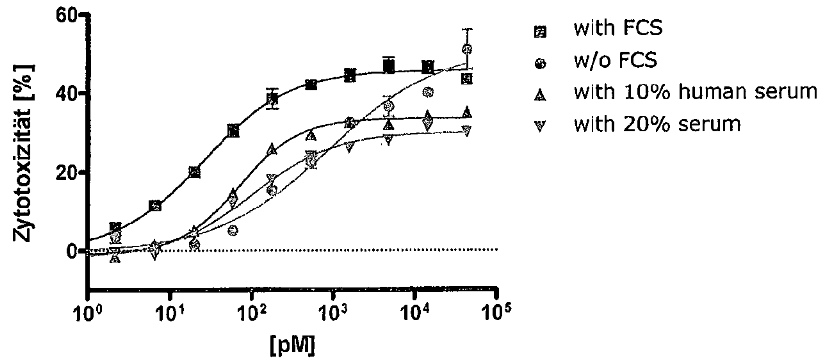


EpCAM BiTE-SA21

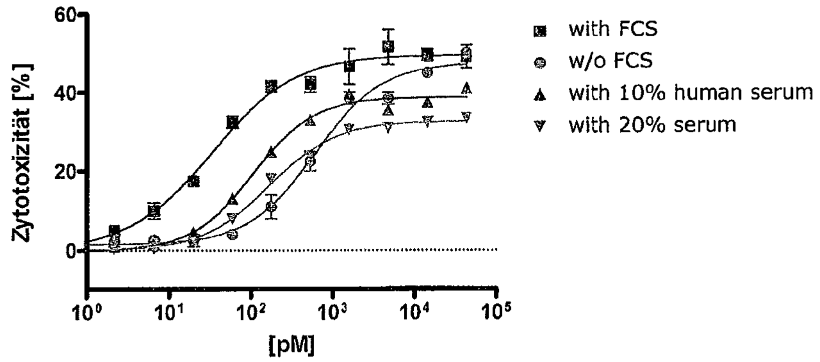


EpCAM BiTE-HSA

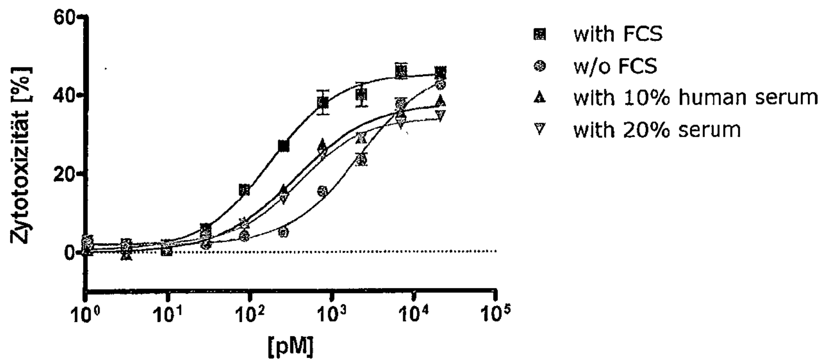

# Figure 4

**EpCAM BiTE**

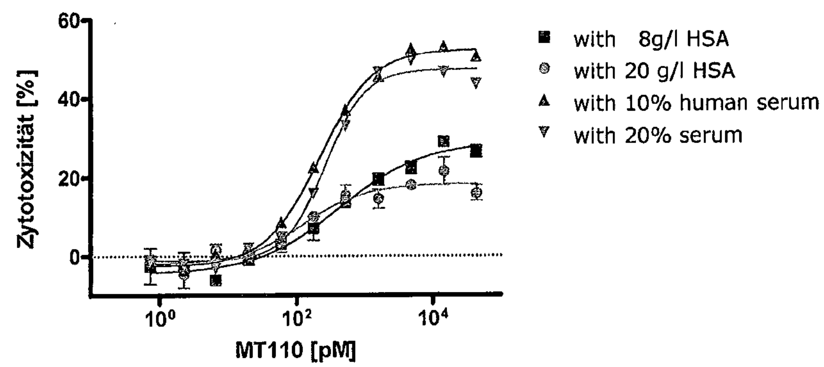


**EpCAM BiTE-SA21**

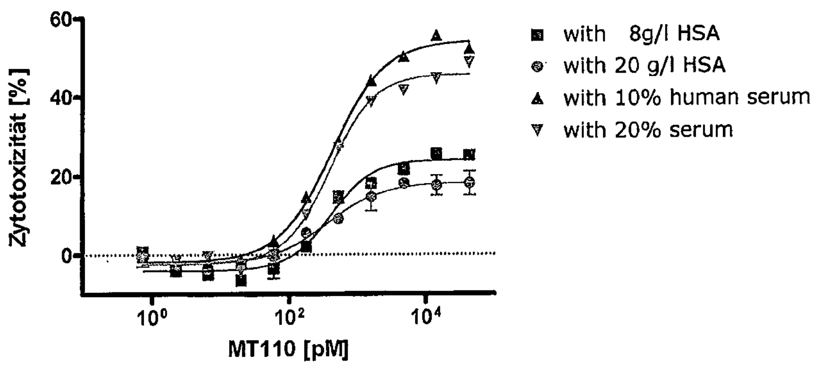


**EpCAM BiTE-HSA**

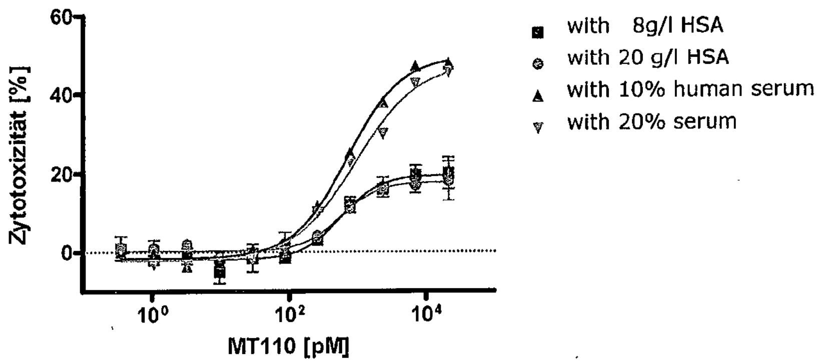

# Figure 5(1)

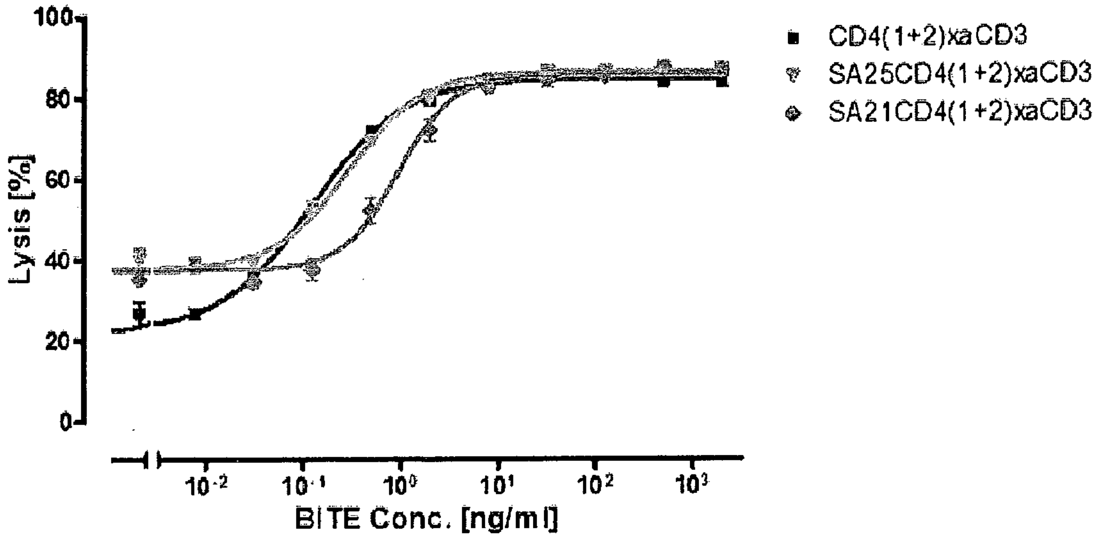
Lysis [%]
100
80
60
40
20
0
10-2
10-1
10^0
10^1
10^2
10^3
BiTE Conc. [ng/ml]
CD4(1+2)xaCD3
SA25CD4(1+2)xaCD3
SA21CD4(1+2)xaCD3

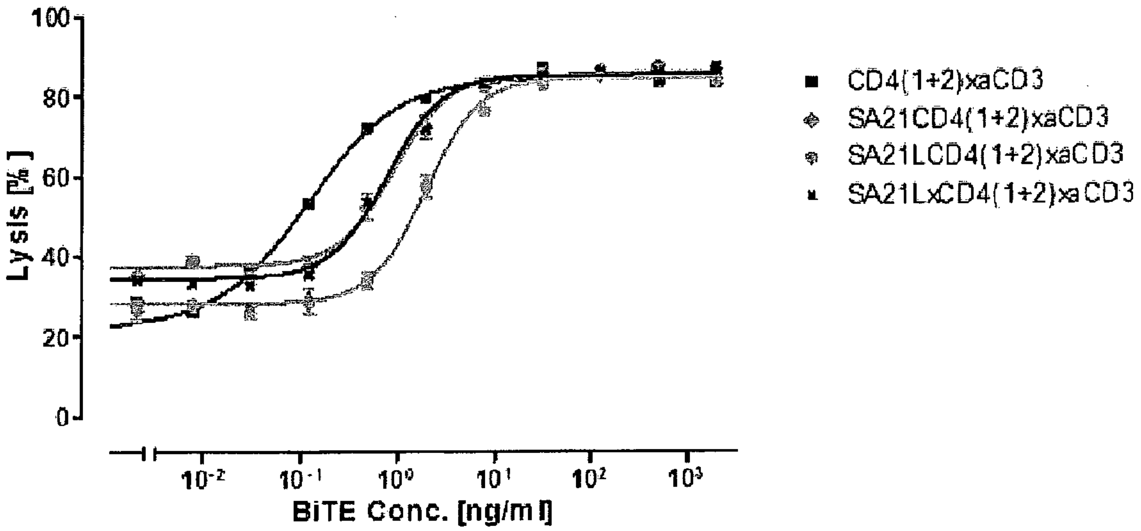
Lysis [%]
100
80
60
40
20
0
10-2
10-1
10^0
10^1
10^2
10^3
BiTE Conc. [ng/ml]
CD4(1+2)xaCD3
SA21CD4(1+2)xaCD3
SA21LCD4(1+2)xaCD3
SA21LxCD4(1+2)xaCD3

**Figure 5(2)**

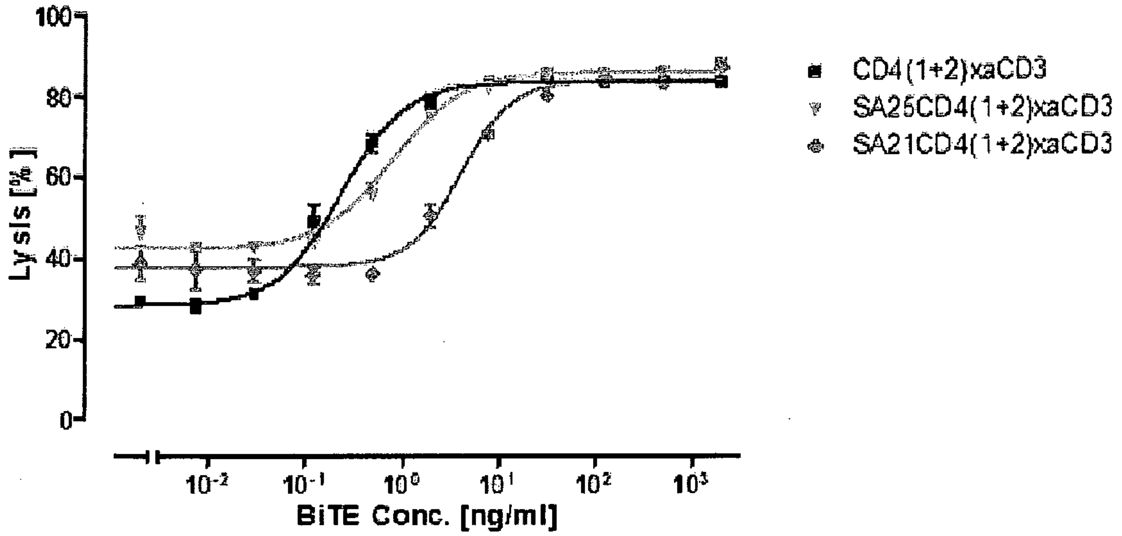
100
80
60
40
20
0
Lysis [%]
10-2
10-1
100
101
102
103
BiTE Conc. [ng/ml]
CD4(1+2)xaCD3
SA25CD4(1+2)xaCD3
SA21CD4(1+2)xaCD3

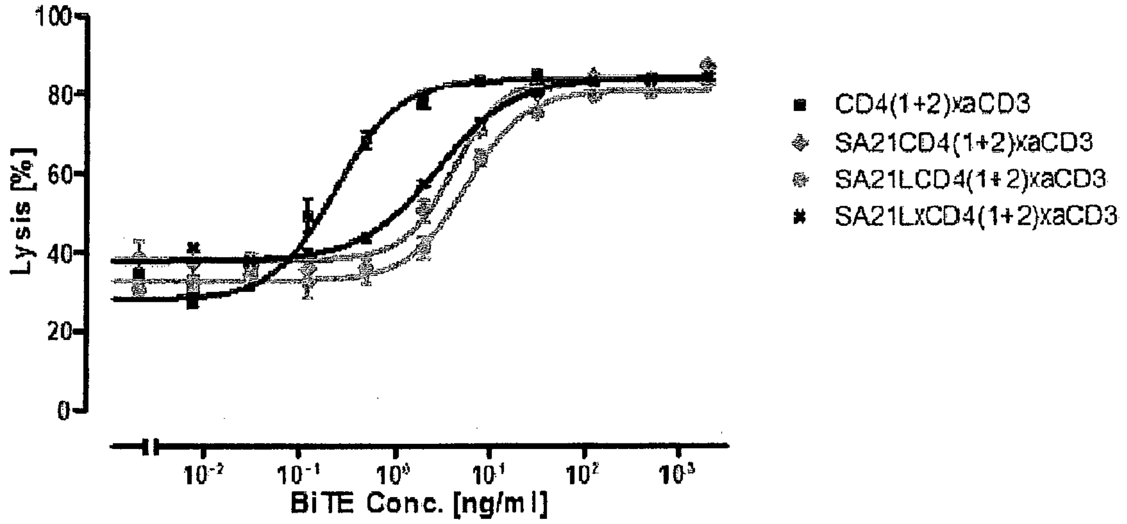
100
80
60
40
20
0
Lysis [%]
10-2
10-1
100
101
102
103
BiTE Conc. [ng/ml]
CD4(1+2)xaCD3
SA21CD4(1+2)xaCD3
SA21LCD4(1+2)xaCD3
SA21LxCD4(1+2)xaCD3

# Figure 5(3)

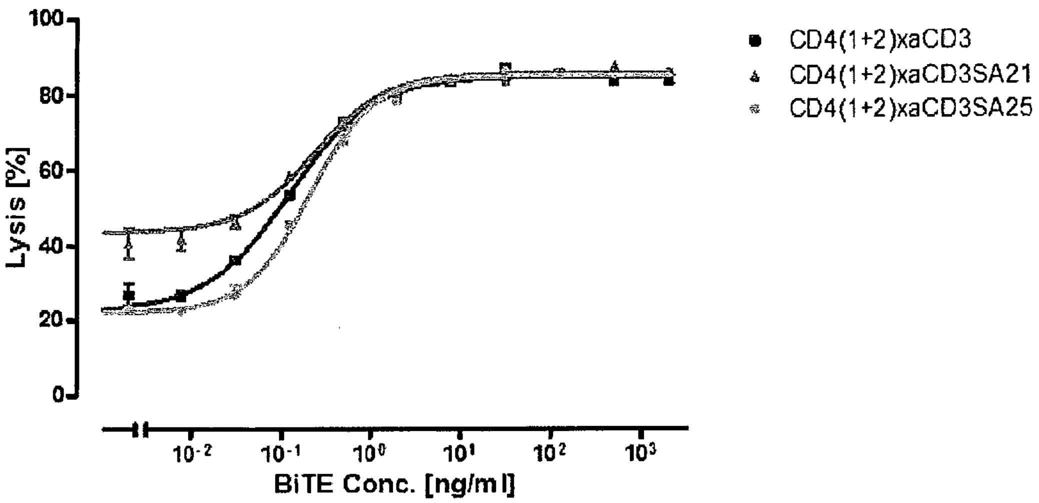
100
80
60
40
20
0
Lysis [%]
10-2
10-1
10^0
10^1
10^2
10^3
BiTE Conc. [ng/ml]
CD4(1+2)xaCD3
CD4(1+2)xaCD3SA21
CD4(1+2)xaCD3SA25

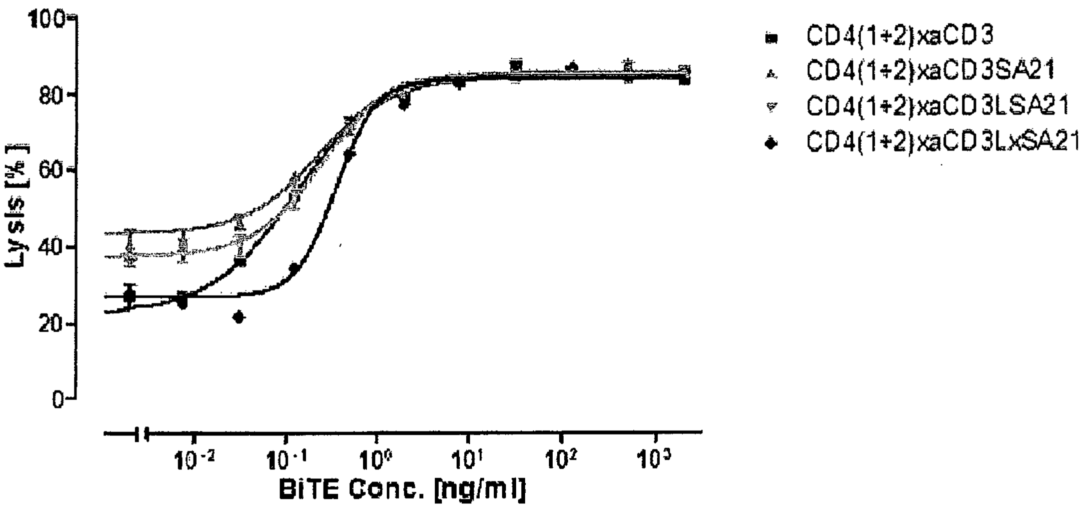
100
80
60
40
20
0
Lysis [%]
10-2
10-1
10^0
10^1
10^2
10^3
BiTE Conc. [ng/ml]
CD4(1+2)xaCD3
CD4(1+2)xaCD3SA21
CD4(1+2)xaCD3LSA21
CD4(1+2)xaCD3LxSA21

# Figure 5(4)

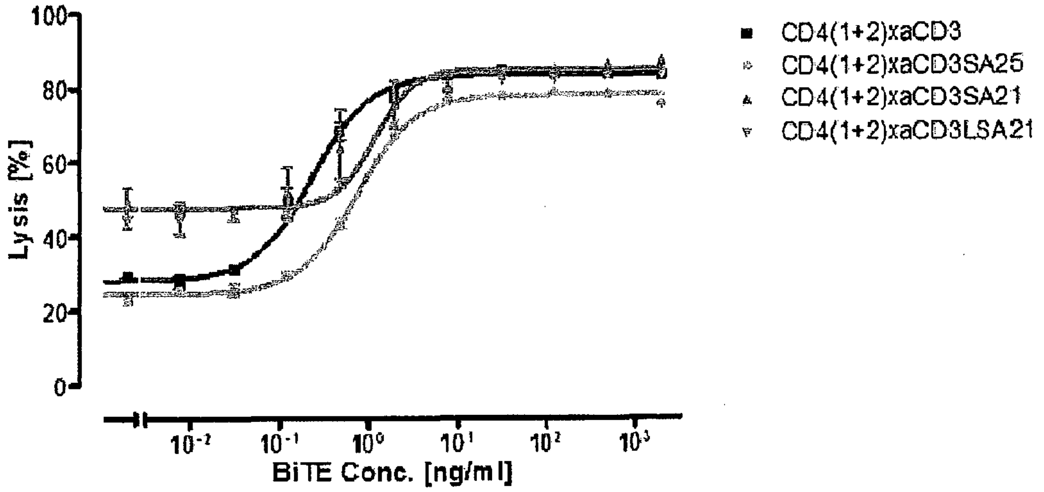
100
80
60
40
20
0
Lysis [%]
10⁻²
10⁻¹
10⁰
10¹
10²
10³
BiTE Conc. [ng/ml]
CD4(1+2)xaCD3
CD4(1+2)xaCD3SA25
CD4(1+2)xaCD3SA21
CD4(1+2)xaCD3LSA21

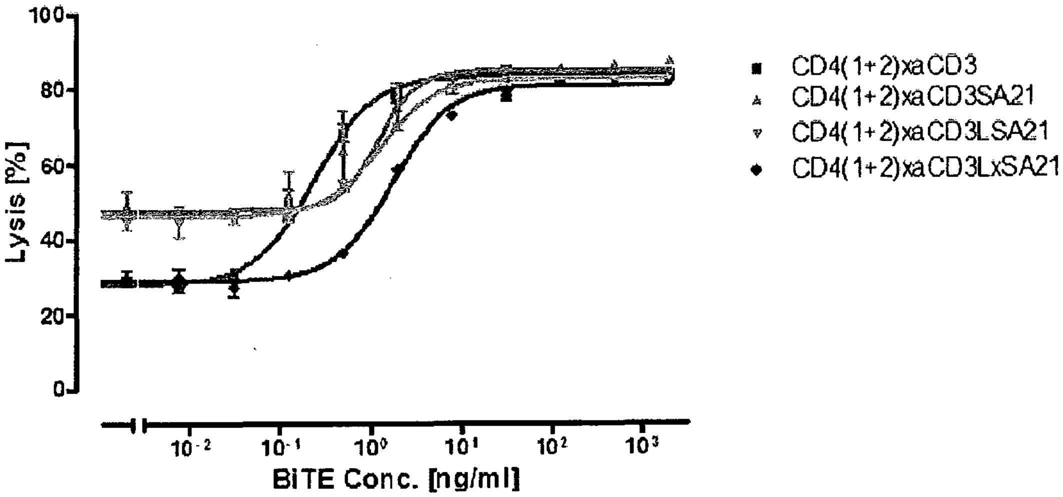
100
80
60
40
20
0
Lysis [%]
10⁻²
10⁻¹
10⁰
10¹
10²
10³
BiTE Conc. [ng/ml]
CD4(1+2)xaCD3
CD4(1+2)xaCD3SA21
CD4(1+2)xaCD3LSA21
CD4(1+2)xaCD3LxSA21

Figure 5(5)

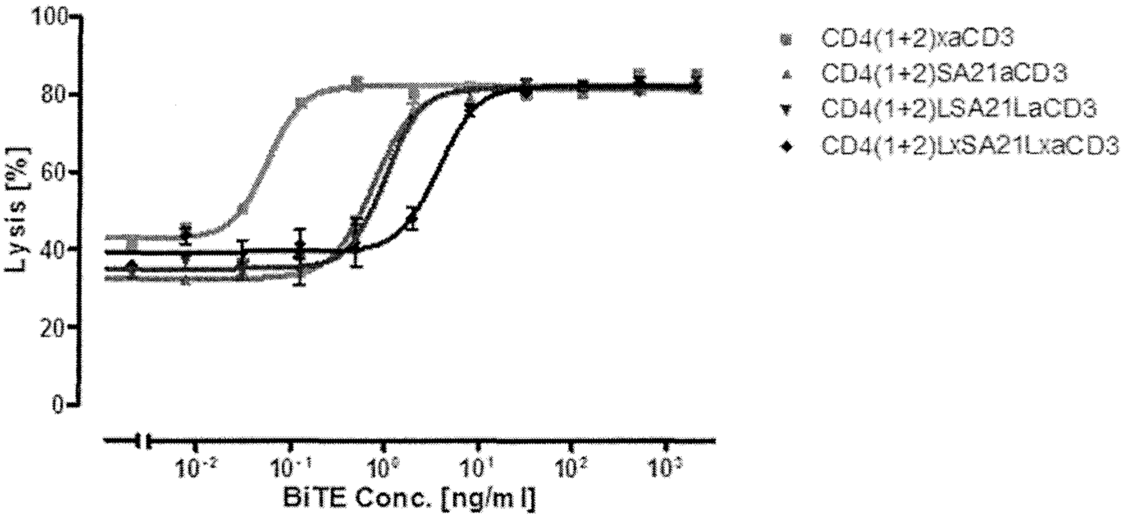
100
80
60
40
20
0
Lysis [%]
10-2
10-1
100
101
102
103
BiTE Conc. [ng/ml]
CD4(1+2)xaCD3
CD4(1+2)SA21aCD3
CD4(1+2)LSA21LaCD3
CD4(1+2)LxSA21LxaCD3

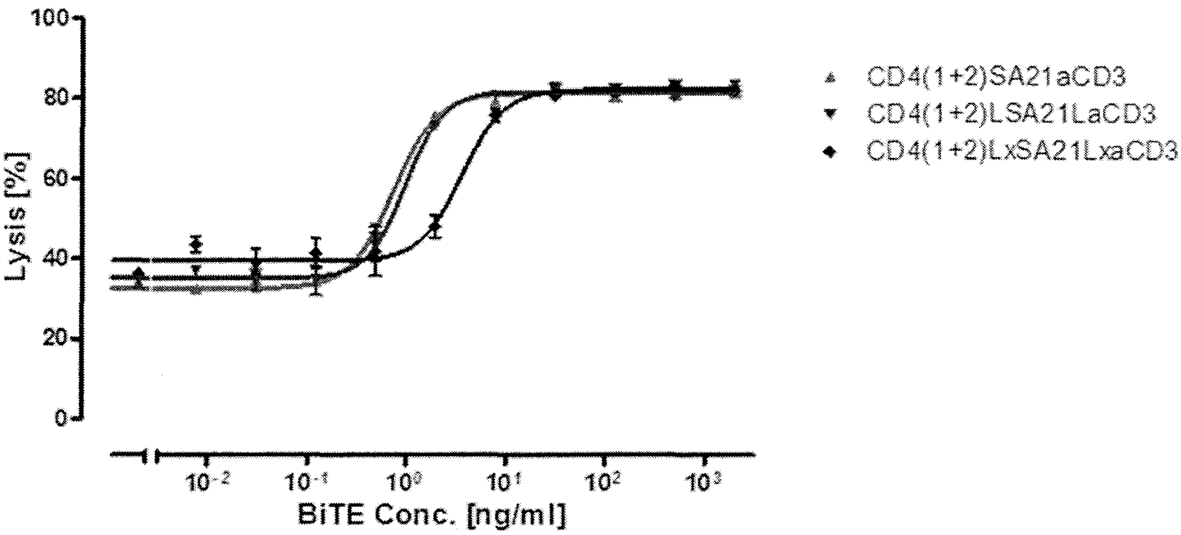
100
80
60
40
20
0
Lysis [%]
10-2
10-1
100
101
102
103
BiTE Conc. [ng/ml]
CD4(1+2)SA21aCD3
CD4(1+2)LSA21LaCD3
CD4(1+2)LxSA21LxaCD3

Figure 5(6)

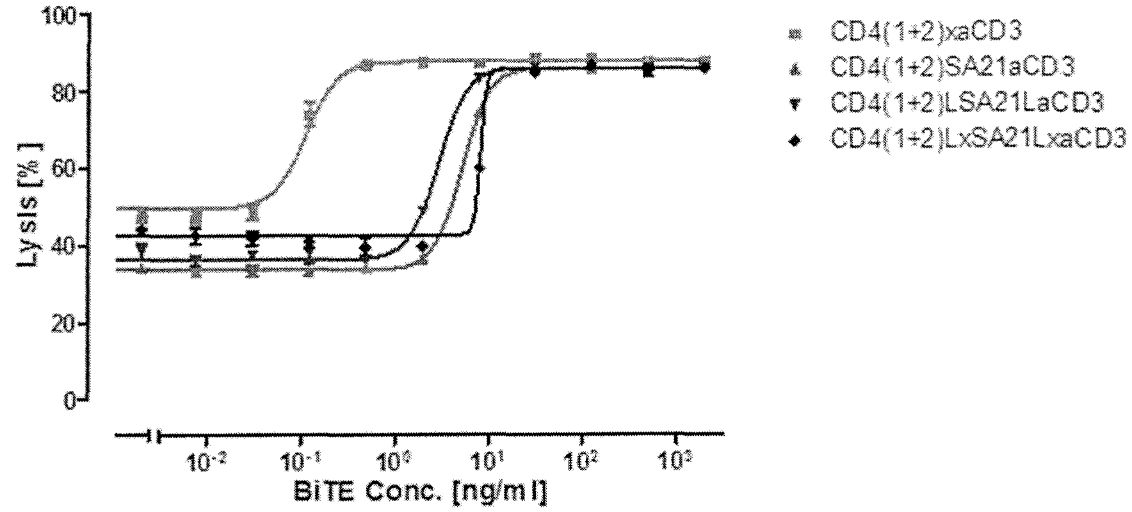
CD4(1+2)xaCD3
CD4(1+2)SA21aCD3
CD4(1+2)LSA21LaCD3
CD4(1+2)LxSA21LxaCD3
Lysis [%]
BiTE Conc. [ng/ml]

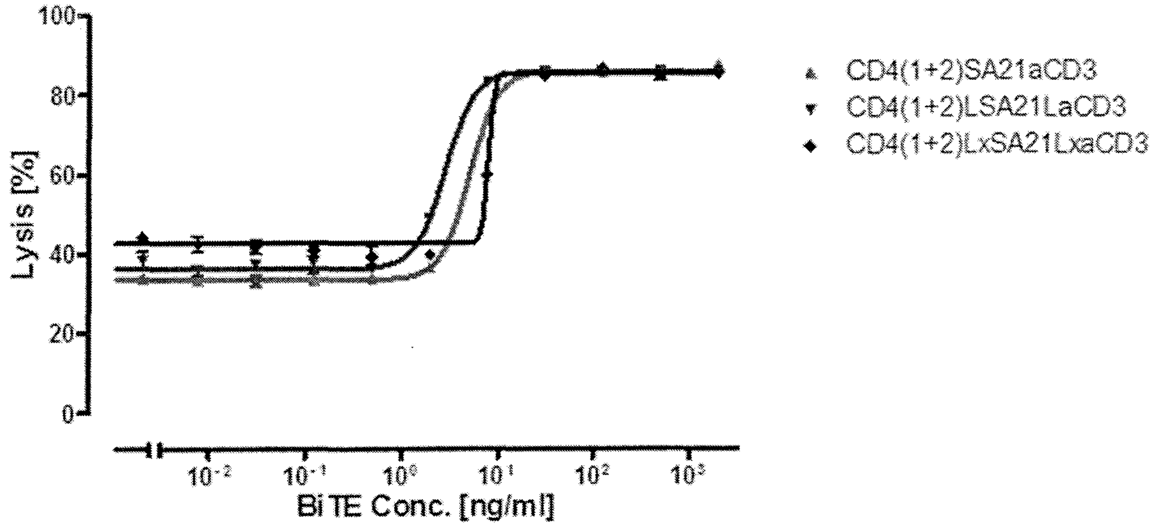
CD4(1+2)SA21aCD3
CD4(1+2)LSA21LaCD3
CD4(1+2)LxSA21LxaCD3
Lysis [%]
BiTE Conc. [ng/ml]

Figure 6(1)

Human Serum Albumin | Mouse Serum Alumin

Response [RU]

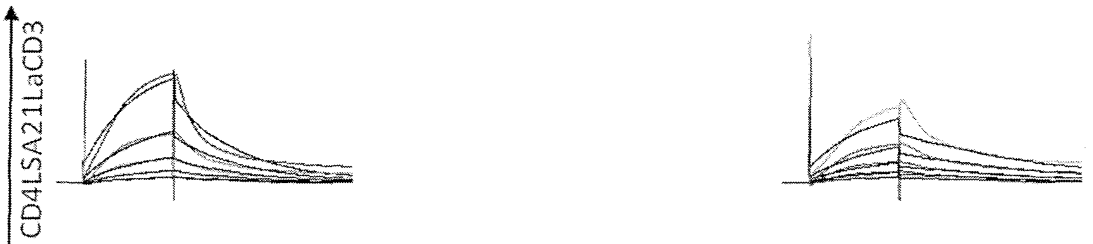


| Ligand | Sample | Fit | ka | kd | KD |
|---|---|---|---|---|---|
| human Serum Albumin | CD4LSA21aCD3 | 1:1 Binding | 3.78E+04 | 2.92E-02 | 7.73E-07 |
| mouse Serum Albumin | CD4LSA21aCD3 | 1:1 Binding | 1.34E+04 | 3.09E-03 | 2.31E-07 |

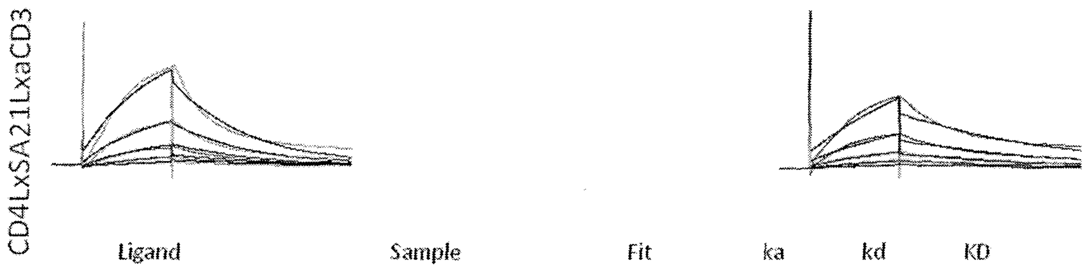


| Ligand | Sample | Fit | ka | kd | KD |
|---|---|---|---|---|---|
| human Serum Albumin | wdh CD4LxSA21 | 1:1 Binding | 4.64E+02 | 6.97E-03 | 1.50E-05 |
| mouse Serum Albumin | wdh CD4LxSA21 | 1:1 Binding | 7.60E+02 | 3.10E-03 | 4.08E-06 |

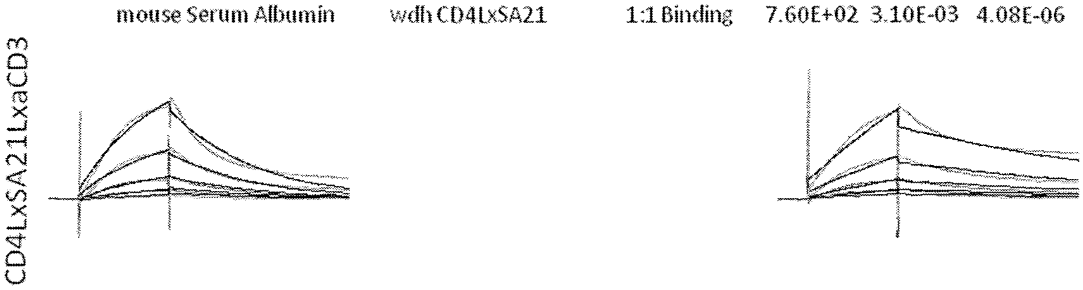


| Ligand | Sample | Fit | ka | kd | KD |
|---|---|---|---|---|---|
| human Serum Albumin | CD4LxSA21LxaCD3 | 1:1 Binding | 1.36E+03 | 6.15E-03 | 4.52E-06 |
| mouse Serum Albumin | CD4LxSA21LxaCD3 | 1:1 Binding | 2.12E+02 | 1.74E-03 | 8.21E-06 |

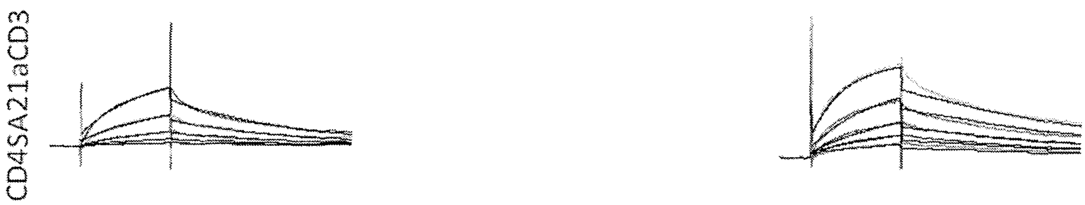


| Ligand | Sample | Fit | ka | kd | KD |
|---|---|---|---|---|---|
| human Serum Albumin | CD4SA21aCD3 | 1:1 Binding | 8.26E+03 | 3.84E-03 | 4.65E-07 |
| mouse Serum Albumin | CD4SA21aCD3 | 1:1 Binding | 3.02E+04 | 2.11E-03 | 6.98E-08 |

Time [seconds]

Figure 6(2)

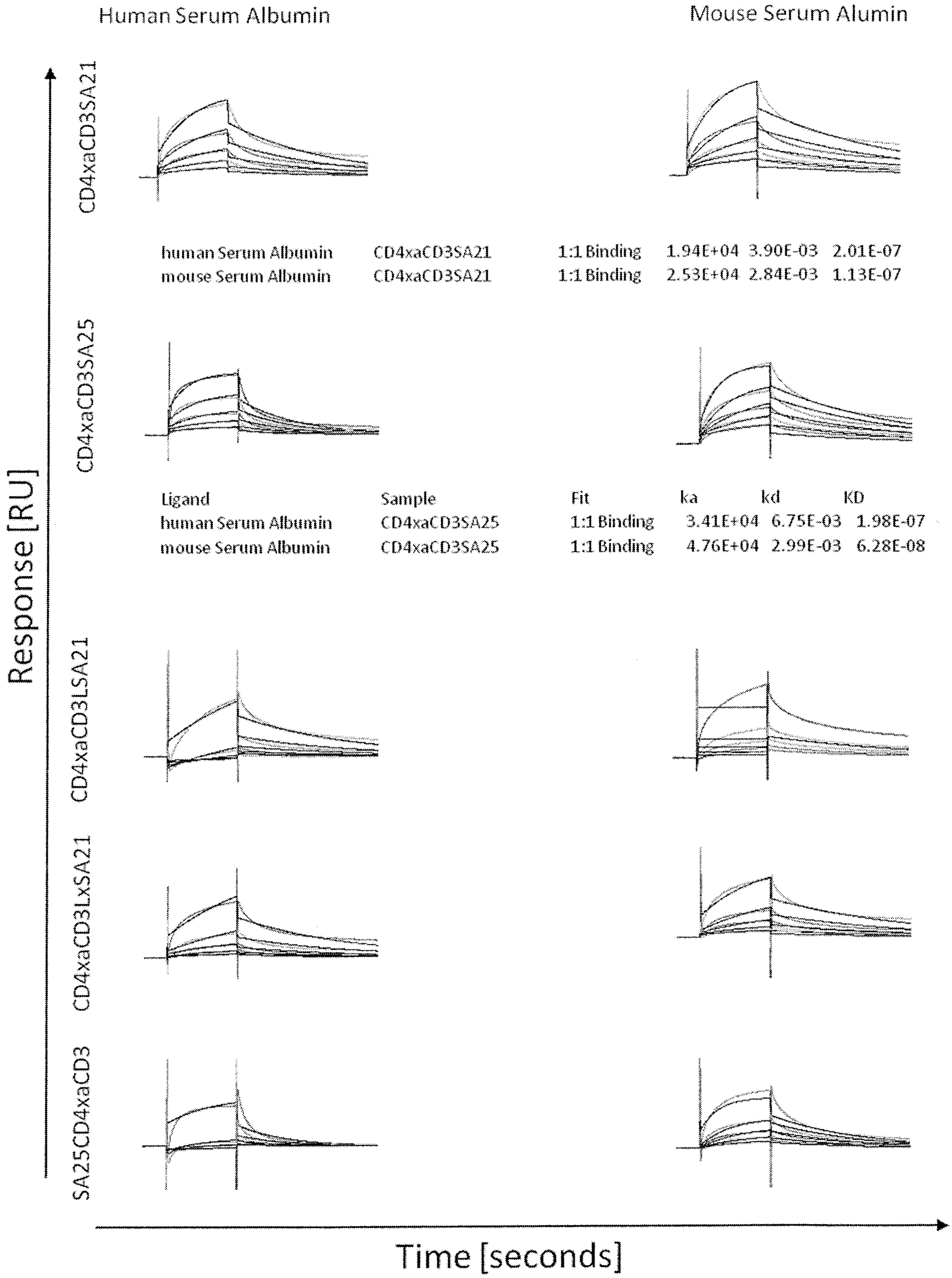
Human Serum Albumin
Mouse Serum Alumin
CD4xaCD3SA21
human Serum Albumin CD4xaCD3SA21 1:1 Binding 1.94E+04 3.90E-03 2.01E-07
mouse Serum Albumin CD4xaCD3SA21 1:1 Binding 2.53E+04 2.84E-03 1.13E-07
CD4xaCD3SA25
Ligand Sample Fit ka kd KD
human Serum Albumin CD4xaCD3SA25 1:1 Binding 3.41E+04 6.75E-03 1.98E-07
mouse Serum Albumin CD4xaCD3SA25 1:1 Binding 4.76E+04 2.99E-03 6.28E-08
CD4xaCD3LSA21
CD4xaCD3LxSA21
SA25CD4xaCD3
Response [RU]
Time [seconds]

Figure 6(3)

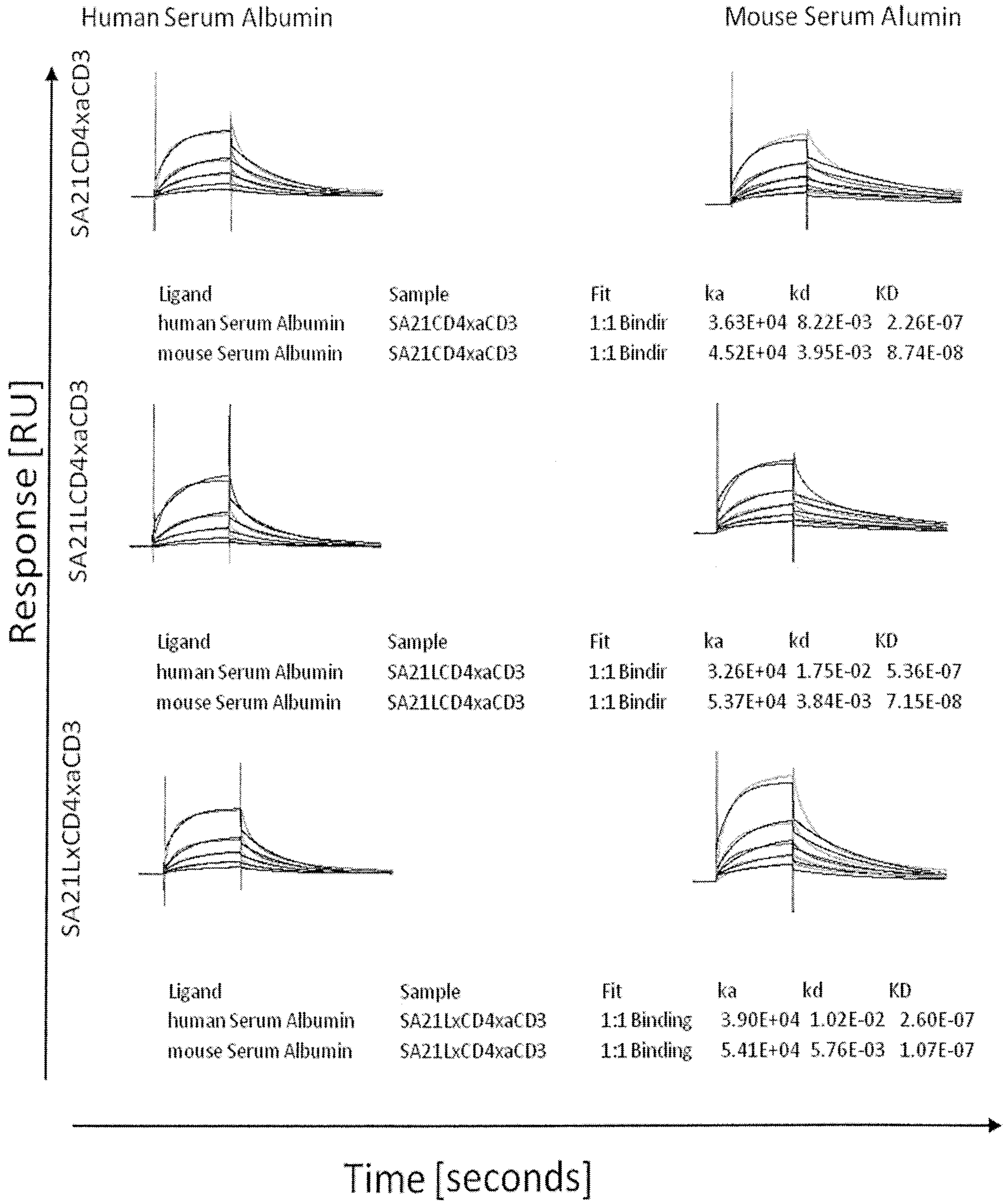


| Ligand | Sample | Fit | ka | kd | KD |
|---|---|---|---|---|---|
| human Serum Albumin | SA21CD4xaCD3 | 1:1 Bindir | 3.63E+04 | 8.22E-03 | 2.26E-07 |
| mouse Serum Albumin | SA21CD4xaCD3 | 1:1 Bindir | 4.52E+04 | 3.95E-03 | 8.74E-08 |

| Ligand | Sample | Fit | ka | kd | KD |
|---|---|---|---|---|---|
| human Serum Albumin | SA21LCD4xaCD3 | 1:1 Bindir | 3.26E+04 | 1.75E-02 | 5.36E-07 |
| mouse Serum Albumin | SA21LCD4xaCD3 | 1:1 Bindir | 5.37E+04 | 3.84E-03 | 7.15E-08 |

| Ligand | Sample | Fit | ka | kd | KD |
|---|---|---|---|---|---|
| human Serum Albumin | SA21LxCD4xaCD3 | 1:1 Binding | 3.90E+04 | 1.02E-02 | 2.60E-07 |
| mouse Serum Albumin | SA21LxCD4xaCD3 | 1:1 Binding | 5.41E+04 | 5.76E-03 | 1.07E-07 |

Figure 7

CD4(1+2)xacD3 on huCD3

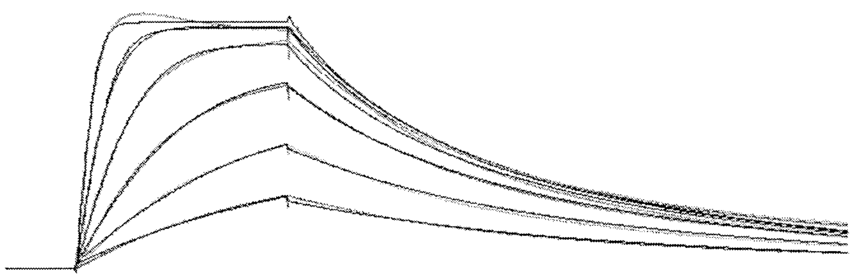

| ka (1/Ms) | kd (1/s) | KD (M) | Rmax (RU) | Chi² (RU²) |
|---|---|---|---|---|
| 3.45E+06 | 7.98E-03 | 2.31E-09 | 67.29 | 1.10E+00 |

CD4(1+2)xI2CSA21 on huCD3

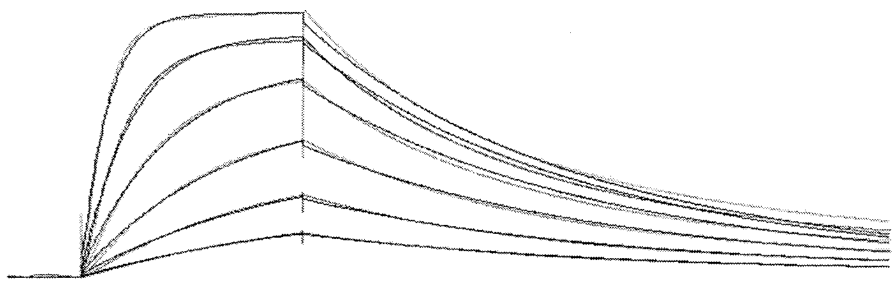

| ka (1/Ms) | kd (1/s) | KD (M) | Rmax (RU) | Chi² (RU²) |
|---|---|---|---|---|
| 1.03E+06 | 4.48E-03 | 4.33E-09 | 75.36 | 9.56E-01 |

CD4(1+2)xI2CSA25on huCD3

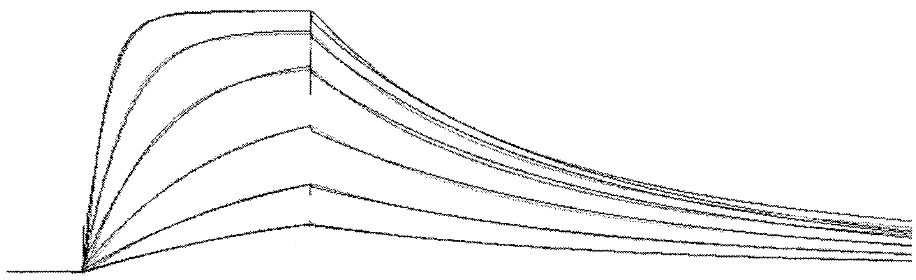

| ka (1/Ms) | kd (1/s) | KD (M) | Rmax (RU) | Chi² (RU²) |
|---|---|---|---|---|
| 1.30E+06 | 4.91E-03 | 3.77E-09 | 72.44 | 5.93E-01 |

Response [RU]

Time [seconds]

Figure 8

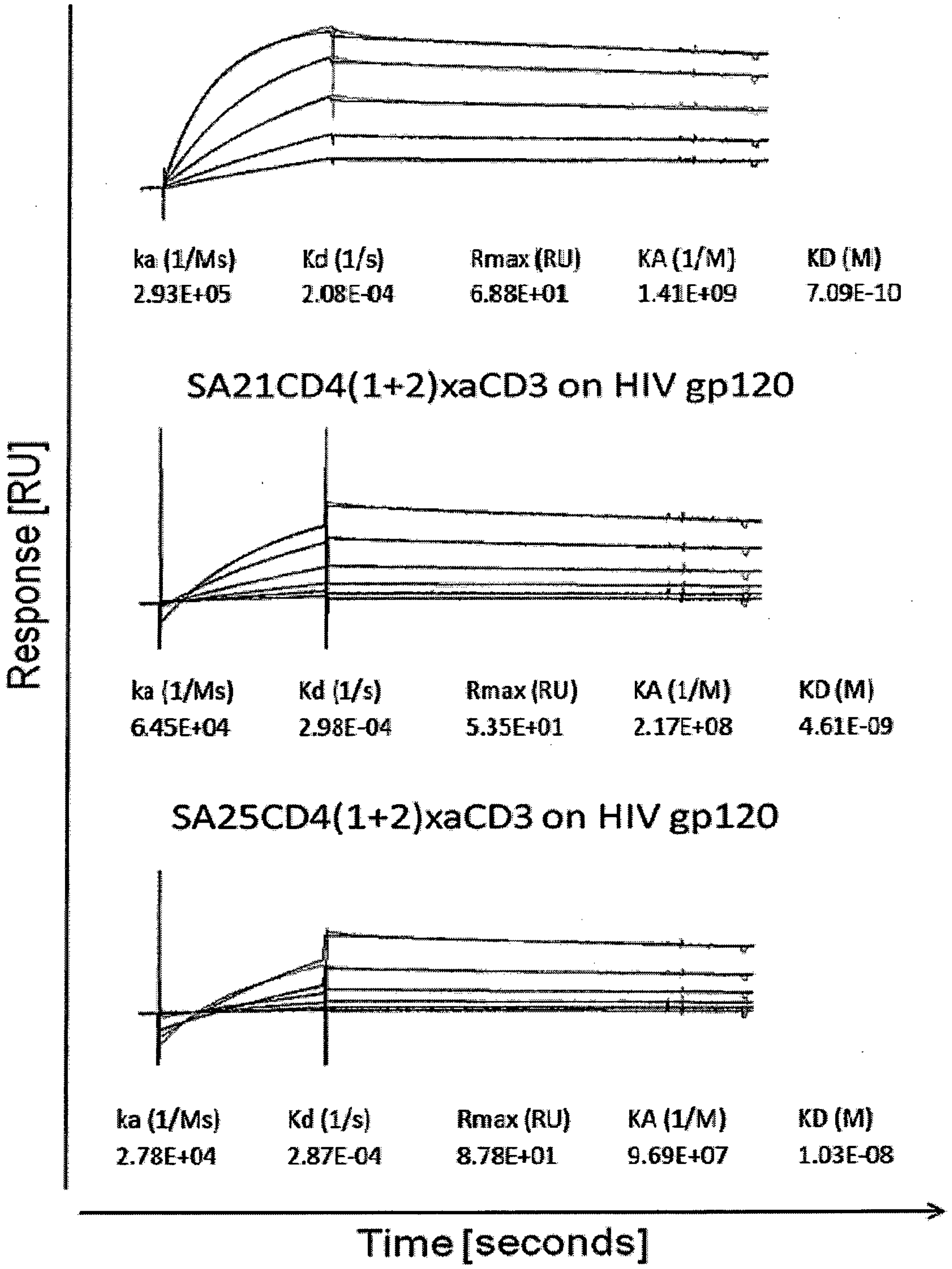
ka (1/Ms) Kd (1/s) Rmax (RU) KA (1/M) KD (M) Chi2
2.93E+05 2.08E-04 6.88E+01 1.41E+09 7.09E-10 0.216
SA21CD4(1+2)xaCD3 on HIV gp120
ka (1/Ms) Kd (1/s) Rmax (RU) KA (1/M) KD (M) Chi2
6.45E+04 2.98E-04 5.35E+01 2.17E+08 4.61E-09 0.073
SA25CD4(1+2)xaCD3 on HIV gp120
ka (1/Ms) Kd (1/s) Rmax (RU) KA (1/M) KD (M) Chi2
2.78E+04 2.87E-04 8.78E+01 9.69E+07 1.03E-08 0,147
Response [RU]
Time [seconds]

**Figure 9**

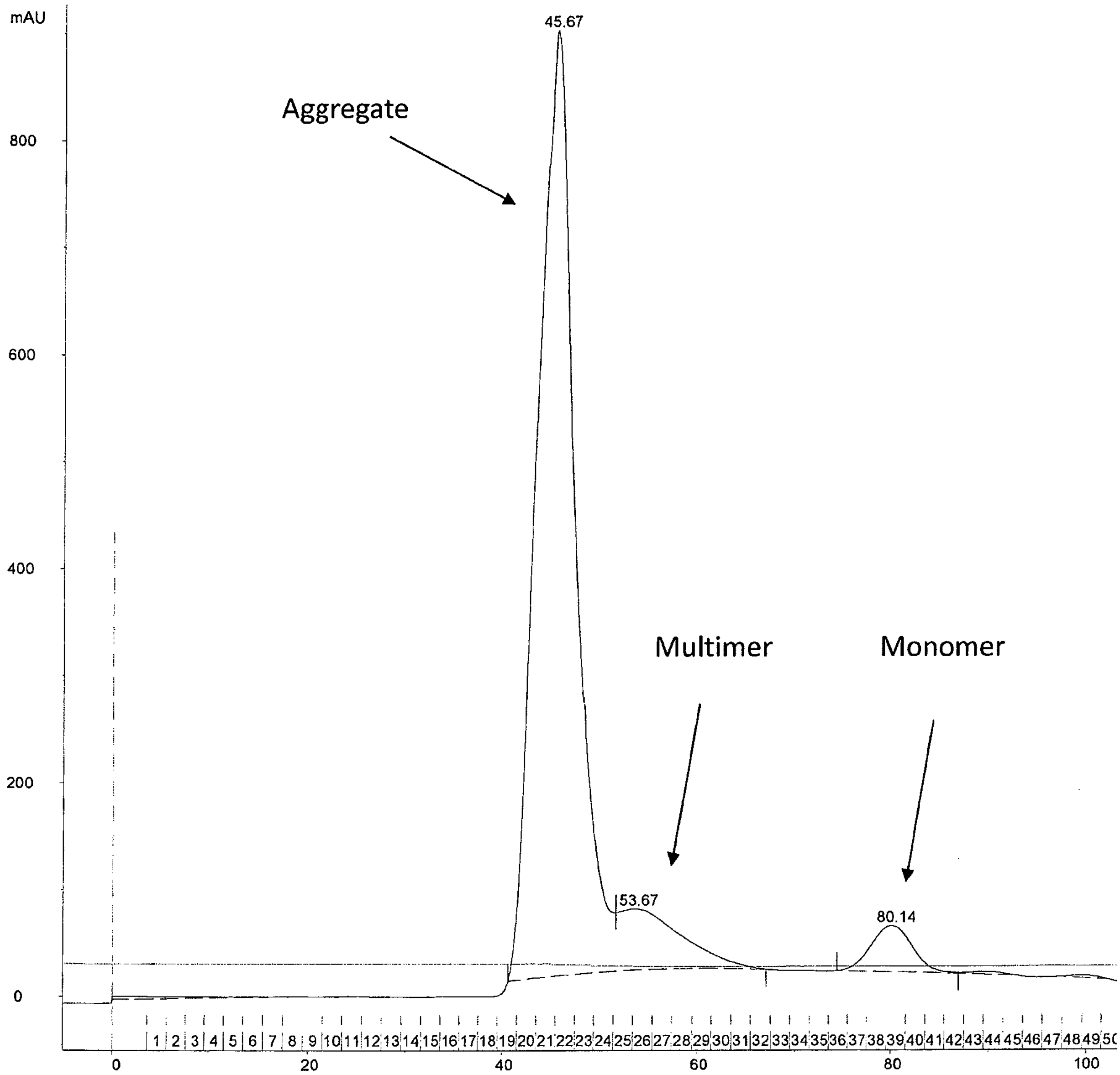
mAU
800
600
400
200
0
45.67
Aggregate
Multimer
Monomer
53.67
80.14
1 2 3 4 5 6 7 8 9 10 11 12 13 14 15 16 17 18 19 20 21 22 23 24 25 26 27 28 29 30 31 32 33 34 35 36 37 38 39 40 41 42 43 44 45 46 47 48 49 50
0
20
40
60
80
100

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010037836 A2
- WO 0145746 A
- WO 2010080538 A
- WO 2007042261 A
- US 4946778 A, Kontermann and Dübel
- US 5648260 A, Kontermann and Dübel
- WO 9954440 A
- US 4751180 A
- US 4935233 A
- WO 8809344 A
- US 4816567 A
- US 4816 A
- US 567 A
- US 5223409 A, Ladner
- US 20030070185 A
- WO 9634096 A
- WO 9633735 A
- US 4816397 A, Boss
- EP 0171496 A, Tanaguchi
- EP 0173494 A
- GB 2177096 A
- US 5225539 A
- US 5585089 A
- US 5693761 A
- US 5693762 A
- US 5859205 A
- US 6407213 B
- EP 239400 A
- WO 9852976 A
- WO 0034317 A
- US 6300064 B
- US 5565332 A
- WO 2008119567 A
- US 4485045 A
- US 4544545 A
- WO 9738731 A
- US 5013 A
- US 556 A
- EP 244234 A
- WO 2010037836 A
- WO 2005040220 A


### Non-patent literature cited in the description


- **BAEUERLE PATRICK A et al.** BiTE: Teaching antibodies to engage T-cells for cancer therapy. *CURRENT OPINION IN MOLECULAR THERAPEUTICS,* 2009, vol. 11, 22-30
- **WOLF E et al.** BiTEs: bispecific antibody constructs with unique anti-tumor activity. *Drug Discovery Today,* September 2005, vol. 10 (18
- **TRAUNECKER A et al.** Janusin: new molecular design for bispecific reagents. *INTERNAT J CANCER,* 1992, vol. 7, 51-52
- **STORK et al.** *Prot Eng Des Sel,* 2007, vol. 20 (11), 569-576
- **MUELLER et al.** *J Biol Chem,* 2007, vol. 282 (17), 12650-12660
- **STUMPP et al.** *Curr Opin Drug Discov Devel.,* 2007, vol. 10 (2), 153-159
- **SKERRA.** *Curr. Opin. Biotechnol.,* 2005, vol. 18, 295-304
- **HOSSE et al.** *Protein Sci.,* 2006, vol. 15, 14-27
- **NICAISE et al.** *Protein Sci.,* 2004, vol. 13, 1882-1891
- **NYGREN ; UHLEN.** *Curr. Opin. Struc. Biol.,* 1997, vol. 7, 463-469
- **MILSTEIN ; KÖHLER.** *Nature,* 1975, vol. 256, 495-7
- **KONTERMANN ; DÜBEL.** Antibody Engineering. Springer, 2010
- **LITTLE.** Recombinant Antibodies for Immunotherapy. Cambridge University Press, 2009
- **KÖHLER ; MILSTEIN.** *Nature,* 1975, vol. 256, 495-497
- **KOZBOR.** *Immunology Today,* 1983, vol. 4, 72
- **COLE et al.** Monoclonal Antibodies and Cancer Therapy. Alan R. Liss, Inc, 1985, 77-96
- **SCHIER.** *Human Antibodies Hybridomas,* 1996, vol. 7, 97-105
- **MALMBORG.** *J. Immunol. Methods,* 1995, vol. 183, 7-13
- **HAWKINS et al.** *J. Mol. Biol.,* 1992, vol. 254, 889-896
- **LOWMAN et al.** *Biochemistry,* 1991, vol. 30, 10832-10837
- **WARD et al.** *Nature,* 1989, vol. 341, 544-546
- **HUSTON et al.** *Proc. Natl. Acad. Sci USA,* 1988, vol. 85, 5879-5883

- **MACK.** *J. Immunol.,* 1997, vol. 158, 3965-3970
- **MACK.** *PNAS,* 1995, vol. 92, 7021-7025
- **KUFER.** *Cancer Immunol. Immunother.,* 1997, vol. 45, 193-197
- **LÖFFLER.** *Blood,* 2000, vol. 95 (6), 2098-2103
- **BRÜHL.** *Immunol.,* 2001, vol. 166, 2420-2426
- **KIPRIYANOV.** *J. Mol. Biol.,* 1999, vol. 293, 41-56
- **DALL'ACQUA et al.** *Biochem.,* 1998, vol. 37, 9266-9273
- **CHEADLE et al.** *Mol Immunol,* 1992, vol. 29, 21-30
- **RAAG ; WHITLOW.** *FASEB,* 1995, vol. 9 (1), 73-80
- **SAMBROOK et al.** Molecular Cloning: A Laboratory Manual. Cold Spring Harbor Laboratory Press, 2001
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **MORRISON et al.** *Proc. Natl. Acad. Sci. USA,* 1984, vol. 81, 6851-6855
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **NGUYEN et al.** *Prot Eng Des Sel,* 2006, vol. 19 (7), 291-297
- Chemical Stability of Pharmaceuticals: A Handbook for Pharmacists. **KENNETL, A ; PETERS et al.** Pharmacokinetc analysis: A Practical Approach. 1996
- **M GIBALDI ; D PERRON.** Pharmacokinetics. Marcel Dekker, 1982
- Chemical Stability of Pharmaceuticals: A Handbook for Pharmacists. **KENNETH, A ; PETERS et al.** Pharmacokinete analysis: A Practical Approach. 1996
- **M GIBALDI ; D PERRON.** Pharmacokinetics. Marcel Dekke, 1982
- **SONGSIVILAI ; LACHMANN.** *Clin. Exp. Immunol.,* 1990, vol. 79, 315-321
- **KOHLER ; MILSTEIN.** *Nature,* 1975, vol. 256, 495-499
- **SMITH.** *Science,* 1985, vol. 228, 1315-1317
- **GREEN et al.** *Nature Genetics,* 1994, vol. 7, 13-21
- **MORRISON et al.** *Proc. Natl. Acad. Sci U.S.A.,* 1985, vol. 81, 6851
- **TAKEDA et al.** *Nature,* 1985, vol. 314, 452
- **MORRISON.** *Science,* 1985, vol. 229, 1202-1207
- **OI et al.** *BioTechniques,* 1986, vol. 4, 214
- **TENG et al.** *Proc. Natl. Acad. Sci. U.S.A.,* 1983, vol. 80, 7308-7312
- **KOZBOR et al.** *Immunology Today,* 1983, vol. 4, 7279
- **OLSSON et al.** *Meth. Enzymol.,* 1982, vol. 92, 3-16
- **TOMLINSON et al.** *J. Mol. Biol.,* 1992, vol. 227, 776-798
- **COOK, G.P. et al.** *Immunol. Today,* 1995, vol. 16 (5), 237-242
- **TOMLINSON et al.** *EMBO J.,* 1995, vol. 14 (14), 4628-4638
- **TOMLINSON, LA. et al.** Protein Engineering. MRC Centre
- **CHOTHIA et al.** *J. Mol. Biol,* 1987, vol. 196, 901
- **MACCALLUM et al.** *J. Mol. Biol,* 1996, vol. 262, 732
- **CHOTHIA, C. et al.** *J. Mol. Biol.,* 1987, vol. 196, 901-917
- **CHOTHIA ; LESK.** *J. Mol. Biol.,* 1987, vol. 196, 901
- **CHOTHIA et al.** *Nature,* 1989, vol. 342, 877
- **MARTIN ; THORNTON.** *J. Mol. Biol,* 1996, vol. 263, 800
- Antibodies: A Laboratory Manual. Cold Spring Harbor Laboratory, 1988
- Sequences of Proteins of immunological Interest. US Department of Health and Human Services, 1991
- **CHOTHIA et al.** *J. Mol. Biol.,* 1987, vol. 227, 799-817
- **TOMLINSON et al.** *EMBO J.,* 1995, vol. 14, 4628-4638
- Protein Sequence and Structure Analysis of Antibody Variable Domains. Antibody Engineering Lab Manual. Springer-Verlag, Heidelberg
- Immunoglobulin Genes, 2nd ed. Academic Press, 1995
- *Cunningham and Wells in Science,* 1989, vol. 244, 1081-1085
- Remington's Pharmaceutical Sciences. 1980
- **EPSTEIN et al.** *Proc. Natl. Acad. Sci. USA,* 1985, vol. 82, 3688
- **HWANG et al.** *Proc. Natl Acad. Sci. USA,* 1980, vol. 77, 4030
- **MARTIN et al.** *J. Biol. Chem.,* 1982, vol. 257, 286-288
- **GABIZON et al.** *J. National Cancer Inst.,* 1989, vol. 81 (19), 1484
- **CARTER et al.** *Bio/Technology,* 1992, vol. 10, 163-167
- **HIATT et al.** *Nature,* 1989, vol. 342, 76-78
- **OWEN et al.** *Bio/Technology,* 1992, vol. 10, 790-794
- **ARTSAENKO et al.** *The Plant J,* 1995, vol. 8, 745-750

- **FECKER et al.** *Plant Mol Biol,* 1996, vol. 32, 979-986
- **GRAHAM et al.** *J. Gen Virol.,* 1977, vol. 36, 59
- **CHO, URLAUB et al.** *Proc. Natl. Acad. Sci. USA,* 1980, vol. 77, 4216
- **MATHER.** *Biol. Reprod.,* 1980, vol. 23, 243-251
- **MATHER et al.** *Annals N. Y Acad. Sci.,* 1982, vol. 383, 44-68
- **SCHLERETH et al.** *Cancer Immunol. Immunother.,* 2005, vol. 20, 1-12
- **CHESON BD ; HORNING SJ ; COIFFIER B ; SHIPP MA ; FISHER RI ; CONNORS JM ; LISTER TA ; VOSE J ; GRILLO-LOPEZ A ; HAGENBEEK A.** Report of an international workshop to standardize response criteria for non-Hodgkin's lymphomas. NCI Sponsored International Working Group. *J Clin Oncol.,* April 1999, vol. 17 (4), 1244
- **CHEN, W. et al.** *J Virol,* 2011, vol. 85 (18), 9395-9405
- **JAMES H. M. ; SIMON.** *J. Exp. Med,* April 1993, vol. 177, 949-954